# EUROPEAN PATENT SPECIFICATION

(11) **EP 1 444 522 B1**
(45) Date of publication and mention of the grant of the patent: **03.01.2007**
(21) Application number: 02772620.7
(22) Date of filing: 07.11.2002
(51) Int. Cl.: G01N 33/576, C12Q 1/70, G01N 33/569, A61P 31/14

(54) **SCREENING FOR ANTIVIRAL AGENTS AFFECTING SIGNAL PEPTIDASE**
SCREENING NACH ANTIVIRALEN VERBINDUNGEN, DIE DIE SIGNALPEPTIDASE BEEINFLUSSEN
DOSAGE

(30) Priority: 07.11.2001 GB 0126782
(43) Date of publication of application: 11.08.2004
(73) Proprietor: Medical Research Council, 20 Park Crescent London W1B 1AL (GB)
(72) Inventor: MCLAUCHLAN, J. MRC Virology Unit Div. of Virology, Glasgow G11 5JR (GB); MARTOGLIO, B. Swiss Federal Inst. of Tech. Zurich, CH-8093 Zurich (CH)
(74) Representative: Harding, Charles Thomas
(86) International application number: PCT/GB2002/005016
(87) International publication number: WO 2003/040684

(56) References cited:
- WO-A-00/43505
- WO-A-01/21189
- WO-A-01/66770
- WO-A-01/79434
- WO-A-95/12677
- WO-A-96/13590
- DE-A1- 19 902 550
- HÜSSY PETER ET AL: "Hepatitis C virus core protein: Carboxy-terminal boundaries of two processed species suggest cleavage by a signal peptide peptidase." VIROLOGY, vol. 224, no. 1, 1996, pages 93-104, XP002070763 ISSN: 0042-6822
- SANTOLINI ELISA ET AL: "Biosynthesis and biochemical properties of the hepatitis C virus core protein." JOURNAL OF VIROLOGY, vol. 68, no. 6, 1994, pages 3631-3641, XP001064220 ISSN: 0022-538X
- ESUMI MARIKO ET AL: "Immunoreactive core peptides of hepatitis C virus produced in Escherichia coli and in vitro DNA amplification-restricted transcription-translation system." JOURNAL OF VIROLOGICAL METHODS, vol. 59, no. 1-2, 1996, pages 91-98, XP001066304 ISSN: 0166-0934
- ZHAO WEI DONG ET AL: "Poliovirus/hepatitis C virus (Internal Ribosomal Entry Site-Core) chimeric viruses: Improved growth properties through modification of a proteolytic cleavage site and requirement for core RNA sequences but not for core-related polypeptides." JOURNAL OF VIROLOGY, vol. 73, no. 2, February 1999 (1999-02), pages 1546-1554, XP001066303 ISSN: 0022-538X

### SEQUENCE LISTING

<110> Medical Research Council
<120> Assay
<130> P12148WO
<160> 27
<170> PatentIn version 3.0
<210> 1
   <211> 377
   <212> PRT
   <213> Homo sapiens
<400> 1
   Met Asp Ser Ala Leu Ser Asp Pro His Asn Gly Ser Ala Glu Ala Gly
   1               5                   10                  15
   Gly Pro Thr Asn Ser Thr Thr Arg Pro Pro Ser Thr Pro Glu Gly Ile
               20                  25                  30
   Ala Leu Ala Tyr Gly Ser Leu Leu Leu Met Ala Leu Leu Pro Ile Phe
           35                  40                  45
   Phe Gly Ala Leu Arg Ser Val Arg Cys Ala Arg Gly Lys Asn Ala Ser
       50                  55                  60
   Asp Met Pro Glu Thr Ile Thr Ser Arg Asp Ala Ala Arg Phe Pro Ile
   65                  70                  75                  80
   Ile Ala Ser Cys Thr Leu Leu Gly Leu Tyr Leu Phe Phe Lys Ile Phe
                   85                  90                  95
   Ser Gln Glu Tyr Ile Asn Leu Leu Leu Ser Met Tyr Phe Phe Val Leu
               100                 105                 110
   Gly Ile Leu Ala Leu Ser His Thr Ile Ser Pro Phe Met Asn Lys Phe
           115                 120                 125
   Phe Pro Ala Ser Phe Pro Asn Arg Gln Tyr Gln Leu Leu Phe Thr Gln
       130                 135                 140
   Gly Ser Gly Glu Asn Lys Glu Glu Ile Ile Asn Tyr Glu Phe Asp Ala
   145                 150                 155                 160
   Lys Asp Leu Val Cys Leu Gly Leu Ser Ser Ile Val Gly Val Trp Tyr
                   165                 170                 175
   Leu Leu Arg Lys His Trp Ile Ala Asn Asn Leu Phe Gly Leu Ala Phe
               180                 185                 190
   Ser Leu Asn Gly Val Glu Leu Leu His Leu Asn Asn Val Ser Thr Gly
           195                 200                 205
   Cys Ile Leu Leu Gly Gly Leu Phe Ile Tyr Asp Val Phe Trp Val Phe
       210                 215                 220
   Gly Thr Asn Val Met Val Thr Val Ala Lys Ser Phe Glu Ala Pro Ile
   225                 230                 235                 240
   Lys Leu Val Phe Pro Gln Asp Leu Leu Glu Lys Gly Leu Glu Ala Asn
                   245                 250                 255
   Asn Phe Ala Met Leu Gly Leu Gly Asp Val Val Ile Pro Gly Ile Phe
               260                 265                 270
   Ile Ala Leu Leu Leu Arg Phe Asp Ile Ser Leu Lys Lys Asn Thr His
           275                 280                 285
   Thr Tyr Phe Tyr Thr Ser Phe Ala Ala Tyr Ile Phe Gly Leu Gly Leu
       290                 295                 300
   Thr Ile Phe Ile Met His Ile Phe Lys His Ala Gln Pro Ala Leu Leu
   305                 310                 315                 320
   Tyr Leu Val Pro Ala Cys Ile Gly Phe Pro Val Leu Val Ala Leu Ala
                   325                 330                 335
   Lys Gly Glu Val Thr Glu Met Phe Ser Tyr Glu Glu Ser Asn Pro Lys
               340                 345                 350
   Asp Pro Ala Ala Val Thr Glu Ser Lys Glu Gly Thr Glu Ala Ser Ala
           355                 360                 365
   Ser Lys Gly Leu Glu Lys Lys Glu Lys
       370                 375
<210> 2
   <211> 1244
   <212> DNA
   <213> Homo sapiens
<220>
   <221> n
   <222> (1159)..(1159)
   <223> n = a, c, t or g
<400> 2
   atggactcgg ccctcagcga tccgcataac ggcagtgccg aggcaggcgg ccccaccaac      60
   agcactacgc ggccgccttc cacgcccgag ggcatcgcgc tggcctacgg cagcctcctg     120
   ctcatggcgc tgctgcccat cttcttcggc gccctgcgct ccgtacgctg cgcccgcggc     180
   aagaatgctt cagacatgcc tgaaacaatc accagccggg atgccgcccg cttccccatc     240
   atcgccagct gcacactctt ggggctctac ctctttttca aaatattctc ccaggagtac     300
   atcaacctcc tgctgtccat gtatttcttc gtgctgggaa tcctggccct gtcccacacc     360
   atcagcccct tcatgaataa gttttttcca gccagctttc caaatcgaca gtaccagctg     420
   ctcttcacac agggttctgg ggaaaacaag gaagagatca tcaattatga atttgacacc     480
   aaggacctgg tgtgcctggg cctgagcagc atcgttggcg tctggtacct gctgaggaag     540
   cactggattg ccaacaacct ttttggcctg gccttctccc ttaatggagt agagctcctg     600
   cacctcaaca atgtcagcac tggctgcatc ctgctgggcg gactcttcat ctacgatgtc     660
   ttctgggtat ttggcaccaa tgtgatggtg acagtggcca agtccttcga ggcaccaata     720
   aaattggtgt ttccccagga tctgctggag aaaggcctcg aagcaaacaa ctttgccatg     780
   ctgggacttg gagatgtcgt cattccaggg atcttcattg ccttgctgct gcgctttgac     840
   atcagcttga agaagaatac ccacacctac ttctacacca gctttgcagc ctacatcttc     900
   ggcctgggcc ttaccatctt catcatgcac atcttcaagc atgctcagcc tgccctccta     960
   tacctggtcc ccgcctgcat cggttttcct gtcctggtgg cgctggccaa gggagaagtg    1020
   acagagatgt tcagttatga ggagtcaaat cctaaggatc cagcggcagt gacagaatcc    1080
   aaagagggaa cagaggcatc agcatcgaag gggctggaga agaaagagaa atgatgcagc    1140
   tggtgccgag ctctcaggnc cagaccagac agatgggggc tgggccacac aggcgtgcac    1200
   cgtagagggc accgggaggc cacggcgagt ccaggacacg ccgg                     1244
<210> 3
   <211> 16
   <212> PRT
   <213> Artificial
<220>
   <223> HCV-core SPPase target sequence
<400> 3
   Ser Phe Ser Ile Phe Leu Leu Ala Leu Leu Ser Cys Leu Thr Val Pro
   1               5                   10                  15
<210> 4
   <211> 16
   <212> PRT
   <213> Artificial
<220>
   <223> Mutant HCV-core SPPase target sequence
<400> 4
   Ser Phe Ser Ile Phe Leu Leu Val Leu Leu Ser Cys Leu Thr Val Pro
   1 5 10 15
<210> 5
   <211> 16
   <212> PRT
   <213> Artificial
<220>
   <223> Mutant HCV-core SPPase target sequence
<400> 5
   Ser Phe Ser Ile Phe Leu Leu Ala Leu Leu Leu Val Leu Thr Val Pro
   1 5 10 15
<210> 6
   <211> 16
   <212> PRT
   <213> Artificial
<220>
   <223> Mutant HCV-core SPPase target sequence
<400> 6
   Ser Phe Ser Ile Phe Leu Leu Val Leu Leu Leu Val Leu Thr Val Pro
   1 5 10 15
<210> 7
   <211> 11
   <212> DNA
   <213> Artificial
<220>
   <223> Oligonucleotide primer
<400> 7
   gctgagatct a 11
<210> 8
   <211> 17
   <212> DNA
   <213> Artificial
<220>
   <223> Oligonucleotide primer
<400> 8
   gtaacctttg agatcta 17
<210> 9
   <211> 22
   <212> DNA
   <213> Artificial
<220>
   <223> Oligonucleotide primer
<400> 9
   cgatagaggc gctgccaggg cc 22
<210> 10
   <211> 12
   <212> DNA
   <213> Artificial
<220>
   <223> Reverse primer
<220>
   <221> n
   <222> (1)..(9)
   <223> n = a, c, t or g
<400> 10
   nnnnnnnnnc ta 12
<210> 11
   <211> 29
   <212> PRT
   <213> Artificial
<220>
   <223> Mutant signal peptide of pre-prolactin
<400> 11
   Met Asp Ser Lys Gly Ser Ser Gln Lys Gly Ser Arg Leu Leu Leu Leu
   1               5                   10                  15
   Leu Val Val Ser Asn Leu Leu Leu Cys Gln Gly Pro Pro
               20                  25
<210> 12
   <211> 18
   <212> DNA
   <213> Artificial
<220>
   <223> Reverse primer
<220>
   <221> n
   <222> (1)..(9)
   <223> n = a, c, t or g
<400> 12
   nnnnnnnnnc tacggcgg 18
<210> 13
   <211> 12
   <212> PRT
   <213> Artificial
<220>
   <223> TBL4K-labelled peptide
<400> 13
   Asn Ala Ser Asp Met Pro Glu Thr Ile Thr Ser Arg
   1 5 10
<210> 14
   <211> 9
   <212> PRT
   <213> Artificial
<220>
   <223> TBL4K-labelled peptide
<400> 14
   Phe Phe Pro Ala Asn Phe Pro Asn Arg
   1 5
<210> 15
   <211> 13
   <212> PRT
   <213> Artificial
<220>
   <223> TBL4K-labelled peptide
<400> 15
   Tyr Gln Leu Leu Phe Thr Gln Gly Ser Gly Glu Asn Lys
   1 5 10
<210> 16
   <211> 9
   <212> PRT
   <213> Artificial
<220>
   <223> TBL4K-labelled peptide
<400> 16
   Glu Glu Ile Ile Asn Tyr Glu Ala Asp
   1               5
<210> 17
   <211> 10
   <212> PRT
   <213> Artificial
<220>
   <223> TBL4K-labelled peptide
<400> 17
   Leu Val Phe Pro Gln Asp Leu Leu Glu Lys
   1               5                   10
<210> 18
   <211> 15
   <212> PRT
   <213> Artificial
<220>
   <223> TBL4K-labelled peptide
<400> 18
   Gly Glu Val Thr Glu Met Phe Ser Tyr Glu Glu Ser Asn Pro Lys
   1               5                   10                  15
<210> 19
   <211> 377
   <212> PRT
   <213> Homo sapiens
<400> 19
   Met Asp Ser Ala Leu Ser Asp Pro His Asn Gly Ser Ala Glu Ala Gly
   1 5 10 15
   Gly Pro Thr Asn Ser Thr Thr Arg Pro Pro Ser Thr Pro Glu Gly Ile
   20 25 30
   Ala Leu Ala Tyr Gly Ser Leu Leu Leu Met Ala Leu Leu Pro Ile Phe
   35 40 45
   Phe Gly Ala Leu Arg Ser Val Arg Cys Ala Arg Gly Lys Asn Ala Ser
   50 55 60
   Asp Met Pro Glu Thr Ile Thr Ser Arg Asp Ala Ala Arg Pro Pro Ile
   65 70 75 80
   Ile Ala Ser Cys Thr Leu Leu Gly Leu Tyr Leu Phe Phe Lys Ile Phe
   85 90 95
   Ser Gln Glu Tyr Ile Asn Leu Leu Leu Ser Met Tyr Phe Phe Val Leu
   100 105 110
   Gly Ile Leu Ala Leu Ser His Thr Ile Ser Pro Phe Met Asn Lys Phe
   115 120 125
   Phe Pro Ala Ser Phe Pro Asn Arg Gln Tyr Gln Leu Leu Phe Thr Gln
   130 135 140
   Gly Ser Gly Glu Asn Lys Glu Glu Ile Ile Asn Tyr Glu Phe Asp Thr
   145 150 155 160
   Lys Asp Leu Val Cys Leu Gly Leu Ser Ser Ile Val Gly Val Trp Tyr
   165 170 175
   Leu Leu Arg Lys His Trp Ile Ala Asn Asn Leu Phe Gly Leu Ala Phe
   180 185 190
   Ser Leu Asn Gly Val Glu Leu Leu His Leu Asn Asn Val Ser Thr Gly
   195 200 205
   Cys Ile Leu Leu Gly Gly Leu Phe Ile Tyr Asp Val Phe Trp Val Phe
   210 215 220
   Gly Thr Asn Val Met Val Thr Val Ala Lys Ser Phe Glu Ala Pro Ile
   225 230 235 240
   Lys Leu Val Pro Pro Gln Asp Leu Leu Glu Lys Gly Leu Glu Ala Asn
   245 250 255
   Asn Phe Ala Met Leu Gly Leu Gly Asp Val Val Ile Pro Gly Ile Phe
   260 265 270
   Ile Ala Leu Leu Leu Arg Pro Asp Ile Ser Leu Lys Lys Asn Thr His
   275 280 285
   Thr Tyr Pro Tyr Thr Ser Phe Ala Ala Tyr Ile Phe Gly Leu Gly Leu
   290 295 300
   Thr Ile Phe Ile Met His Ile Pro Lys His Ala Gln Pro Ala Leu Leu
   305 310 315 320
   Tyr Leu Val Pro Ala Cys Ile Gly Phe Pro Val Leu Val Ala Leu Ala
   325 330 335
   Lys Gly Glu Val Thr Glu Met Phe Ser Tyr Glu Glu Ser Asn Pro Lys
   340 345 350
   Asp Pro Ala Ala Val Thr Glu Ser Lys Glu Gly Thr Glu Ala Ser Ala
   355 360 365
   Ser Lys Gly Leu Glu Lys Lys Glu Lys
   370 375
<210> 20
   <211> 378
   <212> PRT
   <213> Mus musculus
<400> 20
   Met Asp Ser Ala Val Ser Asp Pro His Asn Gly Ser Ala Glu Ala Gly
   1               5                   10                  15
   Thr Pro Ala Asn Gly Thr Thr Arg Pro Pro Ser Thr Pro Glu Gly Ile
               20                  25                  30
   Ala Leu Ala Tyr Gly Ser Leu Leu Leu Met Ala Leu Leu Pro Ile Phe
           35                  40                  45
   Phe Gly Ala Leu Arg Ser Val Arg Cys Ala Arg Gly Lys Ser Ser Ser
       50                  55                  60
   Asp Met Pro Glu Thr Ile Thr Ser Arg Asp Ala Ala Arg Phe Pro Ile
   65                  70                  75                  80
   Ile Ala Ser Cys Thr Leu Leu Gly Leu Tyr Leu Phe Phe Lys Ile Phe
                   85                  90                  95
   Ser Gln Glu Tyr Ile Asn Leu Leu Leu Ser Met Tyr Phe Phe Val Leu
               100                 105                 110
   Gly Ile Leu Ala Leu Ser His Thr Ile Ser Pro Phe Met Asn Lys Phe
           115                 120                 125
   Phe Pro Ala Asn Phe Pro Asn Arg Gln Tyr Gln Leu Leu Phe Thr Gln
       130                 135                 140
   Gly Ser Gly Glu Asn Lys Glu Glu Ile Ile Asn Tyr Glu Phe Asp Thr
   145                 150                 155                 160
   Lys Asp Leu Val Cys Leu Gly Leu Ser Ser Val Val Gly Val Trp Tyr
                   165                 170                 175
   Leu Leu Arg Lys His Trp Ile Ala Asn Asn Leu Phe Gly Leu Ala Phe
               180                 185                 190
   Ser Leu Asn Gly Val Glu Leu Leu His Leu Asn Asn Val Ser Thr Gly
           195                 200                 205
   Cys Ile Leu Leu Gly Gly Leu Phe Ile Tyr Asp Ile Phe Trp Val Phe
       210                 215                 220
   Gly Thr Asn Val Met Val Thr Val Ala Lys Ser Phe Glu Ala Pro Ile
   225                 230                 235                 240
   Lys Leu Val Phe Pro Gln Asp Leu Leu Glu Lys Gly Leu Glu Ala Asp
                   245                 250                 255
   Asn Phe Ala Met Leu Gly Leu Gly Asp Ile Val Ile Pro Gly Ile Phe
               260                 265                 270
   Ile Ala Leu Leu Leu Arg Phe Asp Ile Ser Leu Lys Lys Asn Thr His
           275                 280                 285
   Thr Tyr Phe Tyr Thr Ser Phe Ala Ala Tyr Ile Phe Gly Leu Gly Leu
       290                 295                 300
   Thr Ile Phe Ile Met His Ile Phe Lys His Ala Gln Pro Ala Leu Leu
   305                 310                 315                 320
   Tyr Leu Val Pro Ala Cys Ile Gly Phe Pro Val Leu Val Ala Leu Ala
                   325                 330                 335
   Lys Gly Glu Val Ala Glu Met Phe Ser Tyr Glu Glu Ser Asn Pro Lys
               340                 345                 350
   Asp Pro Ala Ala Glu Thr Glu Ser Lys Glu Glu Ser Thr Glu Ala Ser
           355                 360                 365
   Ala Ser Lys Arg Leu Glu Lys Lys Glu Lys
       370                 375
<210> 21
   <211> 389
   <212> PRT
   <213> Drosophila melanogaster
<400> 21
   Met Ala Glu Glu Val Ile Gly Thr Val Lys Glu Val Leu Lys Gly Ile
   1               5                   10                  15
   Ile Glu Asn Val Asn Ala Pro Lys Asn Glu Ser Ala Pro Gly Glu Lys
               20                  25                  30
   Lys Pro Ser Thr Pro Glu Gly Met Ala Val Ala Tyr Ser Ser Leu Val
           35                  40                  45
   Val Met Ala Met Leu Pro Ile Ile Phe Gly Ser Ile Arg Ser Val Lys
       50                  55                  60
   Leu His Lys Leu Lys Lys Ser Thr Gly Glu Lys Ala Asp Thr Met Thr
   65                  70                  75                  80
   Lys Lys Asp Ala Met Tyr Phe Pro Leu Ile Ala Ser Ala Ala Leu Phe
                   85                  90                  95
   Gly Leu Tyr Leu Phe Phe Lys Ile Phe Gln Lys Val His Ile Asn Tyr
               100                 105                 110
   Leu Leu Thr Gly Tyr Phe Phe Val Leu Gly Val Ile Ala Leu Ala His
           115                 120                 125
   Leu Leu Ser Pro Val Ile Asn Ser Leu Met Pro Ala Ala Val Pro Lys
       130                 135                 140
   Val Pro Phe His Ile Leu Phe Thr Lys Gly Glu Gly Lys His Lys Glu
   145                 150                 155                 160
   Asp Ile Val Asn Tyr Lys Phe Ser Thr His Asp Ile Val Cys Leu Val
                   165                 170                 175
   Ile Ser Ser Ala Ile Gly Val Trp Tyr Leu Leu Lys Lys His Trp Ile
               180                 185                 190
   Ala Asn Asn Leu Phe Gly Leu Ala Phe Ala Ile Asn Gly Val Glu Met
           195                 200                 205
   Leu His Leu Asn Asn Phe Val Thr Gly Val Ile Leu Leu Ser Gly Leu
       210                 215                 220
   Phe Phe Tyr Asp Ile Phe Trp Val Phe Gly Thr Asn Val Met Val Thr
   225                 230                 235                 240
   Val Ala Lys Ser Phe Glu Ala Pro Ile Lys Leu Val Phe Pro Gln Asp
                   245                 250                 255
   Leu Ile Glu Asn Gly Leu Asn Ala Ser Asn Phe Ala Met Leu Gly Leu
               260                 265                 270
   Gly Asp Ile Val Ile Pro Gly Ile Phe Ile Ala Leu Leu Leu Arg Phe
           275                 280                 285
   Asp Asp Ser Lys Lys Arg Lys Thr Arg Ile Tyr Phe Tyr Ser Thr Leu
       290                 295                 300
   Ile Ala Tyr Phe Leu Gly Leu Leu Ala Thr Ile Phe Val Met His Val
   305                 310                 315                 320
   Phe Lys His Ala Gln Pro Ala Leu Leu Tyr Leu Val Pro Ala Cys Met
                   325                 330                 335
   Gly Thr Pro Leu Leu Val Ala Leu Ile Arg Gly Glu Leu Lys Val Leu
               340                 345                 350
   Phe Ala Tyr Glu Asp His Pro Glu Glu Lys Pro Glu Lys Lys Glu Lys
   355 360 365
   Lys Glu Lys Asp Glu Gly Val Ser Ser Ser Gly Ser Lys Lys Lys Glu
   370 375 380
   Ser Lys Lys Gly Lys
   385
<210> 22
   <211> 468
   <212> PRT
   <213> Caenorhabditis elegans
<400> 22
   Met Ala Glu Ala Ala Thr Glu Ile Pro Pro Thr Ala Ser Asn Val Thr
   1               5                   10                  15
   Val Phe Thr Phe Glu Glu Gln Ala Thr Ser Ser Leu Ala Leu Tyr Gly
               20                  25                  30
   Met Ser Ile Leu Cys Ile Ile Ile Gly Ser Ile Arg Ser Ala Gln Tyr
           35                  40                  45
   Ile Arg Thr Asn Ile Asp Lys Lys Arg Leu Ile Glu Gly Ser Ile Thr
       50                  55                  60
   Met Arg Glu Ala Arg Lys Phe Pro Ile Ser Ala Ser Leu Val Leu Phe
   65                  70                  75                  80
   Gly Leu Tyr Leu Phe Phe Lys Pro Ala Ala Glu Arg Phe Leu Trp Val
                   85                  90                  95
   Ala Arg Val Phe Gln Ile Leu Arg Val Pro Glu Glu Tyr Val Gln Lys
               100                 105                 110
   Ile Asn Ser Thr Ile Ile Ser Tyr Thr Ala Asn Thr Thr Thr Thr Gly
           115                 120                 125
   Pro Ser Glu Pro Phe Leu Leu Arg Leu Ala Ser Arg Ile Pro Gln Glu
       130                 135                 140
   Arg Val Pro Glu Ala Ile Gln Asn Ala Ala Thr Tyr Ala Tyr Thr Asn
   145                 150                 155                 160
   Leu Pro Thr Ile Gln Lys Ala Glu Cys Met Gln Leu Leu Thr Phe Leu
                   165                 170                 175
   Ile Cys Phe Glu Gly Val Asn Ala Phe Ala Ser Leu Leu Lys Pro Phe
               180                 185                 190
   Val Thr Ala Phe Leu Lys Lys Met Pro Leu Val Pro Ser Phe Leu Arg
           195                 200                 205
   Phe Asn Ala Pro Tyr Leu Phe Ser Leu Lys Lys Gly Asn Lys Glu Met
       210                 215                 220
   Glu Glu Gly Asp Ile Glu Asp Ala Lys Lys Lys Glu Thr Glu Tyr Leu
   225                 230                 235                 240
   Phe Lys Ile Asp Phe Asp Arg Tyr Asp Ile Ile Ala Leu Leu Met Cys
                   245                 250                 255
   Ser Pro Ile Leu Ile Ser His Leu Leu Lys Arg His Trp Ile Thr Asn
   260 265 270
   Asn Ile Ile Gly Val Ser Phe Ser Ile Leu Gly Ile Glu Arg Leu His
   275 280 285
   Leu Ala Ser Phe Lys Ala Gly Ser Leu Leu Leu Val Gly Leu Phe Phe
   290 295 300
   Tyr Asp Ile Phe Trp Val Phe Gly Thr Asp Val Met Thr Ser Val Ala
   305 310 315 320
   Lys Gly Ile Asp Ala Pro Ile Leu Leu Gln Phe Pro Gln Asp Ile Tyr
   325 330 335
   Arg Asn Gly Ile Met Glu Ala Ser Lys His Ser Met Leu Gly Leu Gly
   340 345 350
   Asp Ile Val Ile Pro Gly Ile Phe Ile Ala Leu Leu Arg Arg Phe Asp
   355 360 365
   Tyr Arg Val Val Gln Thr Thr Ala Glu Ser Lys Ala Pro Gln Gly Ser
   370 375 380
   Leu Lys Gly Arg Tyr Tyr Phe Val Val Thr Val Val Ala Tyr Met Ala
   385 390 395 400
   Gly Leu Phe Ile Thr Met Ala Val Met His His Phe Lys Ala Ala Gln
   405 410 415
   Pro Ala Leu Leu Tyr Leu Val Pro Cys Cys Leu Phe Val Pro Leu Leu
   420 425 430
   Leu Ala Val Ile Arg Gly Glu Leu Ser Ala Leu Trp Asn Tyr Asp Glu
   435 440 445
   Ser Arg His Val Asp Asn Glu Glu Asn Arg Lys Lys Val Asp Ser Gly
   450 455 460
   Lys Lys Asn Asn
   465
<210> 23
   <211> 417
   <212> PRT
   <213> Drosophila melanogaster
<400> 23
   Met Ser His Gly Gly Ala Gly Gly Gly Leu Gly Ala Gln Thr Val Gly
   1               5                   10                  15
   Ala Gly Gln Leu Gly Gly Gly Ala Ala Ala Ala Gly Gly Gly Glu Tyr
               20                  25                  30
   Arg Glu Leu His Trp Thr Val Ser Ser Val Met Asp Ser Ser Arg Val
           35                  40                  45
   Ser Thr Cys Leu Ile Ser Met Leu Leu Ile Val Tyr Gly Ser Phe Arg
       50                  55                  60
   Ser Leu Asn Ile Glu Gln Glu Ala Arg Glu Arg Glu Gln Lys Lys Arg
   65                  70                  75                  80
   Asn Glu Ser Met Thr Asn Leu Leu Thr Gly Glu Pro Val Glu Lys Glu
                   85                  90                  95
   Pro Thr Asp Lys Phe Ala Thr Leu Asp Thr Met His Ala Leu Cys Leu
               100                 105                 110
   Pro Leu Gly Ala Ser Ile Ser Leu Leu Ile Met Phe Phe Phe Phe Asp
           115                 120                 125
   Ser Met Gln Leu Leu Phe Ala Val Cys Thr Ala Ile Ile Ala Thr Val
       130                 135                 140
   Ala Leu Ala Phe Leu Leu Leu Pro Met Cys Gln Tyr Ile Ile Arg Pro
   145                 150                 155                 160
   Cys Thr Asp Gly Lys Arg Phe Ser Phe Gly Phe Cys Gly Arg Phe Thr
                   165                 170                 175
   Ala Ala Glu Leu Phe Ser Phe Thr Leu Ser Val Ser Ile Val Cys Ile
               180                 185                 190
   Trp Val Leu Thr Gly His Trp Leu Leu Met Asp Ala Met Gly Met Gly
           195                 200                 205
   Leu Cys Val Ala Phe Ile Ala Phe Val Arg Leu Pro Ser Leu Lys Val
       210                 215                 220
   Ser Thr Leu Leu Leu Thr Gly Leu Leu Ile Tyr Asp Val Phe Trp Val
   225                 230                 235                 240
   Phe Leu Ser Ser Tyr Ile Phe Ser Thr Asn Val Met Val Lys Val Ala
                   245                 250                 255
   Thr Arg Pro Ala Asp Asn Pro Val Gly Ile Val Ala Arg Lys Leu His
               260                 265                 270
   Leu Gly Gly Ile Val Arg Asp Thr Pro Lys Leu Asn Leu Pro Gly Lys
           275                 280                 285
   Leu Val Phe Pro Ser Leu His Asn Thr Gly His Phe Ser Met Leu Gly
       290                 295                 300
   Leu Gly Asp Val Val Met Pro Gly Leu Leu Leu Cys Phe Val Leu Arg
   305                 310                 315                 320
   Tyr Asp Ala Tyr Lys Lys Ala Gln Gly Val Thr Ser Asp Pro Thr Leu
                   325                 330                 335
   Ser Pro Pro Arg Gly Val Gly Ser Arg Leu Thr Tyr Phe His Cys Ser
               340                 345                 350
   Leu Leu Gly Tyr Phe Leu Gly Leu Leu Thr Ala Thr Val Ser Ser Glu
           355                 360                 365
   Val Phe Lys Ala Ala Gln Pro Ala Leu Leu Tyr Leu Val Pro Phe Thr
       370                 375                 380
   Leu Leu Pro Leu Leu Leu Met Ala Tyr Leu Lys Gly Asp Leu Arg Arg
   385                 390                 395                 400
   Met Trp Ser Glu Pro Phe Ile Ala Gln Gln Pro Ser Lys Gln Leu Glu
                   405                 410                 415
   Val
<210> 24
   <211> 652
   <212> PRT
   <213> Caenorhabditis elegans
<400> 24
   Met Thr Ile Pro Ile Pro Ile Ser Ile Leu Ile Leu Leu Leu Val Phe
   1               5                   10                  15
   Ile Pro Thr Thr Thr Thr Ile Asp Leu Thr Ser Ile Ser Ser Pro Arg
               20                  25                  30
   Gly Pro Phe Arg Thr Ser Tyr Val Phe Met Thr Leu Tyr Asn Lys Arg
           35                  40                  45
   Thr Gly Glu Arg Ser Lys Ser Cys Val Asn Tyr Gln Gln Tyr Leu Arg
       50                  55                  60
   Thr Asp Thr Ser Ser Asn Ile Val Ala Tyr Asp Phe Glu Ser Ala His
   65                  70                  75                  80
   Pro Phe Asn Leu Lys Phe Trp Glu Ala Met Tyr Asn Arg Thr Asn Ile
                   85                  90                  95
   Cys Pro Leu Pro Ser Asp Lys Ala Asp Lys Ile Tyr Tyr Tyr Asn Gln
               100                 105                 110
   Val Ile Pro Leu Asp Tyr Arg Ile Lys Asp Asp Gly Thr Pro Cys Ser
           115                 120                 125
   Lys Arg Phe Ser Lys Ser Gln Thr Ala Phe Arg Asn Ala Ser Gln Phe
       130                 135                 140
   Thr Val Asp Val Leu Lys Asp His Arg Ala Gly Ala Ser Leu Leu Val
   145                 150                 155                 160
   Leu Asp Lys Gly Ala Lys Phe Val Gln Gly Trp Lys Asp Tyr Leu Phe
                   165                 170                 175
   Ser Asp Phe Tyr Asp Pro Tyr Ile Asn Ser Glu Thr Ala Ile Pro Thr
               180                 185                 190
   Phe Tyr Ile Tyr Arg Ser Val Leu Glu Glu Lys Ile Met Lys Phe Ala
           195                 200                 205
   Pro His Asn Asp Ile Ser Asp Asp Gln Val Glu Ile Arg Phe His Arg
       210                 215                 220
   Pro Ser Gly Gly Pro Phe Asp Ala Ser Phe Ala Ile Ile Trp Met Ile
   225                 230                 235                 240
   Ser Met Thr Cys Val Ala Gly Gly Gly Ile Trp Ala Phe Asn Arg His
                   245                 250                 255
   Arg Ala Gly Lys Asp Val Thr Leu Ala Ser Gln Ser Val Asp Asp Asp
               260                 265                 270
   Thr Ser Ser Pro Ser Asn Asp Ser Glu Thr Lys Gly Phe Leu Asp Arg
           275                 280                 285
   Phe Gly Gly Ile Ile Thr Ile Cys Leu Met Met Thr Thr Leu Cys Gly
       290                 295                 300
   Val Leu Leu Leu Gly Tyr Phe Phe Arg Pro Val Leu Val Ile Phe Phe
   305                 310                 315                 320
   Asn Ile Phe Leu Val Ile Phe Gly Thr Cys Ser Leu Tyr Gly Cys Ile
                   325                 330                 335
   Arg Gly Phe Leu Ser Asn Phe Lys Phe Val Gly His Arg Trp Tyr Asn
               340                 345                 350
   Ala Lys Met Glu Trp Phe Pro Thr Cys Gly Gly Arg Ile His Gln Tyr
           355                 360                 365
   Lys Tyr Ser Glu Ala Phe Ile Gly Ile Ile Cys Leu Ser Phe Cys Val
       370                 375                 380
   Thr Trp Phe Ile Ile Arg Arg Gln Pro Tyr Ala Phe Ile Leu Leu Asp
   385                 390                 395                 400
   Val Ile Asn Met Ala Leu Cys Met His Val Leu Lys Cys Leu Arg Leu
                   405                 410                 415
   Pro Ser Leu Lys Trp Ile Ser Ile Leu Met Leu Cys Met Phe Val Tyr
               420                 425                 430
   Asp Ala Phe Met Val Phe Gly Thr Pro Tyr Met Thr Thr Asn Gly Cys
           435                 440                 445
   Ser Val Met Leu Glu Val Ala Thr Gly Leu Ser Cys Ala Ala Lys Gly
       450                 455                 460
   Lys Asn Lys Gly Tyr Pro Val Pro Pro Ile Glu Gln Glu Ser Val Pro
   465                 470                 475                 480
   Glu Lys Phe Pro Met Leu Met Gln Val Ala His Phe Asn Pro Met Asn
                   485                 490                 495
   Glu Cys Leu Asp Met Glu Ile Glu Leu Gly Phe Gln Phe Thr Ile Leu
               500                 505                 510
   Gly Leu Gly Asp Ile Val Met Pro Gly Tyr Leu Val Ala His Cys Phe
           515                 520                 525
   Thr Met Asn Gly Phe Ser Glu Arg Val Arg Leu Ile Tyr Gly Phe Ile
       530                 535                 540
   Ser Val Val Gly Tyr Gly Ile Gly Leu Ile Val Thr Phe Leu Ala Leu
   545                 550                 555                 560
   Ala Leu Met Lys Thr Ala Gln Pro Ala Leu Ile Tyr Leu Val Pro Ser
                   565                 570                 575
   Thr Leu Phe Pro Ile Ile Met Leu Ala Leu Cys Arg Gly Glu Phe Leu
               580                 585                 590
   Lys Ile Trp Asn Gly Val Pro Val Glu Cys Ser Pro Leu Val Ser Asp
           595                 600                 605
   Arg Asn Asp Leu Gln Gly Lys Lys Trp Ile Asn Ser Ser Ser Ser Pro
       610                 615                 620
   Asp Asp Thr Met Glu Asp Ile Glu Ser Asn Ala Val Ile Asp Gln Ser
   625                 630                 635                 640
   Asn Ser Ser Ser Gln Gln Asn Pro Pro Val Asn Val
                   645                 650
<210> 25
   <211> 295
   <212> PRT
   <213> Schizosaccharomyces pombe
<400> 25
   Met Glu Gly Val Ile Leu Ala Ser Ser Ala Leu Phe Thr Val Tyr Ile
   1               5                   10                  15
   Gly Ala Lys Trp Ser Ala Gln Glu Glu Glu Pro Glu Glu Lys Gln Leu
               20                  25                  30
   Ile Asn Lys Arg Leu Ala Val Leu Phe Pro Ile Phe Gly Gly Val Thr
           35                  40                  45
   Leu Val Leu Met Tyr Leu Ala Leu Arg Tyr Leu Ser Lys Glu Tyr Ile
       50                  55                  60
   Gln Leu Ile Leu Gln Gly Tyr Ala Ser Leu Ala Ser Ile Ile Cys Phe
   65                  70                  75                  80
   Val Arg Ser Phe Asn Pro Lys Thr Thr Phe Gly Lys Ile Thr Ala Thr
                   85                  90                  95
   Met Ser Ser Ile Ala Ile Ala Leu Phe Tyr Phe Lys Thr Lys His Trp
               100                 105                 110
   Met Ala Ser Asn Ile Leu Ala Trp Ala Leu Ala Ala Asn Ser Ile Ser
           115                 120                 125
   Ile Met Arg Ile Asp Ser Tyr Asn Thr Gly Ala Leu Leu Leu Gly Ala
       130                 135                 140
   Leu Phe Phe Tyr Asp Ile Tyr Phe Val Phe Gly Thr Glu Val Met Val
   145                 150                 155                 160
   Thr Val Ala Thr Gly Ile Asp Ile Pro Ala Lys Tyr Val Leu Pro Gln
                   165                 170                 175
   Phe Lys Asn Pro Thr Arg Leu Ser Met Leu Gly Leu Gly Asp Ile Val
               180                 185                 190
   Met Pro Gly Leu Met Leu Ala Leu Met Tyr Arg Phe Asp Leu His Tyr
           195                 200                 205
   Tyr Ile Asn Ser Thr Ser Gln Pro Lys Lys His Ser Thr Tyr Phe Arg
       210                 215                 220
   Asn Thr Phe Ile Ala Tyr Gly Leu Gly Leu Gly Val Thr Asn Phe Ala
   225                 230                 235                 240
   Leu Tyr Tyr Phe Lys Ala Ala Gln Pro Ala Leu Leu Tyr Leu Ser Pro
                   245                 250                 255
   Ala Cys Ile Val Ala Pro Leu Leu Thr Ala Trp Tyr Arg Asp Glu Leu
               260                 265                 270
   Lys Thr Leu Phe Ser Phe Arg Ser Glu Thr Glu Asp Glu Thr Asp Glu
           275                 280                 285
   Gln Asp Lys Cys Lys Ser Thr
       290                 295
<210> 26
   <211> 587
   <212> PRT
   <213> Saccharomyces cerevisiae
<400> 26
   Met Asp Lys Tyr Leu Asn Ser Phe Val Asp His Leu Ser Glu Trp Ser
   1               5                   10                  15
   Ser Arg Ala Phe Arg Asn Asn Ser Ser Ser Ala Asn Gln Ser Ala Ser
               20                  25                  30
   Asn Lys Glu Leu Glu Gln Val Phe Glu Gln Ile Asn Ala Ile Val Glu
           35                  40                  45
   Asn His Asn Asn Lys Leu Thr Thr Ala Phe Asp Lys Ile Ser Tyr Arg
       50                  55                  60
   Val Ala His Lys Ile Thr His Leu Val Glu Ser His Ser Leu Val Phe
   65                  70                  75                  80
   Asn Tyr Ala Thr Leu Val Leu Ile Ala Ser Ala Leu Val Val Ile Gly
                   85                  90                  95
   Ser Phe Thr Ser Ile Ser Ser Ile Pro Phe Thr Ala Leu Pro Pro Thr
               100                 105                 110
   Arg Glu His Ser Leu Phe Asp Pro Thr Asp Phe Asp Val Asp His Asp
           115                 120                 125
   Cys His Val Ile Tyr Arg Glu Asn Asp Glu Asp Lys Lys Lys Lys Lys
       130                 135                 140
   Lys Ser Lys Arg Phe Phe Asp Met Met Asp Glu Lys His Ala Ile Ile
   145                 150                 155                 160
   Leu Pro Leu Thr Ser Gly Cys Thr Leu Leu Ala Leu Tyr Phe Val Ile
                   165                 170                 175
   Lys Lys Leu His Leu Asn Trp Leu Lys Tyr Val Val Lys Ile Leu Asn
               180                 185                 190
   Phe Asn Ile Thr Leu Leu Asn Ile Pro Ala Gly Thr Phe Val Tyr Ser
           195                 200                 205
   Tyr Phe Leu Asn Ser Leu Phe Arg Asn Leu Ser His Leu Ala Ser Trp
       210                 215                 220
   Asn Pro Leu Val Val Leu Pro Arg Tyr Arg Val Thr Ile Ala Asp Asp
   225                 230                 235                 240
   Asn Glu Asp Leu Asn Lys Ile Gly Gly Phe Val Thr Asn Leu Asn Tyr
                   245                 250                 255
   Lys Asp Gly Leu Thr Asn Ser Val Val His Lys Lys Thr Leu Asp Glu
               260                 265                 270
   Ile Glu Lys Asp His Trp Met Lys His Phe Tyr Arg Arg Glu Leu Val
           275                 280                 285
   Glu Pro Lys Asp Ile Lys Ser Lys Arg Gln Ile Ser Asn Met Tyr Leu
       290                 295                 300
   Asn Ser Ala Leu Ile Val Ser Phe Val Leu Ser Ile Val Ser Thr Val
   305                 310                 315                 320
   Tyr Phe Tyr Leu Ser Pro Asn Asp Trp Leu Ile Ser Asn Ala Val Ser
                   325                 330                 335
   Met Asn Met Ala Ile Trp Ser Ile Ala Gln Leu Lys Leu Lys Asn Leu
               340                 345                 350
   Lys Ser Gly Ala Leu Ile Leu Ile Ala Leu Phe Phe Tyr Asp Ile Cys
           355                 360                 365
   Phe Val Phe Gly Thr Asp Val Met Val Thr Val Ala Thr Asn Leu Asp
       370                 375                 380
   Ile Pro Val Lys Leu Ser Leu Pro Val Lys Phe Asn Thr Ala Gln Asn
   385                 390                 395                 400
   Asn Phe Asn Phe Ser Ile Leu Gly Leu Gly Asp Ile Ala Leu Pro Gly
                   405                 410                 415
   Met Phe Ile Ala Met Cys Tyr Lys Tyr Asp Ile Trp Lys Trp His Leu
               420                 425                 430
   Asp His Asp Asp Thr Glu Phe His Phe Leu Asn Trp Ser Tyr Val Gly
           435                 440                 445
   Lys Tyr Phe Ile Thr Ala Met Val Ser Tyr Val Ala Ser Leu Val Ser
       450                 455                 460
   Ala Met Val Ser Leu Ser Ile Phe Asn Thr Ala Gln Pro Ala Leu Leu
   465                 470                 475                 480
   Tyr Ile Val Pro Ser Leu Leu Ile Ser Thr Ile Leu Val Ala Cys Trp
                   485                 490                 495
   Asn Lys Asp Phe Lys Gln Phe Trp Asn Phe Gln Tyr Asp Thr Ile Glu
               500                 505                 510
   Val Asp Lys Ser Leu Lys Lys Ala Ile Glu Lys Lys Glu Asn Ser Ile
           515                 520                 525
   Thr Tyr Ser Thr Phe Ile Leu Ser Glu Tyr Tyr Asn Asp Ala Asp Lys
       530                 535                 540
   Tyr Ala Leu Leu Gly Asp Asp Val Asn Glu Asn Phe Asp Asp Asp Glu
   545                 550                 555                 560
   Glu Phe Val Gln Glu Glu Asp Leu Ser Asp Ser Ser Glu Glu Glu Leu
                   565                 570                 575
   Ser Glu Glu Asp Leu Leu Asp Asp Glu Ser Ser
               580                 585
<210> 27
   <211> 344
   <212> PRT
   <213> Arabidopsis thaliana
<400> 27
   Met Lys Asn Cys Glu Arg Phe Ala Asn Leu Ala Leu Ala Gly Leu Thr
   1               5                   10                  15
   Leu Ala Pro Leu Val Val Arg Val Asn Pro Asn Leu Asn Val Ile Leu
               20                  25                  30
   Thr Ala Cys Ile Thr Val Tyr Val Gly Cys Phe Arg Ser Val Lys Asp
           35                  40                  45
   Thr Pro Pro Thr Glu Thr Met Ser Lys Glu His Ala Met Arg Phe Pro
       50                  55                  60
   Leu Val Gly Ser Ala Met Leu Leu Ser Leu Phe Leu Leu Phe Lys Phe
   65                  70                  75                  80
   Leu Ser Lys Asp Leu Val Asn Ala Val Leu Thr Ala Tyr Phe Phe Val
                   85                  90                  95
   Leu Gly Ile Val Ala Leu Ser Ala Thr Leu Leu Pro Ala Ile Arg Arg
               100                 105                 110
   Phe Leu Pro Asn Pro Trp Asn Asp Asn Leu Ile Val Trp Arg Phe Pro
           115                 120                 125
   Tyr Phe Lys Ser Leu Glu Val Glu Phe Thr Lys Ser Gln Val Val Ala
       130                 135                 140
   Gly Ile Pro Gly Thr Phe Phe Cys Ala Trp Tyr Ala Trp Lys Lys His
   145                 150                 155                 160
   Trp Leu Ala Asn Asn Ile Leu Gly Leu Ser Phe Cys Ile Gln Gly Ile
                   165                 170                 175
   Glu Met Leu Ser Leu Gly Ser Phe Lys Thr Gly Ala Ile Leu Leu Ala
               180                 185                 190
   Gly Leu Phe Phe Tyr Asp Ile Phe Trp Val Phe Phe Thr Pro Val Met
           195                 200                 205
   Val Ser Val Ala Lys Ser Phe Asp Ala Pro Ile Lys Leu Leu Phe Pro
       210                 215                 220
   Thr Gly Asp Ala Leu Arg Pro Tyr Ser Met Leu Gly Leu Gly Asp Ile
   225                 230                 235                 240
   Val Ile Pro Gly Ile Phe Val Ala Leu Ala Leu Arg Phe Asp Val Ser
                   245                 250                 255
   Arg Arg Arg Gln Pro Gln Tyr Phe Thr Ser Ala Phe Ile Gly Tyr Ala
               260                 265                 270
   Val Gly Val Ile Leu Thr Ile Val Val Met Asn Trp Phe Gln Ala Ala
           275                 280                 285
   Gln Pro Ala Leu Leu Tyr Ile Val Pro Ala Val Ile Gly Phe Leu Ala
       290                 295                 300
   Ser His Cys Ile Trp Asn Gly Asp Ile Lys Pro Leu Leu Ala Phe Asp
   305                 310                 315                 320
   Glu Ser Lys Thr Glu Glu Ala Thr Thr Asp Glu Ser Lys Thr Ser Glu
                   325                 330                 335
   Glu Val Asn Lys Ala His Asp Glu
               340

## Description

### Field of the invention

This invention relates to an assay. In particular the present invention relates to assays for identifying agents (substances) capable of modulating the interaction between viral proteins capable of binding to intracellular signal peptide peptidase.

### Background to the invention

Hepatitis C virus (HCV) is a major causative agent of chronic hepatitis and liver disease. It is estimated that, worldwide approximately 300 million individuals are infected with the virus. Most of the patients become chronically infected of whom 20% are likely to develop mild to severe liver disease, potentially culminating in either cirrhosis or hepatocellular carcinoma (Di Bisceglie 1998, *Lancet,* **351**, 351-355). Apart from the risk of succumbing to the long term effects of infection, these individuals also harbour a large reservoir of virus for future transmissions.

To date, the only widely used therapy for HCV is treatment with interferon, either alone or in combination with ribovirin. However, sustained response is achieved in less than 50% of cases. Moreover, no vaccine currently exists to protect against infection. Since growth of the virus has not been possible to date in tissue culture systems, our knowledge of the molecular events that arbitrate viral infection, replication and gene expression is limited.

WO95/12677 describes a polypeptide of about 8 to about 100 amino acids comprising or consisting of at least 8 contigous amino acids selected from the core, and/or the E1, and/or the E2, and/or the NS3 regions of the HCV polyprotein with said contiguous amino acids containing a T-cell stimulating epitope.

WO01/21189 describes a fragment consitising of the amino acid sequence SFSIFLLALLSCLTV.

WO00/43505 describes a protease with two aspartate radicals in a catalytically active structure.

WO96/13590 describes HCV type and subtype sequences.

The present invention seeks to provide novel targets for anti-viral agents.

### Summary of the Invention

According to the present invention we have now identified novel anti-viral targets.

In one aspect, we have identified novel anti-HCV targets.

HCV has been classified in a separate genus in the family of *Flaviviridae* and has a positive-sense, single-stranded RNA genome of approximately 9400 nucleotides and contains one open reading frame coding for a single poly-protein precursor of some 3000 amino acids (Choo et al., 1989, *Science,* 244, 359-362; Choo et al., 1991, *PNAS,* 88, 2451-2455; Takamizawa et al., 1991, *J Virol,* 65, 1105-1113).

It is known that HCV core protein can associate with lipid droplets within the cytoplasm of cells (Barba, G. *et al.,* 1997 *PNAS,* **94**, 1200-1205; Moradpour, D*. et al.,* 1996 *Virology,* **222**, 51-63). A defining feature of HCV core protein in mammalian cells after processing of the HCV poly-protein is its association with cytoplamic lipid droplets (Hope and McLauchlan, 2000, *J Gen Virol,* **81**, 1913-1925). HCV-core contains a composit targeting sequence which contains three seperable but interdependent motifs.

It is known that the HCV-poly-protein is initially cleaved. We call that initial cleavage event a primary (or 1⁰) cleavage event. The 1⁰ cleavage event to generate ten individual proteins NH₂-Core-E1-E2-p7-NS2-NS3-NS4A-NS4B-NS5A-NS5B-COOH. NS2-NS5B are putative non-structural proteins involved in the replication of HCV. NS2/3 is a metaloprotease which cleaves *in cis* at the NS2/3 junction. NS3 possesses serine protease and RNA helicase activity and is responsible for the cleavage of the remaining non-structural proteins. NS4A is suggested to be a cofactor for NS3 protease activity. Although the functions of p7, NS4B and NS5A are not well understood, a mutation in NS5A has been linked to susceptibility to interferon (IFN) (Enomoto et al., 1996 *N. Engl. J. Med.* **334**:77-81 ).

All non-structural HCV-encoded proteins are thought to be generated by cleavage by virus encoded proteases.

Core and E1/E2 are considered to be a capsid protein and envelope glycoproteins respectively which are thought to be generated by cleavage by host cell signal peptidases (Hijikita et al., 1991 *PNAS,* **88,** 6647-5551; Reed and Rice, 1998 *Curr. Stud. Hematol. Blood Transf.* **62,** 1-37; Santolini et al., 1994, *J Virol* **68,** 3631-3641; Hussy et al., 1996, *Virology,* **224,** 93-104; Wu L., 2001; *IUBMB Life,* **51,** 19-23; also reviewed in Suzuki *et al.,* 1999, *Intervirology,* **42,** 145-152; McLauchlan J., 2000, *J Viral Hepat* **7**, 2-14).

The 1⁰ cleavage event of the nascent HCV poly-protein results in the compartmentalisation of the mature viral proteins to the endoplasmic reticulum (ER) membrane. It has been proposed that signal peptide processing of the HCV-poly-protein in the ER membrane preludes virion assembly and budding from the ER membrane.

Importantly, and in accordance with the present invention, we have now identified a secondary (or 2⁰) processing/cleavage event of the HCV poly-protein. We believe that this 2⁰ processing/cleavage event has hitherto been unrecognised in the virus kingdom. The 2⁰ processing of the HCV poly-protein as described herein is a consequence of a proteolytic cleavage of the HCV poly-protein by specific cellular signal peptide peptidases (SPPase). Cleavage by SPPase follows signal peptidase cleavage of the HCV poly-protein in a sequential manner. In one aspect, we believe that it is important (e.g. critical) for SPPase cleavage of the HCV polyprotein to occur in order to allow release of the HCV-core from the ER membrane which then localises in the cytoplasm.

The identification of novel enzymes which are an essential component in the life cycle of HCV and virus associated pathology has provided a powerful tool for modulating the underlying molecular events which regulate the processivity of the poly-protein leading to the production of infectious virus particles and thus a much needed therapy for HCV-associated diseases.

In addition, we have also identified a cellular signal peptide peptidase (SPPase) which can process the HCV genome. This SPPase can cleave the poly-protein at a specific signal peptide peptidase site located at the junction of core-E1 sequences. The data presented hereinwith provides compelling evidence that the core-E1 sequence is first cleaved by signal peptidases in the 1⁰ cleavage event, and is then subsequently processed by a cellular SPPase in the 2⁰ cleavage event. We were also successful in finely mapping the peptide sequences cleaved by the cellular signal peptidase, 1⁰ cleavage site, and the cellular SPPase, 2⁰ cleavage site. These are significant findings.

The sequence of the specific SPPase was disclosed in WO-A-01/12660. However, that document disclosed no technical information about the sequence. In particular, no specific function was assigned to the sequence. Moreover, there is no mention of HCV.

A targeting sequence may comprise the signal peptide necessary for translocation/targeting and signal peptide cleavage. In HCV this typically occurs between residues 170-197, more in particular 173-188.

A composite targeting sequence may comprise a lipid droplet binding motif (residues 118-169), signal peptide necessary for translocation/targeting and signal peptide cleavage (residues 170-197), and a sub-fragment of the signal peptide that constitutes the SPPase cleavage site (residues 173-188, see SEQ ID No: 3).

Herein, we show that cleavage of the HCV poly-protein by the signal peptide peptidase (SPPase) enzyme, releases the HCV-core protein from the endoplasmic reticulum (ER) membrane. Following the cleavage event there is subsequent trafficking of core to lipid droplets in the cytosole.

The 'minimum' HCV-core sequence, recognised by the SPPase may be a stretch of at least 8 or more hydrophobic amino acid residues which form a helix comprising for example a group of amino acid residues which may contain but is not limited to the amino acids Leu, Ile, Val, Phe, Trp, Met.

Typically the SPPase sequence also contains at least 1 or more helix breaking residues or residues with small side chains which may contain but is not limited to the amino acids Gly, Ser, Asn, Ala, Cys. Without wishing to be bound by theory, we propose that the purpose of the hydrophobic amino acid stretch is to enter/span the lipid bilayer while the helix-breaking (helix-bending) residues generate flexibility in the peptide backbone, which, as a consequence, becomes accessible for SPPase.

Most importantly, we have identified a specific inhibitor which blocks SPPase processing of core *in vitro,* and provide the first formal demonstration that the core-E1 signal peptide is a specific substrate for SPPase. By mutating the core-E1 signal peptide peptidase signal such that SPPase processing is blocked both *in vivo* and *in vitro,* we demonstrate that this secondary cleavage is important (e.g. vital) for release from ER membranes, which in turn allows subsequent lipid droplet association. Interestingly, we have also shown that failure to release a mutant form of core from ER membrane following SPPase-catalysed proteolysis, does not expose same mutant protein to the degradative processes that act on core.

In the absence of any previous formal definition of the activity that describes cleavage by signal peptide peptidases, without wishing to be bound by theory we propose that SPPase is a presenilin-related, ER-localised aspartyl protease that promotes intra-membrane proteolysis of signal peptides. Thus, the catalysis of HCV-core by SPPase which then promotes core to associate with intracellular lipid globules in the cytosole represents a target for therapies designed to prevent or reduce the effects and/or progression of HCV infection.

The present invention provides a method for identifying a candidate agent capable of affecting a viral infection, which method comprises:
(a) providing a first component comprising a signal peptide peptidase targeting sequence;
(b) providing a second component comprising a signal peptide peptidase;
(c) contacting the two components with an agent to be tested under conditions that would permit the two components to interact in the absence of the agent; and
(d) determining whether the agent modulates the interaction between the first and second components;
wherein the signal peptide peptidase targeting sequence is derivable from hepatitis C virus (HCV)-core protein or a homologue thereof having at least 60% identity thereto.

The term "derivable from" as used herein means that the sequence need not actually be obtained from HCV itself. By way of examples, the sequence may be prepared by recombinant DNA techniques.

Modulation (eg. disruption) by the candidate agent of the interaction between the first and second component is typically indicative that the agent is capable of affecting viral infection.

In a preferred embodiment, the agent to be tested is administered to a cell, the SPPase targeting sequence is expressed in said cell.

In a preferred embodiment, the signal peptide peptidase is a recombinant signal peptide peptidase or natural constituent of said cell.

The method of the invention may further comprise:
(e) administering a virus to a cell in the absence of said agent which has been determined to modulate the interaction between the first and second components;
(f) administering the virus to the cell in the presence of the said agent; and
(g) determining if the said agent reduces or abolishes the susceptibility of the cell to viral infection or the effects of viral infection.

The present invention also provides a method for identifying an agent for treating or preventing a hepatitis infection or other viral infection, which method comprises determining whether said agent can modulate expression of a signal peptide peptidase enzyme in a mammalian cell.

Where cells are used in the methods of the invention, the cells are preferably mammalian cells, more preferably primate cells, for example human cells. The target cells which are infected by the virus of interest are especially preferred for use in the assay methods of the invention. In particular, hepatocytes are especially preferred.

Agents identifiable by the methods of the invention may be used in methods of affecting viral infection, such as treating or preventing viral infection.

There is also described a process comprising identifying an agent capable of affecting viral infection by the method of the present invention and then either preparing a pharmaceutical composition comprising the agent together with a pharmaceutically acceptable carrier or diluent or modifying the agent to alter its antiviral properties and then, optionally comprising a suitably modified agent together with a pharmaceutically acceptable carrier or diluent.

There is also described an agent capable of disrupting an interaction between (i) a SPPase targeting sequence and (ii) a SPPase for use in affecting a viral infection, preferably wherein the targeting sequence is derivable from HCV core protein or a derivative, variant or homologue thereof.

Since we show that HCV core-E1 protein and fragments thereof is processed by SPPase (which in turn affects the trafficking to intracellular lipid globules), a protein comprising HCV core-E1 protein or fragments thereof may be used to reduce or prevent the effect of HCV core-E1 protein produced during the course of HCV infection.

There is also described a protein comprising a SPPase targeting sequence for use in preventing or treating a viral infection wherein the targeting sequence is derivable from HCV core-E1 protein or a derivative, variant or homologue thereof.

Preferably, the HCV core-E1 protein amino acids 173 to 188 (SEQ ID No: 3), are present.

Tthe SPPase targeting sequence may comprise a hydrophilic amino acid sequence of at least 4 amino acids. The SPPase targeting sequence may comprises a hydrophilic amino acid sequence of at least 5 amino acids. The SPPase targeting sequence may comprise a hydrophilic amino acid sequence of at least 6 amino acids. The SPPase targeting sequence may comprise a hydrophilic amino acid sequence of at least 7 amino acids. TheSPPase targeting sequence may comprise a hydrophilic amino acid sequence of at least 8 amino acids.

Agents which modulate the interaction of HCV core protein with intracellular SPPase may interfere with normal function of the core protein. An example of a "normal function" would be its association with lipid droplets, by reducing affinity of SPPase for HCV-core target sequences.

In the following commentary signal peptide peptidase will for convenience be referred to as SPPase. Also, and as used herein, "amino acid" refers to peptide or protein sequence and may refer to portions thereof. In addition, the term "amino acid sequence of the present invention" is synonymous with the phrase "polypeptide of the present invention". Also the term "nucleotide sequence of the present invention" is synonymous with the phrase "poly-nucleotide sequence of the present invention".

### Aspects of the present invention.

According to one aspect of the present invention, there is provided a method for identifying a candidate agent capable of affecting a viral infection, which method comprises:
(a) providing a first component comprising a signal peptide peptidase cleavage targeting sequence;
(b) providing a second component comprising a signal peptide peptidase enzyme;
(c) contacting the two components with an agent to be tested under conditions that would permit the two components to interact in the absence of the agent; and
(d) determining whether the agent modulates the interaction between the first and second components;
wherein the signal peptide peptidase targeting sequence is derivable from hepatitis C virus (HCV)-core protein or a homologue thereof having at least 60% identity thereto.

Another aspect of the present invention provides a method for identifying an agent for treating or preventing a hapatitis infection or other viral infection of the human or animal liver, which method comprises determining whether said agent can modulate expression or activity of a signal peptide peptidase enzyme in a mammalian cell.

In a further aspect, there is provided a protein fragment consisiting of a signal peptide peptidase targeting sequence wherein said fragment consists of SEQ ID No: 3 of the HCV core protein or consists of SEQ ID No: 4, 5 or 6.

### Preferable aspects of the present invention.

As a preferred aspect of the present invention, the candidate agent which modulates the interaction between the signal peptide peptidase targeting sequence and the signal peptide peptidase enzyme can be administered to a cell wherein the signal peptide peptidase cleavage targeting sequence is expressed in said cell.

Preferably, the signal peptide peptidase is a recombinant signal peptide peptidase or a natural constituent of said cell.

Preferably, the method further comprises:
(a) administering a virus to a cell in the absence of said candidate agent which has been determined to modulate the interaction between the first and second components;
(b) administering the virus to the cell in the presence of said agent; and
(c) determining if the said agent reduces or abolishes the susceptibility of the cell to viral infection or the effects of viral infection.

A preferable signal peptide peptidase targeting sequence comprises SEQ ID No. 3 of the HCV-core protein or or a homologue thereof having at least 60% identity thereto.

Preferably, the said cell is a liver cell (hepatocyte).

In a preferred aspect of the present invention the protein fragment is used in preventing or treating a viral infection.

Preferably, the polynucleotide encoding the protein fragment is used in treating a viral infection.

Preferably there is provided the use of the protein fragment or the polynucleotide in the manufacture of a medicament for use in treating or preventing a viral infection.

Preferably, the viral infection is a hepatitis infection or other viral infection of the human or animal liver.

### Detailed Description of the Present Invention

Although in general the techniques mentioned herein are well known in the art, reference may be made in particular to Sambrook *et al.,* Molecular Cloning, A Laboratory Manual (1989) and Ausubel *et al.,* Current Protocols in Molecular Biology (1995), John Wiley & Sons, Inc.

### A. Proteins/Polypeptides

The term "protein" includes single-chain polypeptide molecules as well as multiple-polypeptide complexes where individual constituent polypeptides are linked by covalent or non-covalent means. The term "polypeptide" includes peptides of two or more amino acids in length, typically having more than 5, 10 or 20 amino acids.

### SPPase targeting sequence

The term "SPPase targeting sequence" means an amino acid sequence which is capable of association with SPPase, preferably a biologically occurring SPPase such as an intracellular SPPase. SPPase association may take place within a non-cellular and/or extra-cellular environment, such as in an apparatus - for example a dish, tube, flask or even a vat. Alternatively, it may take place in a cellular environment where the expressed targeting sequence is directed to intracellular SPPase protein.

The SPPase targeting sequence is derived from viruses whose genomic sequence has at least 60% identity to HCV core protein.

Thus, by way of example, from sequence comparisons, the region of the HCV core may be present in the corresponding protein of GB virus-B (GBV-B), a virus whose genomic sequence has significant similarity to HCV. GBV-B still remains unclassified among the genera of Flaviviridae. GBV-B shares a tropism for liver hepatocytes with HCV and is infectious in tamarins. Thus, it has been suggested that GBV-B infection of tamarins may be a surrogate model system for HCV infection of humans *(Virology* (1999) 262, 470-2478). The sequence of the GBV-B core protein is shown in *J. Biol. Chem.* (2002) 277 p4261-4270. An amino acid comparison between the predicted core proteins of HCV and GBV-B illustrates that the amino acids in GBV-B corresponding to positions 173-188 of HCV are: ¹⁴⁰VHLFVVCLLSLACPCSG¹⁵⁶. Advantageously, the GBV-B core protein may be used to identify agents that are capable of affecting a viral infection - such as HCV infection.

A highly preferred signal peptide peptidase targeting sequence consists of of SEQ ID Nos: 3, 4, 5 or 6.

The ability of an amino acid sequence to associate with/target SPPase can be assessed either *in vitro* or *in vivo.* For example, a candidate target sequence may be linked to a protein of interest and introduced into a milk-producing cell in culture and determining whether, the targeting sequence/protein of interest has been secreted into the culture medium. The immuno-cytochemical technique illustrated in the Examples may also be used.

According to the present invention it may be possible to target both the SPPase targeting sequence according to the present invention and sequences responsible for displacing core from intracellular lipid globules and/or reducing the levels of core protein in a cell when expressed in, or administered to, the cell (for illustration see the Examples). Suitable techniques for determining whether a candidate sequence has these properties are described below.

Suitable SPPase targeting sequences are obtained from an HCV core protein. The amino acid sequence of the HCV core protein has been obtained for a large number of different HCV isolates. These sequences are readily available to the skilled person. One such sequence for HCV is strain Glasgow (Hope and McLauchlan J Gen Virol 2000 Aug; 81 Pt 8:1913-1925). The means for cloning and identifying new HCV strains, and thus obtaining further core sequences may be based on those described in EP-B-318,216.

It is preferred to use fragments of the HCV core protein which are capable of targeting molecules, to which they are linked, to SPPase. Amino acid numbering for preferred fragments set out below is with reference to Glasgow strain of HCV. However it will be understood that equivalent fragments of the core protein of other HCV strains/isolates may also be used. An HCV core protein-derivable SPPase targeting sequence of the invention is preferably a minimal amino acid sequence which can also target a molecule, typically a protein, to lipid globules. The minimal sequence will typically comprise a helix-breaking hydrophobic amino acid sequence derived from amino acids 118 to 197 of an HCV core sequence, preferably linked to a hydrophilic amino acid sequence of at least 5, preferably 10, more preferably at least 12 amino acids. It is not necessary for the hydrophilic sequence to be contiguous with the hydrophobic sequence. For example, a protein of interest may be placed between the two sequences such that the hydrophilic sequence is at the N-terminus and the hydrophobic sequence is at the C-terminus.

The hydrophobic amino acid sequence typically comprises at least 8, preferably at least 15 or 20 contiguous amino acids and has a hydropathy index of at least +40 kJ/mol (determined, for example, theoretically as described by Engelman *et al.,* 1986 *Ann Rev Biophys Chem,* **15,** 343). The hydrophilic amino acid sequence typically has a hydropathy plot of less than -20 kJ/mol, preferably less than -40 kJ/mol.

Preferred HCV core fragments contain amino acids 118 to 197.

In an especially preferred embodiment, the SPPase targeting sequence of the invention comprises a hydrophilic amino acid sequence containing amino acids 170 to 197 of the HCV core sequence or a variant, homoloque or a derivative thereof. More preferably, the fragment contains amino acids 173 to 188.

Since it has also now been shown that amino acids 9 to 43, 49 to 75 and 80 to 118 are not required for SPPase processing, preferred HCV core protein fragments need not include one or more of these sequences. Suitable fragments will be at least about 5, e.g. 10, 12, 15 or 20 amino acids in size and preferably have less than 100, 90, 80, 70, 60 or 50 amino acids. In a preferred aspect, the fragments contain an HCV epitope.

SPPase targeting sequences, for example HCV core protein sequences and fragments thereof, may, however, be part of a larger polypeptide, for example a fusion protein. In this case, the additional polypeptide sequences are preferably polypeptide sequences with which the SPPase targeting sequence of the invention is not normally associated.

In the context of SPPase targeting sequence, a homologue is taken to include an amino acid sequence which is at least 60, 70, 80 or 90% identical, preferably at least 95% or 98% identical at amino acid level at least 5, preferably 8, 10, 15, 20, 30 or 40 amino acids with an HCV core protein SPPase targeting sequence, for example as shown in the sequence listing herein. In particular, homology should typically be considered with respect to those regions of the targeting sequence known to be essential for SPPase association rather than non-essential neighbouring sequences. For different homology comparisons methods see below.

### SPPase

As described above, the SPPase enzyme is a presenilin-related, ER localised aspartyl protease that promotes intra-membrane proteolysis of signal peptides.

In another embodiment of the *in vitro* assay methods for the present invention, SPPase may be added to the mixture to compete with a SPPase targeting sequence.

The SPPase protein may be mammalian SPPase or a variant, derivative, homologue or variant thereof.

By way of example, a homologue of the SPPase enzyme is present in the human malaria parasite Plasmodium falciparum. The sequence of this homologue has the accession number 23509765 (*Nature* 419 (6906), 498-511 (2002)). Advantageously, the Plasmodium falciparum SPPase may be used in an assay to identify agents that modulate SPPase in accordance with the present invention that may be useful in the treatment of malaria and/or malaria associated diseases. Background teachings on Malaria are available in the art, for example, in Parassitologia (2002) 44 33-42, Clin. Microbiol Rev. (2002) 15(4):564-94 and Chem. Immunol. (2002) 80:50-69.

The SPPase protein can be obtained in a number of ways for example by purification from mammalian cell lines (Heid et al., 1998, *Cell Tissue Res,* **294**, 309-321). Alternatively, SPPase can be obtained by recombinant means as detailed below.

The sequence of human SPPase is shown in SEQ ID. No. 1.

Polypeptides of the present invention may be in a substantially isolated form. It will be understood that the polypeptide may be mixed with carriers or diluents which will not interfere with the intended purpose of the polypeptide and still be regarded as substantially isolated. A polypeptide of the present invention may also be in a substantially purified form, in which case it will generally comprise the polypeptide in a preparation in which more than 90%, e.g. 95%, 98% or 99% of the polypeptide in the preparation is a polypeptide of the present invention. Polypeptides of the present invention may be modified for example by the addition of histidine residues to assist their purification or by the addition of a signal sequence to promote their secretion from a cell as discussed below.

Polypeptides of the present invention may be produced by synthetic means (e.g. as described by Geysen *et al* 1996 *Chem Biol.* **8:**679-88) or recombinantly, as described below.

Recombinant SPPase may be expressed in a SPPase-negative background - such as a host cell that does not comprises and/or express a nucleotide sequence encoding SPPase.

The amino acid sequence covers the native SPPase (SEQ ID No 1), when it is in its natural environment and when it has been expressed by its native nucleotide coding sequence which is also in its natural environment and when that nucleotide sequence is under the control of its native promoter which is also in its natural environment.

The terms "variant", "homologue" or "fragment" in relation to the amino acid sequence for the enzyme include any substitution of, variation of, modification of, replacement of, deletion of or addition of one (or more) amino acid from or to the sequence providing the resultant enzyme has SPPase activity, preferably being at least as biologically active as the enzyme shown in the attached sequence listings. In particular, the term "homologue" covers homology with respect to structure and/or function. With respect to sequence homology, preferably there is at least 75%, more preferably at least 85%, more preferably at least 90% homology to the sequence shown (see SEQ ID No 1). More preferably there is at least 95%, more preferably at least 98%, homology to the sequence shown (see SEQ ID No 2). More preferably there is at least 75%, more preferably at least 85%, more preferably at least 90% homology to any one of the sequences shown in the attached sequence listings. More preferably there is at least 95%, more preferably at least 98%, homology to any one of the sequence shown as shown in the attached sequence listings.

Typically, the types of amino acid substitutions that could be made should maintain the hydrophobicity/hydrophilicity of the amino acid sequence. Amino acid substitutions may be made, for example from 1, 2 or 3 to 10, 20 or 30 substitutions provided that the modified sequence retains the ability to act as an SPPase enzyme in accordance with the present invention. Amino acid substitutions may include the use of non-naturally occurring analogues, for example to increase blood plasma half-life.

The amino acid sequence of the present invention may be produced by expression of a nucleotide sequence coding for same in a suitable expression system.

In addition, or in the alternative, the protein itself could be produced using chemical methods to synthesise a SPPase amino acid sequence, in whole or in part. For example, peptides can be synthesised by solid phase techniques, cleaved from the resin, and purified by preparative high performance liquid chromatography (e.g., Creighton (1983) Proteins Structures And Molecular Principles, WH Freeman and Co, New York NY). The composition of the synthetic peptides may be confirmed by amino acid analysis or sequencing (e.g., the Edman degradation procedure).

Direct peptide synthesis can be performed using various solid-phase techniques (Roberge JY *et al* (1995) *Science* **269,** 202-204) and automated synthesis may be achieved, for example, using the ABI 43 1 A Peptide Synthesizer (Perkin Elmer) in accordance with the instructions provided by the manufacturer. Additionally, the amino acid sequence of SPPase, or any part thereof, may be altered during direct synthesis and/or combined using chemical methods with a sequence from other subunits, or any part thereof, to produce a variant polypeptide.

A SPPase natural, modified or recombinant sequence may be ligated to a heterologous sequence to encode a fusion protein. For example, for screening of peptide libraries for inhibitors of SPPase activity, it may be useful to encode a chimeric SPPase protein expressing a heterologous epitope that is recognised by a commercially available antibody. A fusion protein may also be engineered to contain a cleavage site located between a SPPase sequence and the heterologous protein sequence, so that the SPPase may be cleaved and purified away from the heterologous moiety.

SPPase may also be expressed as a recombinant protein with one or more additional polypeptide domains added to facilitate protein purification. Such purification facilitating domains include, but are not limited to, metal chelating peptides such as histidine-tryptophan modules that allow purification on immobilised metals (Porath J (1992) *Protein Expr Purif* 3), protein A domains that allow purification on immobilised immunoglobulin, and the domain utilised in the FLAGS extension/affinity purification system (Immunex Corp, Seattle, WA). The inclusion of a cleavable linker sequence such as Factor XA or enterokinase (Invitrogen, San Diego, CA) between the purification domain and SPPase is useful to facilitate purification.

Specific amino acid sequences of SPPase are shown as SEQ ID No. 1. However, the present invention encompasses amino acid sequences encoding other members from the SPPase family which would include amino acid sequences having at least 60% identity (more preferably at least 75% identity) to any one of the amino acid sequences of SEQ ID No 1. As indicated, suitable generic formulae for the SPPase family in accordance with the present invention are presented.

### B. Nucleotide sequence of the present invention.

The term "nucleotide sequence" as used herein refers to an oligonucleotide sequence or polynucleotide sequence. The nucleotide sequence may be DNA or RNA which may be of genomic, synthetic or recombinant origin which may be double-stranded or single-stranded whether representing the sense or antisense strand.

For some embodiments, the term "nucleotide sequence" means DNA. In these instances, preferably, the term "nucleotide sequence" means DNA prepared by use of recombinant DNA techniques (i.e. recombinant DNA).

In a preferred embodiment, the nucleotide sequence *per se* of the present invention does not cover the native nucleotide coding sequence according to the present invention in its natural environment when it is under the control of its native promoter which is also in its natural environment. For ease of reference, we have called this preferred embodiment the "non-native nucleotide sequence".

The nucleotide sequences of the present invention may include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the nucleotide sequences described herein may be modified by any method available in the art. Such modifications may be carried out in order to enhance the *in vivo* activity and/or life span of nucleotide sequences of the present invention.

Nucleotide sequences that are complementary to the sequences presented herein, or any derivative, fragment or derivative thereof are described. If the sequence is complementary to a fragment thereof then that sequence can be used as a probe to identify similar coding sequences in other organisms etc.

Nucleotide sequences that are capable of hybridising to the sequences presented herein, or any derivative, fragment or derivative thereof are described.

Nucleotide sequences that are capable of hybridising to the sequences that are complementary to the sequences presented herein, or any derivative, fragment or derivative thereof are described.

The term "variant" also encompasses sequences that are complementary to sequences that can hybridise to the nucleotide sequences presented herein.

Preferably, the term "variant" encompasses sequences that are complementary to sequences that can hybridise under stringent conditions (e.g. 65°C and 0.1xSSC {1xSSC = 0.15 M NaCl, 0.015 Na₃ citrate pH 7.0}) to the nucleotide sequences presented herein.

Nucleotide sequences that can hybridise to the nucleotide sequences of the present invention (including complementary sequences of those presented herein) are aslo described.

Nucleotide sequences that are complementary to sequences that can hybridise to the nucleotide sequences of the present invention (including complementary sequences of those presented herein) are also described.

Polynucleotide sequences that can hybridise to the nucleotide sequences presented herein under conditions of intermediate to maximal stringency are also described.

Nucleotide sequences that can hybridise to the nucleotide sequence of the present invention, or the complement thereof, under stringent conditions (e.g. 65°C and 0.1xSSC) are also described.

Exemplary nucleic acids can alternatively be characterised as those nucleotide sequences which encode a SPPase protein and hybridise to any one or more of the DNA sequences shown in the attached sequence listings. Preferred are such sequences encoding SPPase sequences which hybridise under high-stringency conditions to any one of the sequences shown in the attached sequence listings or the complement thereof.

Nucleic acid sequences which are capable of hybridising, under stringent conditions, to a fragment of any one of the sequences shown in the attached sequence listings or the complement thereof are described. Preferably, the fragment is between 15 and 50 bases in length. Advantageously, it is about 25 bases in length.

The terms "variant", "homologue" or "fragment" in relation to the nucleotide sequence coding for the SPPase enzyme include any substitution of, variation of, modification of, replacement of, deletion of or addition of one (or more) nucleic acid from or to the sequence providing the resultant nucleotide sequence codes for or is capable of coding for an enzyme having SPPase activity, preferably being at least as biologically active as the enzyme encoded by any one of the sequences shown in the attached sequence listings. In particular, the term "homologue" covers homology with respect to structure and/or function providing the resultant nucleotide sequence codes for or is capable of coding for an enzyme having SPPase activity. With respect to sequence homology, preferably there is at least 75%, more preferably at least 85%, more preferably at least 90% homology to a nucleotide sequence SEQ ID No.2 coding for the amino acid sequence shown in SEQ ID No.1. More preferably there is at least 95%, more preferably at least 98% homology to a nucleotide sequence see SEQ ID No.2 coding for the amino acid sequence shown in SEQ ID No.1. Preferably, with respect to sequence homology, preferably there is at least 75%, more preferably at least 85%, more preferably at least 90% homology to any one of the sequences shown in the attached sequence listings. More preferably there is at least 95%, more preferably at least 98%, homology to any one of the sequences shown in the attached sequence listings.

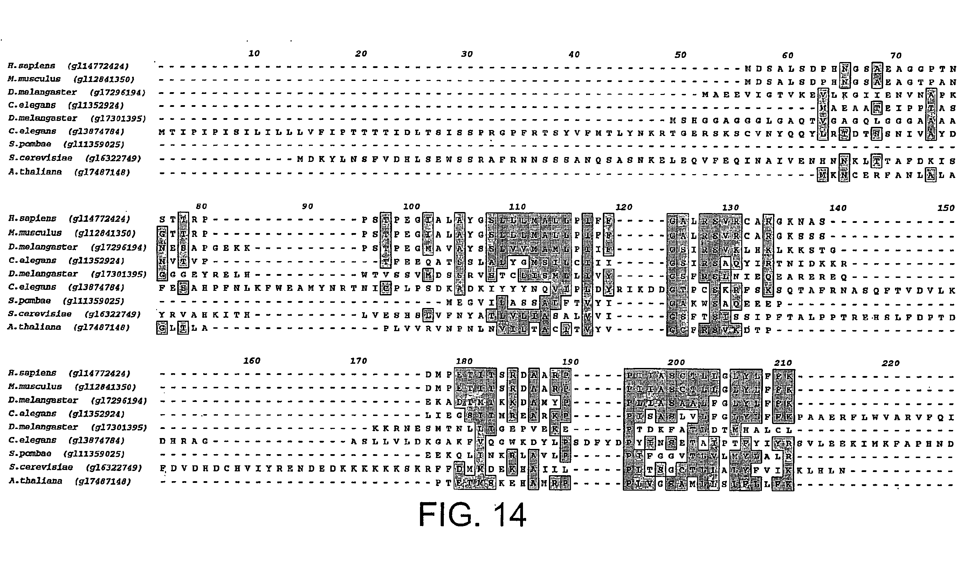

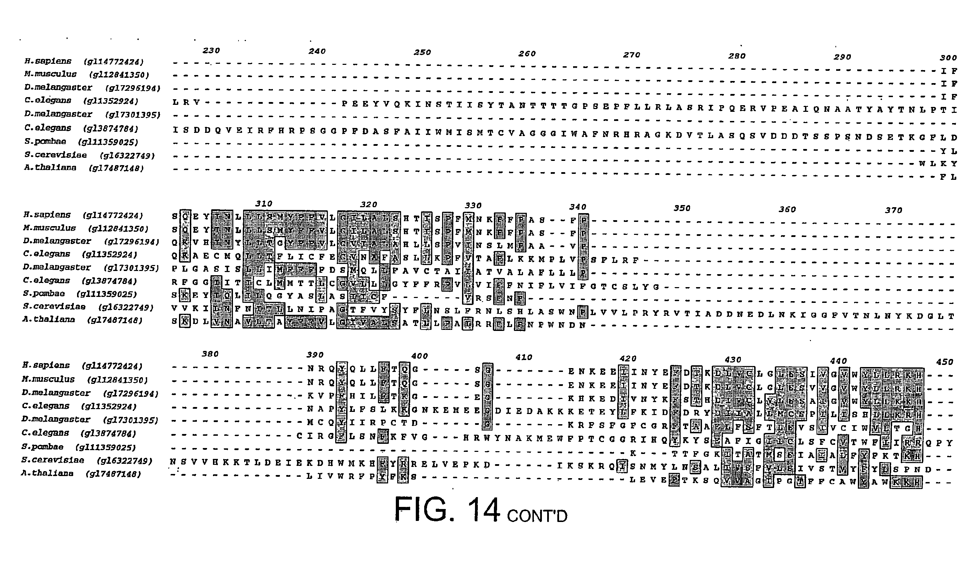

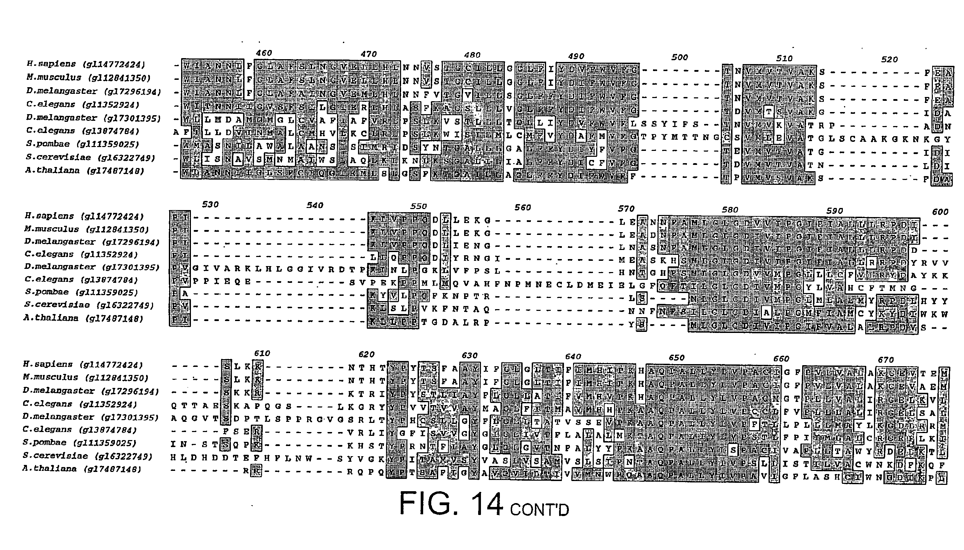

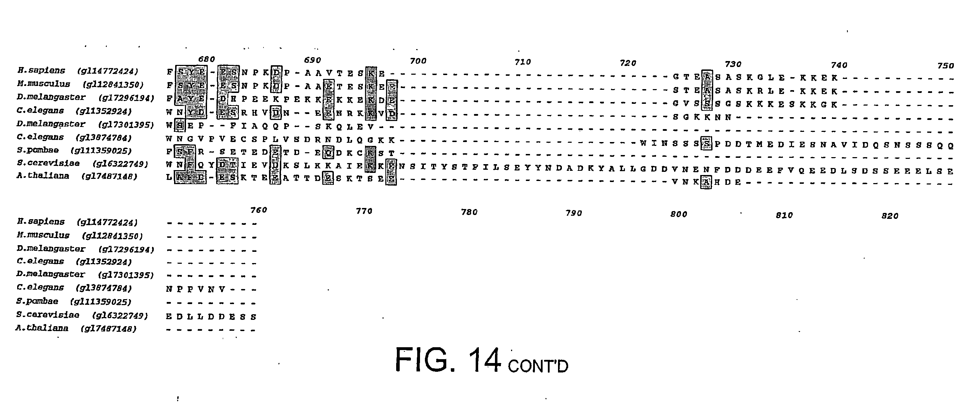

Homologous sequences to the nucleotide sequence coding for the SPPase enzyme are shown in Figure 14 and are also described in *Science* (2002) 296 p2215-2218.

DNA segments comprising the DNA sequence of any one of the sequences shown in the attached sequence listings or allelic variations of such sequences are described.

Polypeptides produced by expression in a host cell into which has been incorporated the foregoing DNA sequences or allelic variations thereof are described.

DNA comprising the DNA sequence of any one of the sequences shown in the attached sequence listings or an allelic variation thereof is described.

Non-native DNA comprising the DNA sequence of any one of the sequences shown in the attached sequence listings or an allelic variation thereof is described.

Recombinant DNA comprising the DNA sequence of any one of the sequences shown in the attached sequence listings or an allelic variation thereof is described.

Polynucleotides of the present invention include nucleic acid sequences encoding the polypeptides of the present invention. It will be appreciated that a range of different polynucleotides encode a given amino acid sequence as a consequence of the degeneracy of the genetic code.

By knowledge of the amino acid sequences set out herein it is possible to devise partial and full-length nucleic acid sequences such as cDNA and/or genomic clones that encode the polypeptides of the present invention. For example, polynucleotides of the present invention may be obtained using degenerate PCR which will use primers designed to target sequences encoding the amino acid sequences presented herein. The primers will typically contain multiple degenerate positions. However, to minimise degeneracy, sequences will be chosen that encode regions of the amino acid sequences presented herein containing amino acids such as methionine which are coded for by only one triplet. In addition, sequences will be chosen to take into account codon usage in the organism whose nucleic acid is used as the template DNA for the PCR procedure. PCR will be used at stringency conditions lower than those used for cloning sequences with single sequence (non-degenerate) primers against known sequences.

Nucleic acid sequences obtained by PCR that encode polypeptide fragments of the present invention may then be used to obtain larger sequences using hybridisation library screening techniques. For example an identified clone may be labelled by PCR with radioactive atoms and used to screen a cDNA or genomic library from other species, preferably other mammalian species. Hybridisation conditions will typically be conditions of medium to high stringency (for example 0.03M sodium chloride and 0.03M sodium citrate at from about 50°C to about 60°C).

Degenerate nucleic acid probes encoding all or part of the amino acid sequence may also be used to probe cDNA and/or genomic libraries from other species, preferably other mammalian species. However, it is preferred to carry out PCR techniques initially to obtain a single sequence for use in further screening procedures.

SPPase polynucleotide sequences which encode SPPase, fragments of the polypeptide, fusion proteins or functional equivalents thereof, may be used to generate recombinant DNA molecules that direct the expression of SPPase in appropriate host cells. Due to the inherent degeneracy of the genetic code, other DNA sequences which encode substantially the same or a functionally equivalent amino acid sequence, may be used to clone and express SPPase. As will be understood by those of skill in the art, it may be advantageous to produce SPPase-encoding nucleotide sequences possessing non-naturally occurring codons. Codons preferred by a particular prokaryotic or eukaryotic host (Murray E *et al* (1989) *Nuc Acids Res* **17**, 477-508) can be selected, for example, to increase the rate of SPPase expression or to produce recombinant RNA transcripts having desirable properties, such as a longer half-life, than transcripts produced from naturally occurring sequence.

Polynucleotide sequences of the present invention obtained using the techniques described above may be used to obtain further homologous sequences and variants using the techniques described above. They may also be modified for use in expressing the polypeptides of the present invention in a variety of host cells systems, for example to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Altered SPPase polynucleotide sequences which may be used in accordance with the invention include deletions, insertions and/or substitutions of different nucleotide residues resulting in a polynucleotide that encodes the same or a functionally equivalent SPPase enzyme. The protein may also have deletions, insertions or substitutions of amino acid residues which produce a silent change and result in a functionally equivalent SPPase enzyme. Deliberate amino acid substitutions may be made on the basis of similarity in polarity, charge, solubility, hydrophobicity, hydrophilicity, and/or the amphipathic nature of the residues as long as the biological activity of SPPase protein is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine (see amino acid Table 1 below).

Included within the scope of the present invention are alleles of SPPase. As used herein, an "allele" or "allelic sequence" is an alternative form of the endogenous SPPase enzyme. Alleles result from a mutation, i.e. a change in the nucleic acid sequence, and generally produce altered mRNAs or polypeptides the structure or function of which may or may not be altered. Any given gene may have none, one or many allelic forms. Common mutational changes which give rise to alleles are generally ascribed to deletions, additions or substitutions of amino acids. Each of these types of changes may occur alone, or in combination with the others, one or more times in a given sequence.

The nucleotide sequences of the present invention may be engineered in order to alter the SPPase coding sequence for a variety of reasons, including but not limited to, alterations which modify the cloning, processing and/or expression of the gene product. For example, mutations may be introduced using techniques which are well known in the art, e.g., site-directed mutagenesis to insert new restriction sites, to alter glycosylation patterns or to change codon preference.

Polynucleotides of the present invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length.

Polynucleotides or primers may carry an identifiable label. Suitable labels include radioisotopes such as ³²P, ³³P or ³⁵S, enzyme labels, or other protein labels such as biotin. Such labels may be added to polynucleotides or primers of the present invention and may be detected using by techniques known *per se.*

Polynucleotides such as a DNA polynucleotide and primers may be produced recombinantly, synthetically, or by any means available to the skilled in the art artisan. They may also be cloned by standard techniques.

In general, primers will be produced by synthetic means, involving a stepwise manufacture of the desired nucleic acid sequence one nucleotide at a time. Techniques for accomplishing this using automated techniques are readily available in the art.

Longer polynucleotides will generally be produced using recombinant means, for example using PCR (polymerase chain reaction) cloning techniques. This will involve making a pair of primers (e.g. preferably between 15 and 30 nucleotides in length) to a region of the nucleotide sequence which it is desired to clone, bringing the primers into contact with mRNA or cDNA obtained from a fungal, plant or prokaryotic cell, performing a polymerase chain reaction under conditions which bring about amplification of the desired region, isolating the amplified fragment (e.g. by purifying the reaction mixture on an agarose gel) and recovering the amplified DNA fragment. The primers may be specifically designed to contain suitable endonuclease restriction sites so that the amplified DNA fragment can be cloned into a suitable cloning vector. DNA molecules may be deliberately modified to increase intracellular stability and half-life, for example, but are not limited to, the addition of flanking sequences of the 5' and/or 3' ends of the molecule or the use of phosphorothioate or 2' O-methyl rather than phosphodiesterase linkages within the backbone of the molecule.

As mentioned earlier, nucleotide sequences that are capable of hybridising to all or part of any one of the sequences shown in the attached sequence listings or an allelic variation thereof are described. These nucleotide sequences may be used in anti-sense techniques, known in the art, to modify SPPase expression. Alternatively, these sequences (or portions thereof) can be used as a probe, or for amplifying all or part of such sequence when used as a polymerase chain reaction primer.

In addition to the recombinant DNA sequences, genomic sequences are also of utility in the context of drug discovery. It may be valuable to inhibit the mRNA transcription of a particular isoform rather than to inhibit its translated protein. This may be true with SPPase, if there are splice variants and wherein those different splice variants may be transcribed from different promoters. There is precedent for multiple promoters directing the transcription of a mouse brain 2',3'-cyclic-nucleotide 3' phosphodiesterase (Kurihara T *et al.,* (1990) *Biochem. Biophys. Res. Comm.* **170,** 1074).

Another utility of the invention is that the DNA sequences, once known, give the information needed to design assays to specifically detect isoenzymes or splice variants. Isozyme-specific PCR primer pairs are but one example of an assay that depends completely on the knowledge of the specific DNA sequence of the isozyme or splice variant. Such an assay allows detection of mRNA for the isozyme to access the tissue distribution and biological relevance of each isozyme to a particular disease state. It also allows identification of cell lines that may naturally express only one isozyme - a discovery that might obviate the need to express recombinant genes. If specific SPPase isozymes are shown to be associated with susceptibility to HCV infection, the invention would be valuable in the design of diagnostic assays to detect the presence of isozyme mRNA.

The coding sequence of SPPase could be synthesised, in whole or in part, using chemical methods well known in the art (see Caruthers MH *et al* (1980) *Nuc Acids Res Symp Ser* 215-23, Horn T *et al* (1980) *Nuc Acids Res Symp Ser* 225-232).

As used herein "naturally occurring" refers to SPPase with an amino acid sequence found in nature.

The terms "isolated" and "purified" as used herein, refer to molecules, either nucleic or amino acid sequences, that are removed from their natural environment and isolated or separated from at least one other component with which they are naturally associated.

"Biologically active" as used herein, refers to SPPase - such as a recombinant SPPase - having a similar structural function (but not necessarily to the same degree), and/or similar regulatory function (but not necessarily to the same degree), and/or similar biochemical function (but not necessarily to the same degree) and/or immunological activity (but not necessarily to the same degree) of the naturally occurring SPPase. Specifically, SPPase has the ability to proteolitically cleave the HCV core, which is one of the characteristic activities of the enzyme.

"Immunological activity" is defined as the capability of the natural, recombinant or synthetic SPPase or any oligopeptide thereof, to induce a specific immune response in appropriate animals or cells and to bind with specific antibodies.

"Derivative" as used herein in relation to the amino acid sequence includes chemical modification of SPPase. Illustrative of such modifications would be replacement of hydrogen by an alkyl, acyl, or amino group.

"Deletion" defines a change in either nucleotide or amino acid sequence in which one or more nucleotides or amino acid residues, respectively, are absent.

As used herein the terms "insertion" or "addition" define a change in a nucleic or amino acid sequence which has resulted in the addition of one or more nucleotides or amino acid residues, respectively, as compared to the naturally occurring SPPase.

The term "substitution" as used herein describes a replacement of one or more nucleotides or amino acids by different nucleotides or amino acids, respectively.

The term "silenced" as used herein describes the process of rendering an enzyme restriction site at least subatnatially inoperable, such as inunrecrognisable by its restriction enzyme. This may be achieved by, but is not limited to, amino acid deletion, insertion or addition, substitution and/or modification.

The term "homologue" as used herein with respect to the nucleotide sequence and the amino acid sequence may be synonymous with allelic variations of the listed sequences.

In particular, the term "homology" as used herein may be equated with the term "identity". Here, sequence homology with respect to the nucleotide sequence and the amino acid sequence can be determined by a simple "eyeball" comparison (i.e. a strict comparison) of any one or more of the sequences with another sequence to see if that other sequence has at least 75% identity to the sequence(s). Relative sequence homology (i.e. sequence identity) can also be determined by commercially available computer programs that can calculate % homology between two or more sequences. A typical example of such a computer program is CLUSTAL.

% homology may be calculated over contiguous sequences, i.e. one sequence is aligned with the other sequence and each amino acid in one sequence directly compared with the corresponding amino acid in the other sequence, one residue at a time. This is called an "ungapped" alignment. Typically, such ungapped alignments are performed only over a relatively short number of residues (for example less than 50 contiguous amino acids).

Although this is a very simple and consistent method, it fails to take into consideration that, for example, in an otherwise identical pair of sequences, one insertion or deletion will cause the following amino acid residues to be put out of alignment, thus potentially resulting in a large reduction in % homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible - reflecting higher relatedness between the two compared sequences - will achieve a higher score than one with many gaps. "Affine gap costs" are typically used that charge a relatively high cost for the existence of a gap and a smaller penalty for each subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package (see below) the default gap penalty for amino acid sequences is -12 for a gap and -4 for each extension.

Calculation of maximum % homology therefore firstly requires the production of an optimal alignment, taking into consideration gap penalties. A suitable computer program for carrying out such an alignment is the GCG Wisconsin Bestfit package (University of Wisconsin, U.S.A.; Devereux *et al.,* 1984, *Nucleic Acids Research* 12, 387). Examples of other software than can perform sequence comparisons include, but are not limited to, the BLAST package (see Ausubel *et al.,* 1999 *ibid -* Chapter 18), FASTA (Altschul *et al.,* 1990, *J. Mol. Biol.,* 215, 403-410) and the GENEWORKS suite of comparison tools. Both BLAST and FASTA are available for off-line and on-line searching (see Ausubel *et al.,* 1999 *ibid,* pages 7-58 to 7-60). However, for some applications it is preferred to use the GCG Bestfit program.

Although the final % homology can be measured in terms of identity, in some cases, the alignment process itself is typically not based on an all-or-nothing pair comparison. Instead, a scaled similarity score matrix is generally used that assigns scores to each pair-wise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see user manual for further details). It is preferred to use the public default values for the GCG package, or in the case of other software, the default matrix, such as BLOSUM62.

Once the software has produced an optimal alignment, it is possible to calculate % homology, preferably % sequence identity. The software typically does this as part of the sequence comparison and generates a numerical result.

In the context of the present invention, a homologous sequence is taken to include an amino acid sequence which is at least 60, 70, 80 or 90% identical, preferably at least 95 or 98% identical at the amino acid level over at least 5, preferably 8, 10, 15, 20, 30 or 40 amino acids with cellular SPPase, for example as shown in the sequence listing herein. In particular, homology should typically be considered with respect to those regions of the targeting sequence known to be essential for SPPase association rather than non-essential neighbouring sequences. Homology comparisons can be conducted by eye, or more usually, with the aid of readily available sequence comparison computer programs. These commercially available computer programs can calculate % homology between two or more sequences. As mentioned above, a typical example of such a computer program is CLUSTAL.

As indicated, for some applications, sequence homology (or identity) may moreover be determined using any suitable homology algorithm, using for example default parameters. Advantageously, the BLAST algorithm is employed, with parameters set to default values. The BLAST algorithm is described in detail at http://www.ncbi.nih.gov/BLAST/blast_help.html. Alternative sequence homology algorithms can be found in http://www.biochem.ucl.ac.uk/bsm/virus_database/bookmark.htm. The search parameters are defined as follows, and are advantageously set to the defined default parameters.

Advantageously, "substantial homology" when assessed by BLAST equates to sequences which match with an EXPECT value of at least about 7, preferably at least about 9 and most preferably 10 or more. The default threshold for EXPECT in BLAST searching is usually 10.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul (see http://www.ncbi.nih.govBLAST/blast_help.html) with a few enhancements. The BLAST programs were tailored for sequence similarity searching, for example to identify homologues to a query sequence. The programs are not generally useful for motif-style searching. Discussion of basic issues in similarity searching of sequence databases have been published (Altschul *et al.,* 1994 *Nature Genetics,* **6,** 119-129).

The five BLAST programs available at http://www.ncbi.nlm.nih.gov perform the following tasks:
**blastp** - compares an amino acid query sequence against a protein sequence database;
**blastn** - compares a nucleotide query sequence against a nucleotide sequence database;
**blastx** - compares the six-frame conceptual translation products of a nucleotide query sequence (both strands) against a protein sequence database;
**tblastn** - compares a protein query sequence against a nucleotide sequence database dynamically translated in all six reading frames (both strands).
**tblastx** - compares the six-frame translations of a nucleotide query sequence against the six-frame translations of a nucleotide sequence database.

BLAST uses the following search parameters:

HISTOGRAM - Display a histogram of scores for each search; default is yes. (See parameter H in the BLAST Manual).

DESCRIPTIONS - Restricts the number of short descriptions of matching sequences reported to the number specified; default limit is 100 descriptions. (See parameter V in the manual page). See also EXPECT and CUTOFF.

ALIGNMENTS - Restricts database sequences to the number specified for which high-scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).

EXPECT - The statistical significance threshold for reporting matches against database sequences; the default value is 10, such that 10 matches are expected to be found merely by chance, according to the stochastic model (Karlin and Altschul 1990 *Proc Natl Acad Sci USA.* **87**(6), 2264-8). If the statistical significance ascribed to a match is greater than the EXPECT threshold, the match will not be reported. Lower EXPECT thresholds are more stringent, leading to fewer chance matches being reported. Fractional values are acceptable (See parameter E in the BLAST Manual).

CUTOFF - Cutoff score for reporting high-scoring segment pairs (HSPs). The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.

MATRIX - Specify an alternate scoring matrix for BLASTP, BLASTX, TBLASTN and TBLASTX. The default matrix is BLOSUM62 (Henikoff & Henikoff, 1992 *Proc Natl Acad Sci USA.* **89**(22), 10915-9). The valid alternative choices include: PAM40, PAM120, PAM250 and IDENTITY. No alternate scoring matrices are available for BLASTN; specifying the MATRIX directive in BLASTN requests returns an error response.

STRAND - Restrict a TBLASTN search to just the top or bottom strand of the database sequences; or restrict a BLASTN, BLASTX or TBLASTX search to just reading frames on the top or bottom strand of the query sequence.

FILTER - Mask off segments of the query sequence that have low compositional complexity, as determined by the SEG program (Wootton and Federhen, 1993 *Computers and Chemistry,* **17**, 149-163), or segments consisting of short-periodicity internal repeats, as determined by the XNU program (Claverie and States 1993 *Computers and Chemistry,* **17**, 191-201) or, for BLASTN, by the DUST program of Tatusov and Lipman (see http://www.ncbi.nlm.nih.gov). Filtering can eliminate statistically significant but biologically uninteresting reports from the blast output (e.g. hits against common acidic-, basic- or proline-rich regions), leaving the more biologically interesting regions of the query sequence available for specific matching against database sequences.

Low complexity sequence found by a filter program is substituted using the letter "N" in nucleotide sequence (e.g., "NNNNNNNNNNNNN") and the letter "X" in protein sequences (e.g., "XXXXXXXXX").

Filtering is only applied to the query sequence (or its translation products), not to database sequences. Default filtering is DUST for BLASTN, SEG for other programs.

It is not uncommon for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.

NCBI-gi causes NCBI gi identifiers to be shown in the output, in addition to the accession and/or locus name.

Most preferably, sequence comparisons are conducted using the simple BLAST search algorithm provided at http://www.ncbi.nlm.nih.gov/BLAST.

Should Gap Penalties be used when determining sequence identity, then preferably the following parameters are used:

| **FOR BLAST** | |
|---|---|
| GAP OPEN | 0 |
| GAP EXTENSION | 0 |

| **FOR CLUSTAL** | DNA | PROTEIN | |
|---|---|---|---|
| WORD SIZE | 2 | 1 | K triple |
| GAP PENALTY | 10 | 10 | |
| GAP EXTENSION | 0.1 | 0.1 | |

Other computer program methods to determine identify and similarity between the two sequences include but are not limited to the GCG program package (Devereux et al 1984 *Nucleic Acids Research* **12**: 387 and FASTA (Alschul *et al.,* 1990 *J Molec Biol.,* **215**, 403-410).

### Polypeptides variants and derivatives

The terms "variant" or "derivative" in relation to the amino acid sequences includes any substitution of, variation of, modification of, replacement of, deletion of or addition of one (or more) amino acids from or to the sequence providing the resultant amino acid sequence has SPPase activity, preferably having at least the same activity as any one of the polypeptides presented in the sequence listings.

The sequences may be modified for use in the present invention. Typically, modifications are made that maintain the signal peptide peptidase cleavage activity of the sequence. Amino acid substitutions may be made, for example from 1, 2 or 3 to 10, 20 or 30 substitutions provided that the modified sequence retains the signal peptide peptidase cleavage activity.

Amino acid substitutions may include the use of non-naturally occurring analogues, for example to increase blood plasma half-life of a therapeutically administered polypeptide provided that the modified sequence retains at least 25% to 50% of, or substantially the same relevant activity.

However, in an alternative embodiment, modifications to the amino acid sequences of the polypeptides, may be made intentionally to reduce the biological activity of the polypeptide. For example truncated polypeptide sequences that remain capable of binding to target molecules but lack functional effector domains may be useful as inhibitors/competitors of the biological activity of the full length molecule.

In general, preferably less than 20%, 10% or 5% of the amino acid residues of a variant or derivative are altered with the corresponding region depicted in the sequence listings.

Conservative substitutions may be made, for example according to the **Table 1** below. Amino acids in the same block in the second column and preferably in the same line in the third column may be substituted for each other:

| | | |
|---|---|---|
| ALIPHATIC | Non-polar | GAP |
| | | ILV |
| | Polar - uncharged | C S T M |
| | | NQ |
| | Polar - charged | D E |
| | | KR |
| AROMATIC | | H F W Y |

Polypeptides also include fragments of the above mentioned full length polypeptides and variants thereof, including fragments of the sequences set out in SEQ ID No: 1. Preferred fragments include those which include an epitope. Suitable fragments will be at least about 5, e.g. 10, 12, 15 or 20 amino acids in length. They may also be less than 200, 100 or 50 amino acids in length. Polypeptide fragments of the proteins and allelic and species variants thereof may contain one or more (e.g. 2, 3, 5, or 10) substitutions, deletions or insertions, including conserved substitutions. Where substitutions, deletion and/or insertions have been made, for example by means of recombinant technology, preferably less than 20%, 10% or 5% of the amino acid residues depicted in the sequence listings are altered.

Polypeptides may be branched as a result of ubiquitination, and they may be cyclic, with or without branching. Cyclic, branched and branched cyclic polypeptides may result from post-translational natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-inks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, *Proteins - Structure and Molecular Properties,* 2nd Ed., T. E. Creighton, W. H. Freeman and Company, New York, 1993 and Wold, F., *Posttranslational Protein Modifications: Perspectives and Prospects,* pgs. 1-12 in *Posttranslational Covalent Modification of Proteins, B.* C. Johnson, Ed., Academic Press, New York, 1983; Seifter *et al.,* 1990 "Analysis for protein modifications and nonprotein cofactors", *Meth Enzymol* **182,** 626-646 and Rattan et al, 1992 "Protein Synthesis: Posttranslational Modifications and Aging", *Ann NY Acad Sci* **668**, 48-62.

As indicated above, proteins are typically made by recombinant means, for example as described herein, and/or by using synthetic means using techniques well known to skilled persons such as solid phase synthesis. Variants and derivatives of such sequences include fusion proteins, wherein the fusion proteins comprise at least the amino acid sequence being linked (directly or indirectly) to another amino acid sequence. These other amino acid sequences - which are sometimes referred to as fusion protein partners - will typically impart a favourable functionality - such as to aid extraction and purification of the amino acid sequence of the present invention. Examples of fusion protein partners include glutathione-S-transferase (GST), 6xHis, GAL4 (DNA binding and/or transcriptional activation domains) and β-galactosidase. It may also be convenient to include a proteolytic cleavage site between the fusion protein partner and the protein sequence of the present invention so as to allow removal of the latter. Preferably the fusion protein partner will not hinder the function of the protein.

### Therapeutic peptides

Peptides may be administered therapeutically to patients. It is preferred to use peptides that do not consist solely of naturally-occurring amino acids but which have been modified, for example to reduce immunogenicity, to increase circulatory half-life in the body of the patient, to enhance bio-availability and/or to enhance efficacy and/or specificity.

A number of approaches have been used to modify peptides for therapeutic application. One approach is to link the peptides or proteins to a variety of polymers, such as polyethylene glycol (PEG) and polypropylene glycol (PPG) - see for example U.S. Patent Nos. 5,091,176, 5,214,131 and US 5,264,209.

Replacement of naturally-occurring amino acids with a variety of uncoded or modified amino acids such as D-amino acids and N-methyl amino acids may also be used to modify peptides.

Another approach is to use bi-functional crosslinkers, such as N-succinimidyl 3-(2 pyridyldithio) propionate, succinimidyl 6-[3-(2 pyridyldithio) propionamido] hexanoate, and sulfosuccinimidyl 6-[3-(2 pyridyldithio) propionamido]hexanoate (see US Patent 5,580,853).

It may be desirable to use derivatives of the peptides which are conformationally constrained. Conformational constraint refers to the stability and preferred conformation of the three-dimensional shape assumed by a peptide. Conformational constraints include local constraints, involving restricting the conformational mobility of a single residue in a peptide; regional constraints, involving restricting the conformational mobility of a group of residues, which residues may form some secondary structural unit; and global constraints, involving the entire peptide structure.

The active conformation of the peptide may be stabilised by a covalent modification, such as cyclization or by incorporation of β-lactam or other types of bridges. For example, side chains can be cyclized to the backbone so as create a L-β-lactam moiety on each side of the interaction site. See, generally, Hruby et al., "Applications of Synthetic Peptides," in Synthetic Peptides: A User's Guide: 259-345 (W. H. Freeman & Co. 1992). Cyclization also can be achieved, for example, by formation of cystine bridges, coupling of amino and carboxy terminal groups of respective terminal amino acids, or coupling of the amino group of a Lys residue or a related homologue with a carboxy group of Asp, Glu or a related homologue. Coupling of the .alpha-amino group of a polypeptide with the epsilon-amino group of a lysine residue, using iodoacetic anhydride, can be also undertaken. See Wood and Wetzel, 1992, *Int'l J. Peptide Protein Res.* **39,** 533-39.

Another approach described in US 5,891,418 is to include a metal-ion complexing backbone in the peptide structure. Typically, the preferred metal-peptide backbone is based on the requisite number of particular co-ordinating groups required by the co-ordination sphere of a given complexing metal ion. In general, most of the metal ions that may prove useful have a co-ordination number of four to six. The nature of the co-ordinating groups in the peptide chain includes nitrogen atoms with amine, amide, imidazole, or guanidino functionalities; sulfur atoms of thiols or disulfides; and oxygen atoms of hydroxy, phenolic, carbonyl, or carboxyl functionalities. In addition, the peptide chain or individual amino acids can be chemically altered to include a co-ordinating group, such as for example oxime, hydrazino, sulfhydryl, phosphate, cyano, pyridino, piperidino, or morpholino. The peptide construct can be either linear or cyclic, however a linear construct is typically preferred. One example of a small linear peptide is Gly-Gly-Gly-Gly which has four nitrogen atoms (an N₄ complexation system) in the back bone that can complex to a metal ion with a co-ordination number of four.

A further technique for improving the properties of therapeutic peptides is to use non-peptide peptidomimetics. A wide variety of useful techniques may be used to elucidating the precise structure of a peptide. These techniques include amino acid sequencing, x-ray crystallography, mass spectroscopy, nuclear magnetic resonance spectroscopy, computer-assisted molecular modelling, peptide mapping, and combinations thereof. Structural analysis of a peptide generally provides a large body of data which comprise the amino acid sequence of the peptide as well as the three-dimensional positioning of its atomic components. From this information, non-peptide peptidomimetics may be designed that have the required chemical functionalities for therapeutic activity but are more stable, for example less susceptible to biological degradation. An example of this approach is provided in US 5,811,512.

Techniques for chemically synthesising therapeutic peptides of the invention are described in the above references and also reviewed by Borgia and Fields, 2000, *TibTech* **18,** 243-251 and described in detail in the references contained therein.

### Polynucleotides variants and derivatives

Polynucleotides of the invention (or for use in the invention) comprise nucleic acid sequences encoding the sequences of the invention. It will be understood by a skilled person that numerous different polynucleotides can encode the same polypeptide as a result of the degeneracy of the genetic code. In addition, it is to be understood that skilled persons may, using routine techniques, make nucleotide substitutions that do not affect the polypeptide sequence encoded by the polynucleotides of the invention to reflect the codon usage of any particular host organism in which the polypeptides of the invention are to be expressed.

Polynucleotides of the invention may comprise DNA or RNA. They may be single-stranded or double-stranded. They may also be polynucleotides which include within them synthetic or modified nucleotides. A number of different types of modification to oligonucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or life span of polynucleotides of the invention.

The terms "variant", "homologue" or "derivative" in relation to the nucleotide sequences include any substitution of, variation of, modification of, replacement of, deletion of or addition of one (or more) nucleic acid from or to the sequence providing the resultant nucleotide sequence codes for a polypeptide having signal peptide peptidase activity, preferably having at least the same activity as the polypeptide sequences presented in the sequence listings.

As indicated above, with respect to sequence homology, preferably there is at least 75%, more preferably at least 85%, more preferably at least 90% homology to the sequences shown in the sequence listing herein. More preferably there is at least 95%, more preferably at least 98%, homology. Nucleotide homology comparisons may be conducted as described above. A preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

Nucleotide sequences that are capable of hybridising selectively to the sequences presented herein, or any variant, fragment or derivative thereof, or to the complement of any of the above are described. Nucleotide sequences are preferably at least 15 nucleotides in length, more preferably at least 20, 30, 40 or 50 nucleotides in length.

The term "hybridization" as used herein shall include "the process by which a strand of nucleic acid joins with a complementary strand through base pairing" as well as the process of amplification as carried out in polymerase chain reaction technologies.

Polynucleotides capable of selectively hybridising to the nucleotide sequences presented herein, or to their complement, will be generally at least 70%, preferably at least 80 or 90% and more preferably at least 95% or 98% homologous to the corresponding nucleotide sequences presented herein over a region of at least 20, preferably at least 25 or 30, for instance at least 40, 60 or 100 or more contiguous nucleotides. Preferred polynucleotides will comprise regions homologous to nucleotides (see SEQ ID No 2), preferably at least 80 or 90% and more preferably at least 95% homologous to see SEQ ID No 2.

The term "selectively hybridizable" means that the polynucleotide used as a probe is used under conditions where a target polynucleotide is found to hybridize to the probe at a level significantly above background. The background hybridization may occur because of other polynucleotides present, for example, in the cDNA or genomic DNA library being screened. In this event, background implies a level of signal generated by interaction between the probe and a non-specific DNA member of the library which is less than 10 fold, preferably less than 100 fold as intense as the specific interaction observed with the target DNA. The intensity of interaction may be measured, for example, by radio-labelling the probe, e.g. with ³²P.

Hybridization conditions are based on the melting temperature (Tm) of the nucleic acid binding complex, as taught in Berger and Kimmel (1987, Guide to Molecular Cloning Techniques, *Methods in Enzymology,* **152**, Academic Press, San Diego CA), and confer a defined "stringency" as explained below.

Maximum stringency typically occurs at about Tm-5°C (5°C below the Tm of the probe); high stringency at about 5°C to 10°C below Tm; intermediate stringency at about 10°C to 20°C below Tm; and low stringency at about 20°C to 25°C below Tm. As will be understood by those of skill in the art, a maximum stringency hybridization can be used to identify or detect identical polynucleotide sequences while an intermediate (or low) stringency hybridization can be used to identify or detect similar or related polynucleotide sequences.

Nucleotide sequences that can hybridise to the nucleotide sequence of the present invention under stringent conditions (e.g. 65°C and 0.1xSSC {1xSSC = 0.15 M NaCl, 0.015 M Na₃ Citrate pH 7.0}) are described.

Where the polynucleotide of the invention is double-stranded, both strands of the duplex, either individually or in combination, are encompassed by the present invention. Where the polynucleotide is single-stranded, it is to be understood that the complementary sequence of that polynucleotide is also included within the scope of the present invention.

Polynucleotides which are not 100% homologous to the sequences of the present invention can be obtained in a number of ways. Other variants of the sequences described herein may be obtained for example by probing DNA libraries made from a range of individuals, for example individuals from different populations. In addition, other viral/bacterial, or cellular homologues particularly cellular homologues found in mammalian cells (e.g. rat, mouse, bovine and primate cells), may be obtained and such homologues and fragments thereof in general will be capable of selectively hybridising to the sequences shown in the sequence listing herein. Such sequences may be obtained by probing cDNA libraries or genomic DNA libraries from animal species, and probing such libraries with probes comprising all or part of SEQ ID No 2 under conditions of medium to high stringency. Similar considerations apply to obtaining species homologues and allelic variants of the polypeptide or nucleotide sequences of SPPase

Variants and strain/species homologues may also be obtained using degenerate PCR which will use primers designed to target sequences within the variants and homologues encoding conserved amino acid sequences within the sequences of the present invention. Conserved sequences can be predicted, for example, by aligning the amino acid sequences from several variants/homologues. Sequence alignments can be performed using computer software known in the art. For example the GCG Wisconsin PileUp program is widely used.

The primers used in degenerate PCR will contain one or more degenerate positions and will be used at stringency conditions lower than those used for cloning sequences with single sequence primers against known sequences.

Alternatively, such polynucleotides may be obtained by site-directed mutagenesis of characterised sequences, such as SEQ ID. No 2. This may be useful where for example silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

Polynucleotides may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will be at least 15, preferably at least 20, for example at least 25, 30 or 40 nucleotides in length, and are also encompassed by the term polynucleotides of the invention as used herein. Preferred fragments are less than 5000, 2000, 1000, 500 or 200 nucleotides in length.

Polynucleotides such as a DNA polynucleotides and probes may be produced recombinantly, synthetically, or by any means available to those of skill in the art. They may also be cloned by standard techniques.

In general, primers will be produced by synthetic means, involving a step wise manufacture of the desired nucleic acid sequence one nucleotide at a time. Techniques for accomplishing this using automated techniques are readily available in the art.

Longer polynucleotides will generally be produced using recombinant means, for example using PCR cloning techniques. This will involve making a pair of primers (e.g. of about 15 to 30 nucleotides) flanking a region of the sequence which it is desired to clone, bringing the primers into contact with mRNA or cDNA obtained from an animal or human cell, performing a polymerase chain reaction under conditions which bring about amplification of the desired region, isolating the amplified fragment (e.g. by purifying the reaction mixture on an agarose gel) and recovering the amplified DNA. The primers may be designed to contain suitable restriction enzyme recognition sites so that the amplified DNA can be cloned into a suitable cloning vector

The terms "variant" or "derivative" in relation to the nucleotide sequences described herein include any substitution of, variation of, modification of, replacement of, deletion of or addition of one (or more) nucleic acid from or to the sequence providing the resultant nucleotide sequence codes for a polypeptide having signal peptide peptidase cleavage activity, preferably having at least the same activity as sequences presented in the sequence listings.

As indicated above, with respect to sequence homology, preferably there is at least 75%, more preferably at least 85%, more preferably at least 90% homology to the sequences shown in the sequence listing herein. More preferably there is at least 95%, more preferably at least 98%, homology. Nucleotide homology comparisons may be conducted as described above. For some applications, a preferred sequence comparison program is the GCG Wisconsin Bestfit program described above. The default scoring matrix has a match value of 10 for each identical nucleotide and -9 for each mismatch. The default gap creation penalty is -50 and the default gap extension penalty is -3 for each nucleotide.

As used herein, the terms "variant", "homologue", "fragment" and "derivative" embrace allelic variations of the sequences.

The term "variant" also encompasses sequences that are complementary to sequences that are capable of hydridising to the nucleotide sequences presented herein.

### Regulatory sequences

Preferably, the polynucleotide of the present invention is operably linked to a regulatory sequence which is capable of providing for the expression of the coding sequence, such as by the chosen host cell. By way of example, a vector comprising the polynucleotide of the present invention operably linked to such a regulatory sequence, i.e. the vector is an expression vector is described.

The term "operably linked" refers to a juxtaposition wherein the components described are in a relationship permitting them to function in their intended manner. A regulatory sequence "operably linked" to a coding sequence is ligated in such a way that expression of the coding sequence is achieved under condition compatible with the control sequences.

The term "regulatory sequences" includes promoters and enhancers and other expression regulation signals.

The term "promoter" is used in the normal sense of the art, e.g. an RNA polymerase binding site.

Enhanced expression of the polynucleotide encoding the polypeptide of the present invention may also be achieved by the selection of heterologous regulatory regions, e.g. promoter, secretion leader and terminator regions, which serve to increase expression and, if desired, secretion levels of the protein of interest from the chosen expression host and/or to provide for the inducible control of the expression of the polypeptide of the present invention

Preferably, the nucleotide sequence of the present invention may be operably linked to at least a promoter.

Besides the promoter native to the gene encoding the polypeptide of the present invention, other promoters may be used to direct expression of the polypeptide of the present invention. The promoter may be selected for its efficiency in directing the expression of the polypeptide of the present invention in the desired expression host.

In another embodiment, a constitutive promoter may be selected to direct the expression of the desired polypeptide of the present invention. Such an expression construct may provide additional advantages since it circumvents the need to culture the expression hosts on a medium containing an inducing substrate.

Examples of strong constitutive and/or inducible promoters which are preferred for use in fungal expression hosts are those which are obtainable from the fungal genes for xylanase *(xln*A*),* phytase, ATP-synthetase, subunit 9 (*oli*C), triose phosphate isomerase *(tpi),* alcohol dehydrogenase *(Adh*A*),* α-amylase (*amy*), amyloglucosidase (AG - from the *gla*A gene), acetamidase (*amd*S) and glyceraldehyde-3-phosphate dehydrogenase (*gpd*) promoters.

Examples of strong yeast promoters are those obtainable from the genes for alcohol dehydrogenase, lactase, 3-phosphoglycerate kinase and triosephosphate isomerase.

Examples of strong bacterial promoters are the α-amylase and *SP02* promoters as well as promoters from extra-cellular protease genes.

Hybrid promoters may also be used to improve inducible regulation of the expression construct.

The promoter can additionally include features to ensure or to increase expression in a suitable host. For example, the features can be conserved regions such as a Pribnow Box, Kozak sequence or a TATA box. The promoter may even contain other sequences to affect (such as to maintain, enhance, decrease) the levels of expression of the nucleotide sequence of the present invention. For example, suitable other sequences include the Sh1-intron or an ADH intron. Other sequences include inducible elements - such as temperature, chemical, light or stress inducible elements. Also, suitable elements to enhance transcription or translation may be present. An example of the latter element is the TMV 5' signal sequence (see Sleat, 1987, *Gene* **217**, 217-225; and Dawson 1993, *Plant Mol. Biol.* **23**, 97).

### Secretion

Often, it is desirable for the polypeptide of the present invention to be secreted from the expression host into the culture medium from where the polypeptide of the present invention may be readily purified. The secretion leader sequence may be selected on the basis of the desired expression host. Additionally, hybrid signal sequences may also be used within the context of the present invention.

Typical examples of heterologous secretion leader sequences are those originating from the fungal amyloglucosidase (AG) gene (*gla*A - both 18 and 24 amino acid versions e.g. from *Aspergillus*), the a-factor gene (yeasts e.g. *Saccharomyces* and *Kluyveromyces*) or the α-amylase gene (*Bacillus*).

### Constructs

The term "construct" - which is synonymous with terms such as "conjugate", "cassette" and "hybrid" - includes the nucleotide sequence according to the present invention directly or indirectly attached to a promoter. An example of an indirect attachment is the provision of a suitable spacer group such as an intron sequence, such as the Sh1-intron or the ADH intron, intermediate the promoter and the nucleotide sequence of the present invention. The same is true for the term "fused" which includes direct or indirect attachment. In each case, the terms do not cover the natural combination of the nucleotide sequence coding for the protein ordinarily associated with the wild type gene promoter and when they are both in their natural environment.

The construct may even contain or express a marker which allows for the selection of the genetic construct in, for example, a bacterium, preferably of the genus Bacillus, such as *Bacillus subtilis,* or plants into which it has been transferred. Various markers exist which may be used, such as for example those encoding mannose-6-phosphate isomerase (especially for plants) or those markers that provide for antibiotic resistance - e.g. resistance to G418, hygromycin, bleomycin, kanamycin and gentamycin.

Preferably the construct comprises at least the nucleotide sequence of the present invention operably linked to a promoter.

### Vectors

The term "vector" includes expression vectors, transformation vectors, shuttle vectors and transduction vectors.

The term "expression vector" means a construct capable of *in vivo* or *in vitro* expression.

The term "transformation vector" means a construct capable of being transferred from one entity to another entity - which may be of the species or may be of a different species. If the construct is capable of being transferred from one species to another - such as from an *E.coli* plasmid to a bacterium, such as of the genus *Bacillus,* then the transformation vector is sometimes called a "shuttle vector". It may even be a construct capable of being transferred from an *E.coli* plasmid to an Agrobacterium to a plant.

The term "transduction vector" defines a retrovirus based vector which has been genetically engineered so that it can not replicate and produce progeny infectious virus particles once the virus has entered the target cell. There are many retroviruses that are widely used for delivery of genes both in tissue culture conditions and in living organisms. Examples include and are not limited to murine leukemia virus (MLV), human immunodeficiency virus (HIV), equine infectious anaemia virus (EIAV), mouse mammary tumour virus (MMTV), Rous sarcoma virus (RSV), Fujinami sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other *retroviridiae* including lentiviruses.

Details on the genomic structure of some lentiviruses may be found in the art. By way of example, details on HIV and EIAV may be found from the NCBI Genbank database (i.e. Genome Accession Nos. AF033819 and AF033820 respectively). Details of HIV variants may also be found at http://hiv-web.lanl.gov. Details of EIAV variants may be found through http://www.ncbi.nlm.nih.gov.

Lentivirus vectors can be engineered either to carry polynucleic acids encoding for a single gene or preferably a single lentivirus vector can be engineered to carry both HCV-core signal peptide peptidase targeting sequence and derivatives thereof and SPPase and derivatives thereof, if there is a need for expression in the same target cell. As an additional embodiment it is also preferable after the first coding sequence to include an internal ribosome entry site (IRES) to initiate translation of the second coding sequence in a poly-cistronic message.

A detailed list of retroviruses may be found in Coffin *et al* ("Retroviruses" 1997 Cold Spring Harbour Laboratory Press Eds: JM Coffin, SM Hughes, HE Varmus pp 758-763).

Lentiviruses also belong to the retrovirus family, but advantageously, they can infect both dividing and non-dividing cells (Lewis et al., 1992 *EMBO J.* **8,** 3053-3058).

The vectors may be for example, plasmid, virus or phage vectors provided with an origin of replication, optionally a promoter for the expression of the said polynucleotide and optionally a regulator of the promoter or an IRES.

All vectors may be specifically designed to carry bi-cistronic polynucleotide sequences coding for proteins of the present invention and a traceable marker such as GFP.

The vectors may be transformed into a suitable host cell as described below under controlled conditions to provide a suitable environment for expression of a polypeptide of the present invention. Thus, there is also provided a process for preparing polypeptides according to the present invention which comprises cultivating a host cell transformed, transfected or transduced with an expression vector as described above under conditions to provide for expression by the vector of a coding sequence encoding the polypeptides, and recovering the expressed polypeptides. Alternatively, vectors comprising the control sequences naturally associated with SPPase gene operably linked to a reporter gene as a reporter construct may be transformed, transfected or transduced into a host cell, for example for use in assay of the invention which measures the effect of candidate agents of transcription from the reporter construct in cellular environment (see Assays below).

Control sequences operably linked to sequences encoding the protein of the invention include promoters/enhancers and other expression regulatory sequences. These control sequences may be selected to be compatible with the host cell for which the expression vector is designed to be used for. The term promoter is well-known in the art e.g. as an RNA polymerase binding site and encompasses nucleic acid regions ranging in size and complexity from minimum promoters to promoters which include upstream regulatory sequences and enhancers.

The promoter is typically selected from promoters which are functional in mammalian cells, although prokaryotic promoters and promoters functional in other eukaryotic cells may be used. The promoter is typically derived from promoter sequences of viral or eukaryotic genes. For example, it may be a promoter derived from the genome of a cell in which expression is to occur. With respect to eukaryotic promoters, they may be promoters that function in a ubiquitous manner (such as promoters of □-actin, □-actin, tubulin) or, alternatively, a tissue-specific manner (such as promoters of the genes for pyruvate kinase). Tissue-specific promoters specific for liver cells are particularly preferred, for example hepatitis B viral promoters, apoliprotein AII promoters, human serum amyloid P component promoters or human protein C gene promoters. There may also be employed promoters that respond to specific stimuli, for example promoters that bind steroid hormone receptors. Viral promoters may also be used, for example the Moloney murine leukaemia virus long terminal repeat (MMLV LTR) promoter, the Rous sarcoma virus (RSV) LTR promoter or the human cytomegalovirus (CMV) IE promoter. As discussed above SPPase promoters are particularly preferred for use in reporter constructs.

It may also be advantageous for the promoters to be inducible so that the levels of expression of the heterologous gene can be regulated during the life-time of the cell. Inducible means that the levels of expression obtained using the promoter can be regulated.

In addition, any of these promoters may be modified by the addition of further regulatory sequences, for example enhancer sequences. Chimeric promoters may also be used comprising sequence elements from two or more different promoters described above.

The vectors may contain one or more selectable marker genes for example an ampicillin resistance gene in the case of a bacterial plasmid or a neomycin resistance gene for mammalian expression vectors.

The preferred selection systems for industrial micro-organisms are those formed by the group of selection markers which do not require a mutation in the host organism. Examples of fungal selection markers are the genes for acetamidase (*amd*S), ATP synthetase, subunit 9 (*oli*C), orotidine-5'-phosphate-decarboxylase (*pvr*A), phleomycin and benomyl resistance (benA). Examples of non-fungal selection markers are the bacterial G418 resistance gene (this may also be used in yeast, but not in filamentous fungi), the ampicillin resistance gene (*E. coli*), the neomycin resistance gene (*Bacillus*) and the *E.coli uid*A gene, coding for β-glucuronidase (GUS).

Vectors may be used *in vitro,* for example for the production of RNA or used to transfect, transform or transduce a host cell.

Thus, polynucleotides can be incorporated into a recombinant vector (typically a replicable vector), for example a cloning or expression vector. The vector may be used to replicate the nucleic acid in a compatible host cell. Thus a method of making polynucleotides of the present invention by introducing a polynucleotide of the present invention into a replicable vector, introducing the vector into a compatible host cell, and growing the host cell under conditions which bring about replication of the vector is described. The vector may be recovered from the host cell. Suitable host cells are described below in connection with expression vectors.

Genetically engineered host cells expressing SPPase protein or variant, homologue, fragment or derivative thereof in screening methods for the identification of inhibitors and antagonists of the SPPase are also described. By way of further example, the use of genetically engineered host cells expressing SPPase protein or variant, homologue, fragment or derivative thereof in screening methods for the identification of inhibitors and antagonists (or combinations of any thereof) of the SPPase that would modulate HCV-core signal peptide peptidase cleavage activity and also affect core trafficking to cytoplasmic lipid globules is also described. Such genetically engineered host cells could be used to screen peptide libraries or an array of organic molecules capable of modulating SPPase activity. Antagonists and inhibitors of SPPase, such as antibodies, peptides or small organic molecules will provide the basis for pharmaceutical compositions for the treatment of HCV associated diseases, by targeting the catalytic activity of SPPase. Such inhibitors or antagonists can be administered alone or in combination with other therapeutics for the treatment of such diseases.

Expression vectors and host cells comprising polynucleotide sequences encoding SPPase or variant, homologue, fragment or derivative thereof for the *in vivo* or *in vitro* production of SPPase protein or to screen for agents that can affect SPPase expression or activity are described.

The term "tissue" as used herein includes tissue *per se* and organ.

The term "host cell" - includes any cell that could comprise the nucleotide sequence coding for the recombinant protein according to the present invention and/or products obtained therefrom, wherein a promoter can allow expression of the nucleotide sequence according to the present invention when present in the host cell. Furthermore, vectors and polynucleotides (the reporter gene constructs described above) may also be introduced into host cells for replicating the vector/polynucleotide.

The Gram-negative bacterium *E*. *coli* is widely used as a host for heterologous gene expression. However, large amounts of heterologous protein tend to accumulate inside the cell in the form of insoluble inclusion bodies which may render purification of proteins of interest from the bulk of *E.coli* intracellular proteins problematic.

In contrast to *E.coli,* the Gram-positive bacteria from the genus *Bacillus* are very suitable as heterologous hosts because of their capability to secrete proteins into the culture medium. Other bacterial species suitable as hosts within the scope of the present invention are the genera of *Streptomyces* and *Pseudomonas.*

A preferred host cell for the expression of mammalian SPPase is *Saccharomyces cerevisiae* as described in *Science* (2002) 296 p2215-2218.

Although the proteins of the present invention may be produced using prokaryotic cells, as outlined above, the desirability for further post-translational modification of the expressed protein renders the eukaryotic cells for example yeast, insect, fungi and in particular mammalian cells more desirable. Particular examples of suitable expression hosts are cells that contain substantial amounts of intracellular lipid deposits/globules, for example adipocytes. Especially preferred cells are those that are naturally targeted by HCV which it is desired to treat, for example liver cells (hepatocytes).

The use of suitable host cells - such as yeast, fungal, plant and mammalian host cells - will provide a suitable environment for post-translational modifications (e.g. myristoylation, glycosylation, sumolation, acetylation, truncation, lipidation and tyrosine, serine or threonine phosphorylation) as may be needed to confer optimal biological activity on recombinant expression products of the present invention.

Where vectors/polynucleotides are to be administered to animals, several techniques are known in the art, for example infection with recombinant viral vectors such as herpes simplex viruses, adenoviruses and lentiviruses, direct injection of nucleic acids and biolistic transformation.

### Protein Expression and Purification

Host cells comprising polynucleotides of the invention may be used to express proteins of the invention. Host cells may be cultured under suitable conditions allowing expression of the proteins of the invention. Expression of the proteins of the invention may be constitutive such that they are continually produced, or inducible, requiring a stimulus to initiate expression. In the case of inducible expression, protein production can be initiated when required by, for example, addition of an inducer agent to the culture medium, for example dexamethasone or IPTG.

Proteins of the invention can be extracted from host cells by a variety of techniques known in the art, including enzymatic, chemical and/or osmotic lysis and physical disruption. Although a large number of different purification protocols may be used, given the ability of the HCV core proteins of the invention to target proteins of interest to lipid globules, a preferred extraction/purification protocol involves centrifuging cell homogenates at high speed (for example 100,000 g for 60 mins at 2 to 4°C) and removing the resulting layer of floating lipids. This will function as a primary purification step. Further purification can then be performed if necessary using, for example, column chromatography such as ion-exchange or affinity chromatography. Cells capable of secreting lipid globules may also conveniently be used and the lipid globules harvested from the culture supernatant.

Proteins associated with the membrane surrounding fat globules can be fractionated into soluble and insoluble fractions by extraction with 1% (w/v) Triton X-100/1.5 M NaCl/10 mM Tris (pH 7.0), by extraction with 1.5% (w/v) dodecyl □-D maltoside/ 0.75 M aminohexanoic acid/10 mM Hepes (pH 7.0) or by sequential extraction with these two detergent-containing solutions (Patton and Huston, 1986 *Lipids* **21**(2):170-4). Suspension of the fat globule components in the detergent-containing solution can be achieved by using an all-glass homogenizer, and keeping on ice for 30 to 60 min, after which insoluble and soluble materials can be separated by centrifugation for 60 min at 2°C and 150,000 g. The above conditions can be modified to analyse whether core protein or a fusion protein containing core as a component is attached to fat globules. Other detergents, both ionic and non-ionic, along with salt solutions at various concentrations could be used to derive the proteinaceous material from fat globules. The incubation times and temperatures may be optimised by empirical means.

### Immunomodulators

Immunomodulators, such as vaccines may be prepared from one or more polypeptides, or even nucleotide sequences, of the present invention. The preparation of immunomodulators which contain an immunogenic polypeptide(s) as active ingredient(s), is known to one skilled in the art. Typically, such immunomodulators are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof.

In addition, if desired, the immunomodulators may contain minor amounts of auxiliary agents such as wetting or emulsifying agents, pH buffering agents, and/or adjuvants which enhance the effectiveness of the immunomodulators. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

Further examples of adjuvants and other agents include aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, *Corynebacterium parvum (Propionobacterium acnes), Bordetella pertussis,* polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

Typically, adjuvants such as Amphigen (oil-in-water), Alhydrogel (aluminum hydroxide), or a mixture of Amphigen and Alhydrogel are used. Only aluminum hydroxide is approved for human use.

The proportion of immunogen and adjuvant can be varied over a broad range so long as both are present in effective amounts. For example, aluminum hydroxide can be present in an amount of about 0.5% of the immunomodulators mixture (Al₂O₃ basis). Conveniently, the immunomodulators are formulated to contain a final concentration of immunogen in the range of from 0.2 to 200 µg/ml, preferably 5 to 50 µg/ml, most preferably 15 µg/ml.

After formulation, the immunomodulators may be incorporated into a sterile container which is then sealed and stored at a low temperature, for example 4°C, or it may be freeze-dried. Lyophilisation permits long-term storage in a stabilised form.

The effectiveness of an adjuvant may be determined by measuring the amount of antibodies directed against an immunogenic polypeptide containing a SPPase antigenic sequence resulting from administration of this polypeptide in immunomodulators which are also comprised of the various adjuvants.

The immunomodulators are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the immunomodulators composition is lyophilised, the lyophilised material may be reconstituted prior to administration, e.g. as a suspension. Reconstitution is preferably effected in buffer

Capsules, tablets and pills for oral administration to a patient may be provided with an enteric coating comprising, for example, Eudragit "S", Eudragit "L", cellulose acetate, cellulose acetate phthalate or hydroxypropylmethyl cellulose.

The polypeptides of the invention may be formulated into the immunomodulators as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric and maleic. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine and procaine.

### Antibodies

Monoclonal or polyclonal antibodies to polypeptides of the invention or fragments thereof are also described. Thus, a process for the production of monoclonal or polyclonal antibodies to polypeptides of the invention is also described.

If polyclonal antibodies are desired, a selected mammal (e.g., mouse, rabbit, goat, horse, etc.) is immunised with an immunogenic polypeptide bearing a SPPase epitope(s). Serum from the immunised animal is collected and treated according to known procedures. If serum containing polyclonal antibodies to an [particular polypeptide] epitope contains antibodies to other antigens, the polyclonal antibodies can be purified by immunoaffinity chromatography. Techniques for producing and processing polyclonal antisera are known in the art. In order that such antibodies may be made, the invention also provides polypeptides of the invention or fragments thereof haptenised to another polypeptide for use as immunogens in animals or humans.

Monoclonal antibodies directed against the polypeptides of the invention can also be readily produced by one skilled in the art. The general methodology for making monoclonal antibodies by hybridomas is well known. Immortal antibody-producing cell lines can be created by cell fusion, and also by other techniques such as direct transformation of B lymphocytes with oncogenic DNA, or transfection with Epstein-Barr virus. Panels of monoclonal antibodies produced against SPPase epitopes can be screened for various properties; i.e., for isotype and epitope affinity.

An alternative technique involves screening phage display libraries where, for example the phage express scFv fragments on the surface of their coat with a large variety of complementarity determining regions (CDRs). This technique is well known in the art.

Antibodies, both monoclonal and polyclonal, which are directed against epitopes are particularly useful in diagnosis, and those which are neutralising are useful in passive immunotherapy. Monoclonal antibodies, in particular, may be used to raise anti-idiotype antibodies. Anti-idiotype antibodies are immunoglobulins which carry an "internal image" of the antigen of the agent against which protection is desired.

Techniques for raising anti-idiotype antibodies are known in the art. These anti-idiotype antibodies may also be useful in therapy.

The term "antibody", unless specified to the contrary, includes fragments of whole antibodies which retain their binding activity for a target antigen. Such fragments include Fv, F(ab') and F(ab')₂ fragments, as well as single chain antibodies (scFv). Furthermore, the antibodies and fragments thereof may be humanised antibodies, for example see description in EP-A-239400.

Antibodies may be used in method of detecting polypeptides of the invention present in biological samples by a method which comprises:
(a) providing an antibody;
(b) incubating a biological sample with said antibody under conditions which allow for the formation of an antibody-antigen complex; and
(c) determining whether an antibody-antigen complex comprising said antibody is formed.

Suitable samples include extracts from tissues such as brain, breast, ovary, lung, colon, pancreas, testes, liver, muscle and bone tissues or from neoplastic growths derived from such tissues.

Antibodies may be bound to a solid support and/or packaged into kits in a suitable container along with suitable reagents, controls, instructions and the like.

### Ribozymes

Ribozymes are enzymatic RNA molecules capable of catalysing the specific cleavage of RNA. The mechanism of ribozyme action involves sequence specific hybridisation of the ribozyme molecule to complementary target RNA, followed by a endonucleolytic cleavage. Engineered hammerhead motif ribozyme molecules that specifically and efficiently catalyse endonucleolytic cleavage of SPPase RNA sequences are described.

Specific ribozyme cleavage sites within any potential RNA target are initially identifiable by scanning the target molecule for ribozyme cleavage sites which include the following sequences, GUA, GUU and GUC. Once identified, short RNA sequences of between 15 and 20 ribonucleotides corresponding to the region of the target gene containing the cleavage site may be evaluated for secondary structural features which may render the oligonucleotide sequence inoperable. The suitability of candidate targets may also be evaluated by testing accessibility to hybridisation with complementary oligonucleotides using ribonuclease protection assays.

Both anti-sense RNA and DNA molecules and ribozymes may be prepared by any method known in the art for the synthesis of RNA, DNA and ribozyme molecules. These include techniques for chemically synthesising oligonucleotides such as solid phase phosphoramidite chemical synthesis. Alternatively, RNA molecules may be generated by *in vitro* or *in vivo* transcription of DNA sequences encoding the anti-sense RNA molecule. Such DNA sequences may be incorporated into a wide variety of vectors with suitable RNA polymerase promoters such as T7 or SP6. Alternatively, anti-sense cDNA constructs that synthesize anti-sense RNA constitutively or inducibly can be introduced into cell lines, cells or tissues.

### Detection

The presence of the SPPase polynucleotide coding sequence can be detected by DNA-DNA or DNA-RNA hybridisation or amplification using probes, portions or fragments of the sequence presented as any one of the sequences shown in the attached sequence listings. Nucleic acid amplification based assays involve the use of oligonucleotides or oligomers based on the SPPase coding sequence to detect transformants containing SPPase DNA or RNA. As used herein "oligonucleotides" or "oligomers" may refer to a nucleic acid sequence of at least about 10 nucleotides and as many as about 60 nucleotides, preferably about 15 to 30 nucleotides, and more preferably about 20 to 25 nucleotides which can be used as a probe or amplimer. Preferably, oligonucleotides are derived from the 3' region of the nucleotide sequence shown as any one of the sequences shown in the attached sequence listings.

A variety of protocols for detecting and measuring the expression of SPPase polypeptide, such as by using either polyclonal or monoclonal antibodies specific for the proteins of the present invention, are known in the art. Examples include and may not be limited to enzyme-linked immunosorbent assay (ELISA), radioimmunoassay (RIA) and fluorescent activated cell sorting (FACS). A two-site, monoclonal-based immunoassay utilising monoclonal antibodies reactive to two non-interfering epitopes on SPPase polypeptides is preferred, although a competitive binding assay may be employed. These and other assays are described, among other places, in Hampton R *et al* (1990, Serological Methods, A Laboratory Manual, APS Press, St Paul MN) and Maddox DE *et al* (1983, *J Exp Med* **15** 8:121 1).
A wide variety of labels and conjugation techniques are known by those skilled in the art and can be used in various nucleic and amino acid assays. Means for producing labelled hybridisation or PCR probes for detecting SPPase polynucleotide sequences include and may not be limited to oligo-labelling, nick translation, end-labelling or PCR amplification using a labelled nucleotide. Alternatively, the SPPase coding sequence, or any portion of it, may be cloned into a vector for the production of an mRNA probe. Such vectors are known in the art, are commercially available, and may be used to synthesise RNA probes *in vitro* by addition of an appropriate RNA polymerase such as T7, T3 or SP6 and labelled nucleotides.

A number of companies such as Amersham-Pharmacia (Piscataway, NJ), Promega (Madison, WI), and US Biochemical Corp (Cleveland, OH) supply commercial kits and protocols for these procedures. Suitable reporter molecules or labels include those radionuclides, enzymes, fluorescent, chemiluminescent, or chromogenic agents as well as substrates, cofactors, inhibitors, magnetic particles and the like. Patents teaching the use of such labels include and are not limited to US-A-3817837; US-A-3850752; US-A-3939350; US-A-3996345; US-A-4277437; US-A-4275149 and US-A-4366241. Also, recombinant immunoglobulins may be produced as shown in US-A-4816567.

Additional methods to quantify the expression of a particular molecule include radio-labelling (Melby PC *et al* 1993, *J Immunol Methods* **159,** 235-44) or biotinylating (Duplaa C *et al.,* 1993, *Anal Biochem* **212,** 229-36) nucleotides, coamplification of a control nucleic acid, and standard curves onto which the experimental results are interpolated. Quantification of multiple samples may be speeded up by running the assay in an ELISA format where the oligomer of interest is presented in various dilutions and a spectrophotometric or calorimetric response gives rapid quantification.

Although the presence/absence of marker gene expression suggests that the gene of interest is also present, its presence and expression should be confirmed. For example, if the SPPase coding sequence is inserted within a marker gene sequence, recombinant cells containing SPPase coding regions can be identified by the absence of marker gene function. Alternatively, a marker gene can be placed in tandem with a SPPase coding sequence under the control of a single promoter of choice. Expression of the marker gene in response to induction or selection is usually indicative of expression of SPPase as well.

Alternatively, host cells which contain the coding sequence for SPPase and express SPPase coding regions may be identified by a variety of procedures known to those of skill in the art. These procedures include, but are not limited to, DNA-DNA or DNA-RNA hybridisation and protein bioassay or immunoassay techniques which include membrane-based, solution-based, or chip-based technologies for the detection and/or quantification of the nucleic acid or protein.

HCV may be detected.

HCV may be detected using a method comprising the steps of: (a) providing a sample - such as a biological sample from a mammal; and (b) detecting one or more cleavage fragments of a signal peptide peptidase cleavage targeting sequence in the sample; wherein, the presence of one or more cleavage fragments of a signal peptide peptidase cleavage targeting sequence in the sample indicates the presence of the HCV virus.

The signal peptide peptidase cleavage targeting sequence may be derivable from the secondary (or 2⁰) processing/cleavage event of the HCV poly-protein as a consequence of a proteolytic cleavage of the HCV poly-protein by SPPase. Accordingly, the signal peptide peptidase cleavage targeting sequence may be derivable from hepatitis C virus (HCV) core protein or a derivative, variant or homologue thereof.

Preferably, the signal peptide peptidase cleavage targeting sequence comprises amino acids 173 to 188 (Seq ID No 3) of the HCV core protein or a mutant, variant or homologue thereof.

One or more cleavage fragments of a signal peptide peptidase cleavage targeting sequence may be detected using an antibody (for example, a monoclonal or a polyclonal antibody, a single chain antibody, a chimeric antibody and a CDR-grafted antibody). In particluar, one or more cleavage fragments of a signal peptide peptidase cleavage targeting sequence may be detected using Western blotting.

### Agents

One or more agents may be identifiable by the assays methods of the present invention.

The agent can be, for example, an organic compound or an inorganic compound. The agent can be, for example, a nucleotide sequence that is anti-sense to all or part of the sequences shown in the attached sequence listings.

The agent may also affect SPPase activity (such as to inhibit, modulate or agonise) in any one or more of the putamen, the Caudate nucleus of the brain, the Occipital lobe of the brain, the heart, ovary, the pituitary gland, kidney, liver, small intestine, thymus, skeletal muscle, leukocyte regions, dorsal root ganglia, uterus, cochlea, small intestine (duodenum), astrocytoma, and appendix (see Examples).

### Probes

Nucleic acid hybridisation or PCR probes which are capable of detecting polynucleotide sequences, including genomic sequences, encoding SPPase coding region or closely related molecules, such as alleles are described. The specificity of the probe, i.e., whether it is derived from a highly conserved, conserved or non-conserved region or domain, and the stringency of the hybridisation or amplification (high, intermediate or low) will determine whether the probe identifies only naturally occurring SPPase coding sequence, or related sequences. Probes for the detection of related nucleic acid sequences are selected from conserved or highly conserved nucleotide regions of cyclic nucleotide SPPase family members, such as the 3' region, and such probes may be used in a pool of degenerate probes. For the detection of identical nucleic acid sequences, or where maximum specificity is desired, nucleic acid probes are selected from the non-conserved nucleotide regions or unique regions of SPPase polynucleotides. As used herein, the term "non-conserved nucleotide region" refers to a nucleotide region that is unique to the SPPase coding sequence disclosed herein and does not occur in related family members.

PCR as described in US-A-4683195, US-A-4800195 and US-A-4965188 provides additional uses for oligonucleotides based upon the SPPase sequence. Such oligomers are generally chemically synthesized, but they may be generated enzymatically or produced from a recombinant source. Oligomers generally comprise two nucleotide sequences, one with sense orientation (5'->3') and one with anti-sense (3'<-5') employed under optimised conditions for identification of a specific gene or condition. The same two oligomers, nested sets of oligomers, or even a degenerate pool of oligomers may be employed under less stringent conditions for detection and/or quantification of closely related DNA or RNA sequences.

The nucleic acid sequence for SPPase can also be used to generate hybridisation probes as previously described, for mapping the endogenous genomic sequence. The sequence may be mapped to a particular chromosome or to a specific region of the chromosome using well known techniques. These include *in situ* hybridisation to chromosomal spreads (Verma *et al* 1988, Human Chromosomes: A Manual of Basic Techniques, Pergamon Press, New York City), flow-sorted chromosomal preparations, or artificial chromosome constructions such as YACs, bacterial artificial chromosomes (BACs), bacterial PI constructions or single chromosome cDNA libraries.

*In situ* hybridisation of chromosomal preparations and physical mapping techniques such as linkage analysis using established chromosomal markers are invaluable in extending genetic maps. Examples of genetic maps can be found in *Science* (1995; **270**:41 Of and 1994; **265:**1981 f). Often the placement of a gene on the chromosome of another mammalian species may reveal associated markers even if the number or arm of a particular human chromosome is not known. New sequences can be assigned to chromosomal arms, or parts thereof, by physical mapping. This provides valuable information to investigators searching for disease genes using positional cloning or other gene discovery techniques. Once a disease or syndrome, such as *ataxia telangiectasia* (AT), has been crudely localised by genetic linkage to a particular genomic region, for example, AT to 11q22-23 (Gatti *et al* 1988, *Nature* **336:**577-580), any sequences mapping to that area may represent associated or regulatory genes for further investigation. The nucleotide sequence of the subject invention may also be used to detect differences in the chromosomal location due to translocation, inversion, etc between normal, carrier or affected individuals.

### Pharmaceuticals

A pharmaceutical composition for treating an individual in need of same due to SPPase activity, the composition comprising a therapeutically effective amount of an agent that affects (such as inhibits) said activity and a pharmaceutically acceptable carrier, diluent, excipient or adjuvant is described.

Pharmaceutical compositions comprising the agents described herein (an agent capable of modulating the expression pattern of the nucleotide sequence of the present invention or the activity of the expression product thereof and/or an agent identifiable by an assay according to the present invention). In this regard, and in particular for human therapy, even though the agents can be administered alone, they will generally be administered in admixture with a pharmaceutical carrier, excipient or diluent selected with regard to the intended route of administration and standard pharmaceutical practice.

By way of example, in the pharmaceutical compositions, the agents may be admixed with any suitable binder(s), lubricant(s), suspending agent(s), coating agent(s), solubilising agent(s) or absorbtion increasing agent(s).

In general, a therapeutically effective daily oral or intravenous dose of the agents of the present invention is likely to range from 0.01 to 50 mg/kg body weight of the subject to be treated, preferably 0.1 to 20 mg/kg. The agents may also be administered by intravenous infusion, at a dose, which is likely to range from 0.001-10 mg/kg/hr.

Tablets or capsules of the agents may be administered singly or two or more at a time, as appropriate. It is also possible to administer the agents in sustained release formulations.

Thus, a method of treating an individual in need of same due to SPPase activity comprising administering to said individual an effective amount of the pharmaceutical composition is described.

Typically, the physician will determine the actual dosage which will be most suitable for an individual patient and it will vary with the age, weight and response of the particular patient. The above dosages are exemplary of the average case. There can, of course, be individual instances where higher or lower dosage ranges are merited.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose, or in capsules or ovules either alone or in admixture with excipients, or in the form of elixirs, solutions or suspensions containing flavouring or colouring agents, or they can be injected parenterally, for example intracavemosally, intravenously, intramuscularly or subcutaneously. For parenteral administration, the compositions may be best used in the form of a sterile aqueous solution which may contain other agents, for example enough salts or monosaccharides to make the solution isotonic with blood. For buccal or sublingual administration the compositions may be administered in the form of tablets or lozenges which can be formulated in a conventional manner.

For certain applications, the preferred administration of the composition is orally in the form of tablets containing excipients such as starch or lactose, or in capsules or ovules either alone or in admixture with excipients, or in the form of elixirs, solutions or suspensions containing flavouring or colouring agents.

For parenteral administration, the compositions are best used in the form of a sterile aqueous solution which may contain other agents, for example enough salts or monosaccharides to make the solution isotonic with blood.

For buccal or sublingual administration the compositions may be administered in the form of tablets or lozenges which can be formulated in a conventional manner.

For oral, parenteral, buccal and sublingual administration to subjects (such as patients), the daily dosage level of the agents of the present invention may typically be from 10 to 500 mg (in single or divided doses). Thus, and by way of example, tablets or capsules may contain from 5 to 100 mg of active agent for administration singly, or two or more at a time, as appropriate. As indicated above, the physician will determine the actual dosage which will be most suitable for an individual patient and it will vary with the age, weight and response of the particular patient. It is to be noted that whilst the above-mentioned dosages are exemplary of the average case there can, of course, be individual instances where higher or lower dosage ranges are merited and such dose ranges are within the scope of this invention.

In some applications, generally, in humans, oral administration of the agents of the present invention is the preferred route, being the most convenient and can in some cases avoid disadvantages associated with other routes of administration - such as those associated with intracavernosal (i.c.) administration. In circumstances where the recipient suffers from a swallowing disorder or from impairment of drug absorption after oral administration, the drug may be administered parenterally, e.g. sublingually or buccally.

For veterinary use, the agent is typically administered as a suitably acceptable formulation in accordance with normal veterinary practice and the veterinary surgeon will determine the dosing regimen and route of administration which will be most appropriate for a particular animal. However, as with human treatment, it may be possible to administer the agent alone for veterinary treatments.

Typically, the pharmaceutical compositions - which may be for human or animal usage - will comprise any one or more of a pharmaceutically acceptable diluent, carrier, excipient or adjuvant. The choice of pharmaceutical carrier, excipient or diluent can be selected with regard to the intended route of administration and standard pharmaceutical practice. As indicated above, the pharmaceutical compositions may comprise as - or in addition to - the carrier, excipient or diluent any suitable binder(s), lubricant(s), suspending agent(s), coating agent(s), solubilising agent(s).

The pharmaceutical compositions will comprise one or more of: an agent that has been screened by an assay of the present invention; an agent that is capable of interacting with any one of the sequences shown in the attached sequence listings including derivatives, fragments, homologues or variants thereof or sequences capable of hybridising to any one of the sequences shown in the attached sequence listings.

Oligonucleotide sequences, anti-sense RNA and DNA molecules and ribozymes, which function to destabilise SPPase mRNA or inhibit translation of SPPase protein are described. Such nucleotide sequences may be used in conditions where it would be preferable to increase SPPase nucleotide levels, such as in HCV infection.

A SPPase anti-sense molecule may provide the basis for treatment of various abnormal conditions related to, for example, increased SPPase activity.

A SPPase nucleic acid anti-sense molecule may be used to block the activity of the SPPase enzyme in conditions where it would be preferable to elevate SPPase nucleotide levels.

Expression vectors derived from retroviruses, adenovirus, herpes or vaccinia viruses, or from various bacterial plasmids, may be used for delivery of recombinant SPPase sense or anti-sense molecules to the targeted cell population. Methods which are well known to those skilled in the art can be used to construct recombinant vectors containing SPPase. Alternatively, recombinant SPPase can be delivered to target cells in liposomes.

The full length cDNA sequence and/or its regulatory elements enable researchers to use SPPase as a tool in sense (Youssoufian H and HF Lodish 1993 *Mol Cell Biol* **13**:98-104) or anti-sense (Eguchi *et al* 1991, *Annu Rev Biochem* **60**, 631-652) investigations of gene function. Oligonucleotides, designed from the cDNA or control sequences obtained from the genomic DNA can be used *in vitro* or *in vivo* to inhibit expression. Such technology is now well known in the art, and sense or anti-sense oligonucleotides or larger fragments can be designed from various locations along the coding or control regions. Appropriate oligonucleotides, which can be 20 nucleotides in length, may be used to isolate SPPase sequences or closely related molecules from human libraries.

Additionally, SPPase expression can be modulated by transfecting a cell or tissue with expression vectors which express high levels of a SPPase fragment in conditions where it would be preferable to block signal peptide peptidase activity thereby increasing the levels of HCV-core in the ER membrane (see Results). Such constructs can flood cells with untranslatable sense or antigens sequences. Even in the absence of integration into the DNA, such vectors may continue to transcribe RNA molecules until all copies of the vector are disabled by endogenous nucleases. Such transient expression may last for a month or more with a non-replicating vector and even longer if appropriate replication or integration elements are part of the vector system such as the use of lentiviruses which have the capacity to become integrated in the host cell genome.

Modifications of gene expression can be obtained by designing anti-sense sequences to the control regions of the SPPase gene, such as the promoters, enhancers, and introns.

Oligonucleotides derived from the transcription initiation site, e.g., between -10 and +10 regions of the leader sequence, are preferred. Anti-sense RNA and DNA molecules may also be designed to block translation of mRNA by preventing the transcript from binding to ribosomes. Similarly, inhibition can be achieved using Hogeboom base-pairing methodology, also known as "triple helix" base pairing. Triple helix pairing compromises the ability of the double helix to open sufficiently for the binding of polymerases, transcription factors, or regulatory molecules.

Thus a pharmaceutical composition comprising an agent (or even a pharmaceutically acceptable salt thereof, or a pharmaceutically acceptable solvate thereof) together with a pharmaceutically acceptable diluent, excipient or carrier is described.

The pharmaceutical composition could be for veterinary (i.e. animal) usage or for human usage.

Pharmaceutical compositions comprising effective amounts of inhibitors or antagonists of the SPPase protein (including anti-sense nucleic acid sequences) in admixture with a pharmaceutically acceptable diluent, carrier, excipient or adjuvant (including combinations thereof) are described.

Pharmaceutical compositions which may comprise all or portions of SPPase polynucleotide sequences, SPPase anti-sense molecules, SPPase polypeptides, protein, peptide or organic modulators of SPPase bio-activity, such as inhibitors, antagonists (including antibodies) or agonists, alone or in combination with at least one other agent, such as stabilising compound, and may be administered in any sterile, biocompatible pharmaceutical carrier, including, but not limited to, saline, buffered saline, dextrose, and water are described.

### General Assays

### 1. Assays for agents that disrupt the interaction between a HCV-core signal peptide peptidase targeting sequence and a SPPase

### i. Candidate agents

An agent which disrupts an interaction between the HCV-core signal peptide peptidase targeting sequence and SPPase may do so in several ways. It may directly disrupt the binding of the HCV-core signal peptide peptidase targeting sequence to SPPase by, for example, binding to the HCV-core signal peptide peptidase targeting sequence and masking or altering the site of interaction with SPPase. Alternatively, the candidate agent may compete for binding sites on the surface of SPPase and displace the HCV-core signal peptide peptidase targeting sequence. Candidate agents of these types may conveniently be screened by *in vitro* binding assays as, for example, described below. Candidate agents may also be screened using an *"in vivo"* whole cell assay as described below. The term *'in vivo'* is intended to encompass experiments with tissue culture cells in laboratory conditions as well as experiments with intact multicellular organisms.

An agent which can bind directly to the HCV-core signal peptide peptidase targeting sequence may also inhibit an interaction between the HCV-core signal peptide peptidase targeting sequence and the SPPase by altering the subcellular localisation of the HCV-core signal peptide peptidase targeting sequence thus preventing the two components from coming into contact within the cell. This can also be tested *in vivo* using, for example the *in vivo* assays described below.

Alternatively, instead of preventing the association of the components directly, the agent may suppress or enhance the biologically available amount of HCV-core signal peptide peptidase targeting sequence. This may be by inhibiting expression of the HCV-core signal peptide peptidase targeting sequence, for example at the level of transcription, transcript stability, translation, post-translational processing or post-translational stability. An example of such an agent would be anti-sense RNA which suppresses the amount of HCV core protein mRNA translated into protein.

Suitable candidate agents include viral peptides, which in some cases may especially be of size from about 5 to 20 amino acids, based on, for example, HCV-core signal peptide peptidase targeting motifs found within the HCV core protein, or variants of such peptides in which one or more residues have been substituted (see Sequence Listings). Peptide fragments of SPPase may also be used, including modified variants thereof. Peptides from panels of peptides comprising random sequences or sequences which have been varied consistently to provide a maximally diverse panel of peptides may be used.

Suitable candidate agents also include antibody products (for example, monoclonal and polyclonal antibodies, single chain antibodies, chimeric antibodies and CDR-grafted antibodies) which are specific for the HCV-core SPPase targeting sequence or surface constituents of SPPase. Furthermore, combinatorial libraries, peptide and peptide mimetics, defined chemical entities such as organic and inorganic compounds, oligonucleotides, and natural product libraries may be screened for activity as inhibitors of an interaction between the HCV-core SPPase targeting sequence and a SPPase in assays such as those described below. The candidate agents may be used in an initial screen in batches of, for example 10 agents per reaction, and the agents of those batches which show inhibition tested individually. Candidate agents which show activity in *in vitro* screens such as those described below can then be tested in *in vivo* systems, such as mammalian cells which will be exposed to the inhibitor and tested, for example, for susceptibility to viral infection.

### ii. Assays

The assays of the invention may be *in vitro* assays or *in vivo* assays, for example using cell lines or an animal model, such as mice or chimpanzees.

### In vitro assay systems

One type of *in vitro* assay for identifying agents which disrupt an interaction between the HCV-core SPPase targeting sequence and SPPase involves measuring in the presence or absence of a candidate agent the association of a targeting sequence with SPPase formed *in vitro.* As described above, a defining feature of core protein in mammalian cells after processing from the HCV polyprotein is its association with cytoplasmic lipid droplets (Hope and McLauchlan, 2000 *J Gen Virol.* **81**, 1913-25), thus identifying agents which disrupt an interaction between HCV-core SPPase targeting sequence and SPPase can be determined by testing the level of association of HCV-core with lipid globules formed *in vitro.* Binding of HCV-core SPPase targeting sequences to SPPase may be assessed by adding a targeting sequence to a dispersion of lipid globules (such as a mixture of phospholipid and triacylglycerol) in an aqueous solvent, sonicating the mixture and determining by fractionation the degree of partition between aqueous and lipid phases. Typically fractionation of the mixture would involve increasing the density of the solution with sorbitol or sodium bromide and ultracentrifuging the solution. The lipid complexes migrate to the top of the centrifuge tube and this upper lipid layer is then examined for HCV-core. This is performed in the presence or absence of the candidate agent whose inhibitory activity it is desired to test. Typically, a candidate agent is deemed to inhibit the HCV-core SPPase targeting sequence if it causes a reduction of at least 50%, preferably at least 60, 70, 80 or 90% in the amount of HCV-core SPPase targeting sequence associated with the lipid phase in the absence of the candidate agent.

The candidate agent may be pre-incubated with the HCV-core SPPase targeting sequence or with SPPase or added to the reaction mixture after pre-incubation of the HCV-core SPPase targeting sequence with SPPase.

Another type of *in vitro* assay for identifying agents which disrupt an interaction between the HCV-core SPPase targeting sequence and SPPase or a lipophilic surface involves the following:

Proteins incorporating the SPPase targeting sequence, for example a fragment of HCV-core protein, and optionally SPPase, may be translated *in vitro* from RNA transcripts encoding these polypeptides. Reactions would be supplemented with membranes originating from tissue culture cells and tissues (e.g. ER derived rough microsomal membranes) or a lipophilic surface which had been artificially created (e.g. a liposome) to which the *in vitro* translated proteins can bind. Binding may be assessed by purification of the lipid components of reactions (e.g. by centrifugation). Alternatively a chip format (for example BIAcore^{™} - Biacore AB) wherein the chip has a lipid surface, may be used to measure binding.

Alternatively, proteins incorporating the SPPase targeting sequence such as for example core-E1/100 or SP-E1/100 using intact microsomes and combine it with carbonate extraction of membranes to determine whether SPPase has processed the core signal peptide. Carbonate dependent release of core into the supernatant requires that core is cleaved by SPPase which can be detected by for example Western blot analysis (for details see Results, Section 4).

A high throughput *in vitro* assay for identifying agents which may disrupt the interaction between the HCV-cote targeting sequence and SPPase enzyme involves the following:

Detergent-solublilised or recombinant SPPase enzyme is incubated with short synthetically generated proteins which carry HCV-core SPPase targeting sequences with tags positioned at the N- and C-termini of the protein. Preferrably the two tags are flourescent moiety tags which are partners for a fluorescent resonance energy transfer (FRET) based assay. The assay is performed in the presence and absence of candidate agent. Thus FRET would be detected if SPPase does not cleave the polypeptide substrate (the candidate agent blocks the interaction between SPPase and the substrate), however energy transfer would be lost upon cleavage (the candidate agent is unable to block SPPase cleavageof the substrate) (for details of how to obtain detergent-soluble SPPase see Sections 10 and 11b in the Maretial and Methods and Section 5 in the Results).

The ability of candidate agents to disrupt association of the HCV-core signal peptide and SPPase which affects the association of HCV-core with lipid globules can be examined by adding to reactions candidate agents during *in vitro* synthesis of the proteins. Preferably, a suitable inhibitor of SPPase targeting sequence is capable of inhibiting binding of HCV-core SPPase targeting sequences to lipid globules by specifically affecting the interaction between SPPase and HCV-core SPPase targeting sequence.

It is known that deleting HCV-core residues 125 to 144 (hydrophobic region) abolishes HCV-core association with lipid droplets and leads to its degradation by proteosome in the cytosole upon release from core-E1-E2 (Hope and McLauchlan, 2000 *J Gen Virol.* 2000 Aug;81 Pt 8:1913-25). Thus blocking cleavage of HCV-core-E1-E2 by SPPase can reduce degradation since immature core protein would be retained at the ER membrane (For detailed description see Example 4). Therefore another *in vitro* type assay testing for inhibitors of specific cleavage of HCV-core-E1-E2 by SPPase is to measure the accumulation of HCV-core protein retained in the ER membrane. Thus the accumulation of the HCV-core with ER membranes is determined in the absence of the candidate agent and in the presence of the candidate agent and the results compared. Suitable procedures are described in the Examples.

SPPase - such as recombinant SPPase - may also be assayed using the methods described in Science (2002) 296, 2215-2218.

### In vivo assay systems

*In vivo* assays for identifying compounds that disrupt an interaction between the HCV-core SPPase targeting sequence and SPPase and thus trafficking of HCV-core to lipid globules typically involve administering a candidate agent to a cell which expresses SPPase targeting sequence, preferably originating from HCV-core protein or a derivative thereof, and testing whether modulating the interaction between the SPPase targeting sequence and SPPase can alter the intracellular lipid globule association, for example reduce or abolish. Thus the association of the HCV-core with intracellular lipid globules is determined in the absence of the candidate agent and in the presence of the candidate agent and the results compared.

Association of the SPPase targeting sequence with SPPase is typically determined by immunofluorescence microscopy using an antibody which recognizes a sequence that is located before the SPPase targeting sequence and an antibody or stain which recognizes intracellular SPPase or a surface component of the lipid globules. Since we show that proteolytic cleavage of HCV-core protein by SPPase is critical for trafficking of core to lipid globules, the co-localization of cleaved core with lipid globules may also be determined in the presence and absence of the candidate agent.

A candidate agent is generally considered to be capable of disrupting the interaction between the HCV-core SPPase targeting sequence and intracellular SPPase if, as determined by immunofluorescence microscopy, less than 50% of the HCV-core protein co-localizes with intracellular lipid globules, preferably less than 60%, more preferably less than 70, 80 or 90%. Preferably, a suitable inhibitor of HCV-core SPPase targeting sequence is capable of disrupting the interaction of core targeting sequences with intracellular SPPase without affecting the interaction of core with intracellular lipid globules/surface components..

It will be appreciated by the skilled artisan that other techniques are available for determining localization of proteins and lipids within intact cells and that these techniques are also applicable to the assays of the present invention (for examples also see Description).

The candidate agent, i.e. the test compound, may be administered to the cell in one or more ways. For example, it may be added directly to the cell culture medium or injected into the cell using micro-manipulation technologies. Alternatively, in the case of polypeptide candidate agents, the cell may be transfected with a nucleic acid construct which directs expression of the polypeptide in the cell. Preferably, the expression of the polypeptide is under the control of a regulatable promoter, for example so that expression is induced shortly before it is desired to administer the candidate agent.

Another suitable test may involve introducing a polynucleotide encoding a targeting sequence of the invention, optionally linked to a protein of interest, into a milk-producing cell in culture and determining whether, the targeting sequence/protein of the present invention has been secreted into the culture medium. This would be carried out in the presence or absence of the candidate agent.

### 2. Assays for agents that are capable of modulating SPPase expression in cells

### i. Candidate agents

Agents that modulate SPPase expression, preferably up-regulate SPPase expression so that the levels of SPPase protein in a cell are increased, may be achieved by one or more.of several mechanisms. For example, a candidate agent may increase levels of transcription from endogenous SPPase genes, stabilize SPPase mRNA levels and/or stabilize SPPase protein. Transcription may be increased from endogenous SPPase genes by, for example, the use of a transcriptional activator that activates transcription from endogenous SPPase genes or an agent that modifies the effect of a transcriptional inhibitor of endogenous SPPase genes. Thus tests for modulation of SPPase expression include determining the effect of a candidate agent on SPPase mRNA levels and/or-SPPase protein-levels.

The term "modulate" in the context of the SPPase expression means a change or alteration in the levels of SPPase mRNA and/or SPPase protein.

Suitable candidate compositions include agents known to modulate cellular transcription, and/or lipid metabolism. Examples of agents which may affect SPPase expression include non-steroidal anti-inflammatory drugs such as ibuprofen and indomethacin, and drugs known to affect lipid metabolism such as fibrates and thiazolidinediones. Furthermore, combinatorial libraries, peptide and peptide mimetics, in .particular.. peptides from panels of peptides comprising random sequences or sequences which have been varied consistently to provide a maximally diverse panel of peptides, defined chemical entities such as organic and inorganic compounds, oligonucleotides, and natural product libraries may be screened for activity as modulators of SPPase expression in assays such as those described below. The candidate agents may be used in an initial screen in batches of, for example 10 agents per reaction, and the agents of those batches which show inhibition tested individually. In addition, candidate agents which show activity in *in vitro* screens such as those described below can then be tested in *in vivo* systems, such as mammalian cells which will be exposed to the inhibitor and tested, for example, for susceptibility to viral infection.

### ii. Assays

The assays of the invention may be *in vitro* assays or *in vivo* assays, for example using cell lines or an animal model.

### In vitro assay systems

An *in vitro* assay system of the invention typically measures the effect on transcription from a polynucleotide construct comprising an SPPase promoter linked to a polynucleotide whose transcribed, and optionally translated, product is capable of detection, for example the naturally occurring SPPase coding sequence or a reporter gene such as luciferase or CAT. Techniques for detecting and quantitating transcription products are well known in the art and include, for example hybridisation to labeled probes and direct quantitation of transcribed products by the use of labeled nucleotides which are incorporated during transcription, also quantitative PCR analysis and others. Translated products may also be detected and quantified using well known techniques such as SDS-PAGE and Western blotting, or in the case of biologically active products, suitable assays for detecting said activity (such as CAT assays or chemiluminescence assays).

A suitable *in vitro* assay may for example, be conducted using whole cell extracts of mammalian cells, typically supplemented with buffers and nucleotide mixes. Reporter constructs comprising polynucleotides containing SPPase promoter constructs linked to a reporter gene may be added to the mix, or endogenous genomic DNA used.

The effect of a candidate agent on SPPase expression may be determined by measuring levels of transcription from the SPPase promoter construct (endogenous or otherwise) in the presence and absence of the candidate agent and comparing the results. Preferably, a control promoter construct should also be tested to ensure that any effect on SPPase expression is specific to the SPPase promoter and is not simply the result of general transcriptional inhibition. A candidate agent is typically considered to modulate SPPase expression if the levels of transcription are altered by at least 30%, preferably at least 50, 60, 70, 80 or 90%. Any affect on the control promoter would preferably be accounted for when calculating changes in SPPase transcription.

### In vivo assay systems

Modulation of SPPase expression can also conveniently be measured *in vivo*, typically using mammalian cell lines. As with *in vitro* systems, both reporter constructs and endogenous SPPase genes may be used. In a preferred embodiment, the assay uses mammalian cells stably transfected with a polynucleotide comprising a reporter - construct which contains an SPPase promoter operably linked to a reporter gene, for example chloramphenicol transferase (CAT) or luciferase. The cell also preferably comprises a stably transfected control promoter sequence operably linked to a second reporter-gene -which can be distinguished front the first reporter gene. Typically, levels of transcription from the SPPase construct and the control construct are measured in the absence of a candidate agent and then measured in the presence of a candidate agent. The effect of the candidate agent on transcription from the SPPase reporter construct (or endogenous SPPase gene) can then be determined, taking into account any general effect on transcription as indicated by the result obtained for the control reporter construct.

In another embodiment, SPPase expression is measured by determining the amount of SPPase protein in cells before administration of the candidate agent and after administration of the candidate agent. As described above, protein levels are typically measured by analyzing cell extracts by SDS-PAGE and detecting the SPPase protein using Western blot analysis. Alternatively, the cells used in the assay of the invention may comprise a reporter construct containing an SPPase promoter operably linked to a nucleotide sequence encoding a detectable polypeptide product, such as CAT. In a particularly preferred embodiment, the detectable product encodes an enzyme which can cleave a cellular or exogenously added agent causing a detectable change in the absorption spectrum or emission spectrum of the cell or cell medium at a particular wavelength. This will facilitate the large-scale screening of candidate agents in, for example a microtitre plate assay format.

Administration of candidate agents to mammalian cell lines and generation of host mammalian cell lines comprising reporter constructs can be carried out as described above.

### 3. Testing candidate agents for anti-viral activity

Candidate, agents that are identified by the method of the invention as disrupting an interaction between a HCV-core SPPase targeting sequence and SPPase and thus trafficking of HCV-core to lipid globules, or modulating SPPase expression may be tested for their ability to, for example, reduce susceptibility of cells to viral infection. Such compounds could be used therapeutically to affect viral infection, for example to prevent or treat viral infection.

Typically, an assay to determine the effect of a candidate agent identifiable by a method of the invention on the susceptibility of cells to viral infection comprises:
(a) administering a virus, for example HCV, to a cell, in the absence of the candidate agent;
(b) administering the virus to the cell in the presence of the candidate agent; and
(c) determining if the candidate agent reduces or abolishes the susceptibility of the cell to viral infection.

The candidate agent may be administered before, or concomitant with, the virus to establish if infection is prevented. Alternatively, the candidate agent may be administered subsequent to viral infection to establish if viral infection can be treated using the candidate agent.. Administration of candidate agents to cells may be performed as described above.

The assay is typically carried out using mammalian cell lines, but an animal model could be employed instead, such as chimpanzees in the case of HCV. The virus is contacted with cells, typically cells in culture. The cells may be a mammalian cell line, in particular mammalian cells susceptible to infection by the virus in the absence of the candidate agent, for example in the case of HCV, liver cells.

Techniques for assaying infectivity of viruses are well-known in the art. As well as using plaque assays, levels of viral infection can be determined by using recombinant viruses which comprise a reporter gene, for example *lacZ.* The use of a histochemically detectable reporter gene is especially preferred when experiments are performed with animals. In the case of HCV, plaque assays cannot be used. A suitable method for determining the level of HCV production in an infected animal is to perform quantitative, semiquantitative or classic RT-PCR analysis to determine the amount of positive-strand nucleic acid material in cells or serum. In addition, the presence of negative-strand RNA in cells, as determined by RT-PCR, is taken to mean that there is active viral replication. Alternatively, another well known technique which can be employed in assessing the level of HCV production is Northern blotting.

In a preferred embodiment of the above-described assays, HCV-core signal peptide peptidase targeting sequence and derivatives thereof are used in an experimental system to study normal cellular interactions. For example, derivatives of viral proteins such as HCV core protein or derivatives of SPPase, including deletion, insertion and substitution mutants, can be used to disrupt an interaction between HCV-core and SPPase and thus lipid globules. This can be tested *in vitro* or *in vivo* using the assays described above. Consequently, the present invention provides the use of a derivative of the protease inhibitor (Z-LL)₂ ketone termed TBL₄K in the manufacture of a medicament for use in affecting a viral infection, preferably an HCV infection.

HCV-core signal peptide peptidase targeting sequences and derivatives thereof can be introduced into the cells using techniques described above, for example transfection of nucleic acid constructs encoding HCV-core targeting sequences, or using viral vectors. The effect of this disruption can be determined as described above. Any *in vitro* data obtained may be used to assist in the rational design of HCV-core targeting sequences for use in the *in vivo* studies. In addition, since the amino acid residues of the HCV-core signal peptide peptidase targeting sequences which bind to SPPase have been precisely mapped (see Sequence Listing), it will assist in the rational design of HCV-core targeting sequence derivatives for use in the *in vivo* studies. For details see Examples below.

Thus HCV-core signal peptide peptidase targeting sequences, which are readily distinguished from cellular constituents, may be used as a tool to investigate intracellular SPPase target proteins and further our understanding of their functions in the cell.

### F. Therapeutic Uses

Agents capable of disrupting an interaction between a HCV-core signal peptidase targeting sequence and an SPPase enzyme may be used to affect a viral infection in a human or animal, in particular to treat or prevent a viral infection. Such agents may have been identified by the assay methods of the invention or otherwise.

Agents that are capable of modulating SPPase expression may also be used to affect a viral infection in a human or animal, in particular to treat or prevent a viral infection. Such agents may have been identified by the assay methods of the invention or otherwise. The use of a derivative of the protease inhibitor (Z-LL)₂ ketone termed TBL₄K in the manufacture of a medicament for use in affecting a viral infection, preferably an HCV infection is described.

### G. Compositions/Administration

Proteins of the invention and agents identified or identifiable by the assay methods of the invention may preferably be combined with various components to produce compositions. Preferably the compositions are combined with a pharmaceutically acceptable carrier or diluent to produce a pharmaceutical composition (which may be for human or animal use). Suitable carriers and diluents include isotonic saline solutions, for example phosphate-buffered saline. The composition may be administered by direct injection. The composition may be formulated for parenteral, intramuscular, intravenous, subcutaneous, intraocular or transdermal administration. Typically, each protein may be administered at a dose of from 0.01 to 30 mg/kg body weight, preferably from 0.1 to 10 mg/kg, more preferably from 0.1 to 1 mg/kg body weight.

Polynucleotides/vectors encoding polypeptide components for use in affecting viral infections may be administered directly as a naked nucleic acid construct, preferably further comprising flanking sequences homologous to the host cell genome. When the polynucleotides/vectors are administered as a naked nucleic acid, the amount of nucleic acid administered may typically be in the range of from 1 µg to 10 mg, preferably from 100 µg to 1 mg.

Uptake of naked nucleic acid constructs by mammalian cells is enhanced by several known transfection techniques for example those including the use of transfection agents. Example of these agents include cationic agents (for example calcium phosphate and DEAE-dextran) and lipofectants (for example lipofectam^{™} and transfectam^{™}). Typically, nucleic acid constructs are mixed with the transfection agent to produce a composition.

Preferably the polynucleotide or vector is combined with a pharmaceutically acceptable carrier or diluent to produce a pharmaceutical composition. Suitable carriers and diluents include isotonic saline solutions, for example phosphate-buffered saline. The composition may be formulated for parenteral, intramuscular, intravenous, subcutaneous, intraocular or transdermal administration.

The routes of administration and dosages described are intended only as a guide since a skilled practitioner will be able to determine readily the optimum route of administration and dosage for any particular patient and condition.

### EXAMPLES

The invention will be described with reference to the following Examples which are intended to be illustrative only and not limiting. The Examples refer to the following Figures.

### Brief Description of the Figures

**Figure 1.** Targeting and translocation mediated by the signal sequence at the junction of core-E1.
   **A**. *In vitro* translation of mRNA coding for SP-E1/100 in the presence of ER-derived rough microsomes.
   **B**. *In vitro* translation of mRNA coding for core-E1/100 in the presence of ER-derived rough microsomes.
**Figure 2**. Affect of mutations in the transmembrane region on signal peptide processing in the context of N-terminally deleted core protein.
   **A.** *In vitro* translation of mRNA coding for SP-E1/100 and mutant derivatives MUTI, MUTII and MUTIII.
   **B.** Quantification of signal peptide processing obtained with SP-E1/100 constructs.
**Figure 3.** Affect of mutations in the transmembrane region on signal peptide processing in the context of the entire core protein.
   **A.** Autoradiography and western blot analysis of *in vitro* translations with mRNA coding for core-E1/100 constructs (wt and mutants MUTI, MUTII and MUTIII).
   **B.** Quantification of core processing by SPPase obtained *in vitro* with core-E1/100 constructs.
**Figure 4**. Intracellular localisation of core and envelope proteins.
   **A**. Confocal images of the intracellular distribution of core and E2 proteins.
   **B**. Glycosylation and relative molecular weights of E1 protein.
**Figure 5**. Confocal images of the intracellular localisation of core proteins and lipid droplets.
**Figure 6.** Effect of SPPase processing on the release of core from ER.
**Figure 7**. Effect of signal peptide mutations on degradation of a core variant.
   **A.** Confocal images of the intracellular distribution of core and E2 proteins.
   **B and C**. Abundance of core and E2 proteins in the presence and absence of MG132.
**Figure 8**. Outline of the stages in the synthesis of TBL₄K.
**Figure 9**. Inhibition of signal peptide peptidase processing by TBL₄K.
**Figure 10**. Photo-labelling of proteins with TBL₄K.
**Figure 11**. Scheme for the purification of protein targeted by TBL₄K.
**Figure 12.** Amino acid sequences of peptides determined by mass spectrometry.
**Figure 13.** A. Predicted polypeptide sequence of the human protein (accession number gi 14772424) with sequence identity to the peptides in Figure 12.
   **B**. Motifs that have been identified in human SPPase.
   **C**. cDNA sequence of the predicted polypeptide sequence shown in A.
**Figure 14.** Alignment of SPPase homologues from lower and higher eukaryotes.

### Detailed Description of the Figures

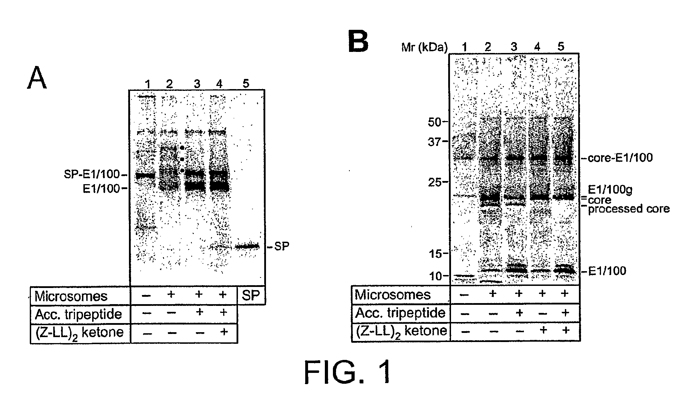

**Figure 1**. Targeting and translocation mediated by the signal sequence at the junction of core-E1.
**A**. *In vitro* translation of mRNA coding for SP-E1/100 in the presence of ER-derived rough microsomes (lanes 2 -4), acceptor tripeptide to inhibit N-glycosylation (lanes 3 and 4) and the SPPase inhibitor, (Z-LL)₂ ketone (lane 4). Dots indicate glycosylated E1 with 1-, 2- and 3-coupled oligosaccharides. Lane 5 shows a reference protein corresponding to *in vitro* translated signal peptide (SP).
**B.** *In vitro* translation of mRNA coding for core-E1/100 in the presence of ER-derived rough microsomes (lanes 2-5), acceptor tripeptide to inhibit N-glycosylation (lanes 3 and 5) and the SPPase inhibitor (Z-LL)₂ ketone (lanes 4 and 5). E1/100g indicates the position ofN-glycosylated E1/100.

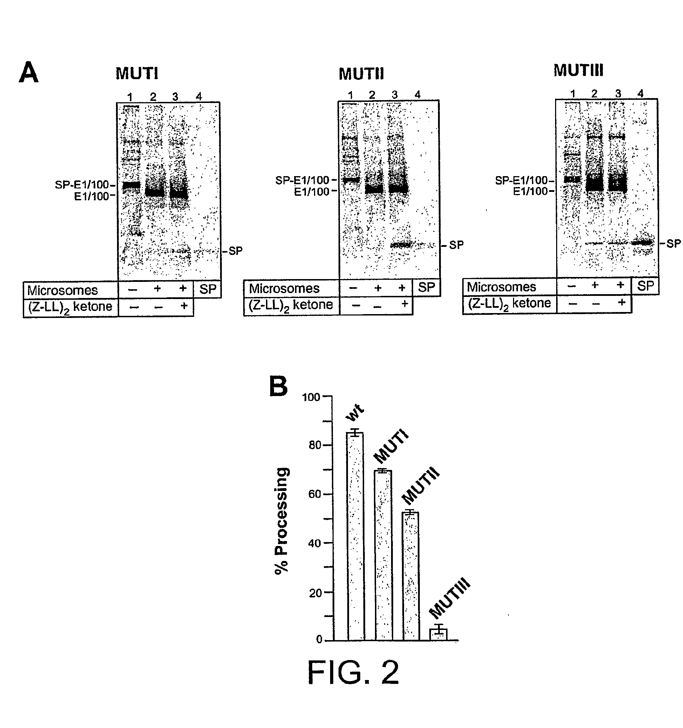

**Figure 2**. Affect of mutations in the transmembrane region on signal peptide processing in the context of N-terminally deleted core protein.
**A**. *In vitro* translation of mRNA coding for SP-E1/100 and mutant derivatives MUTI, MUTII and MUTIII. In lanes 2 and 3, samples contained both the glycosylation acceptor tripeptide and ER-derived rough microsomes. The SPPase inhibitor (Z-LL)₂ ketone was present in reactions shown in lanes 3. Lane 4 shows a reference protein corresponding to *in vitro* translated signal peptide (SP).
**B**. Quantification of signal peptide processing obtained with SP-E1/100 constructs. The amounts of signal peptide found in the membrane fraction were compared in samples with and without SPPase inhibitor. Quantification for the wt SP-E1/100 protein is derived from Fig. 1A.

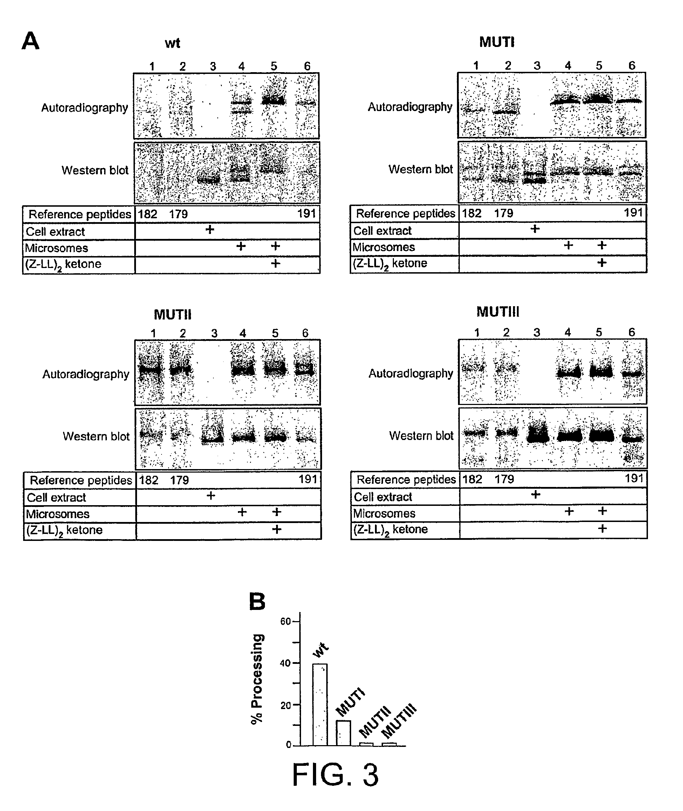

**Figure 3**. Affect of mutations in the transmembrane region on signal peptide processing in the context of the entire core protein.
**A.** Autoradiography and Western blot analysis of *in vitro* translations with mRNA coding for core-E1/100 constructs (wt and mutants MUTI, MLJTII and MUTIII; lanes 4 and 5). In lanes 4 and 5, reactions contained acceptor tripeptide and ER-derived rough microsomes. The SPPase inhibitor, (Z-LL)₂ ketone, was present in reactions in lanes 5. Samples in lanes 3 are from extracts of BHK cells electroporated with RNA from the corresponding pSF vector for each of the core constructs. Lanes 1, 2 and 6 show *in vitro* translated reference peptides corresponding to the N-terminal 182, 179 and 191 amino acid residues of core.
**B**. Quantification of core processing by SPPase obtained *in vitro* with core-E1/100 constructs. The amount of processed core (179 amino acid residues) was correlated to the total amount of core (core₁₉₁+core₁₇₉). Core₁₇₉ was not generated in samples containing SPPase inhibitor.

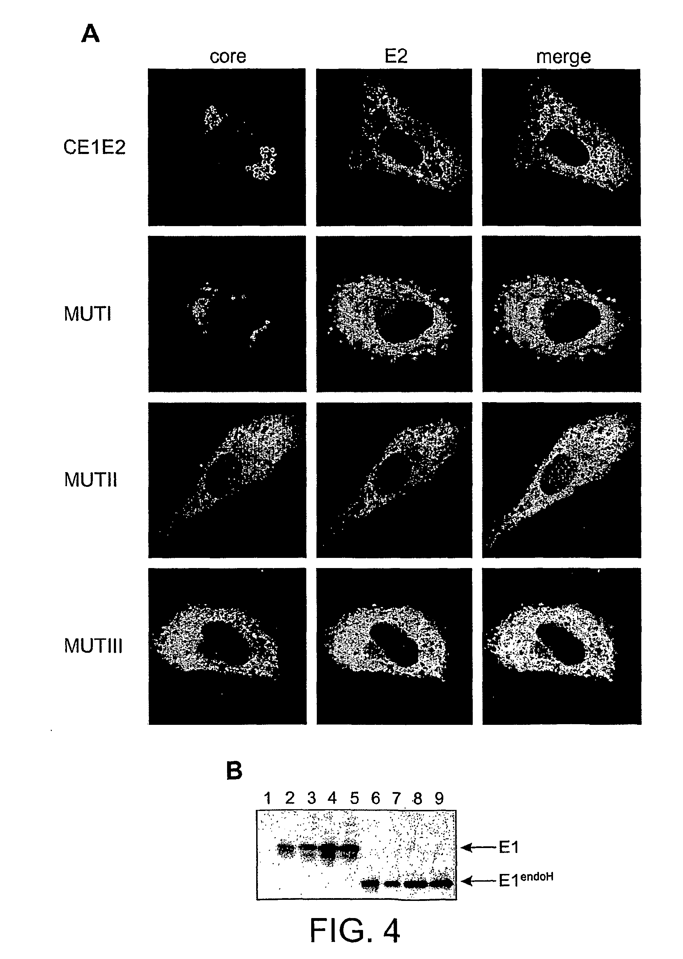

**Figure 4**. Intracellular localisation of core and envelope proteins.
BHK C13 cells were harvested 15 hours after electroporation with RNA from pSF/CE1E2 and pSF/MUT plasmids, and either fixed with methanol for indirect immunofluorescence (A) or lysed to produce cell extracts for Western blot analysis (B).
**A**. Confocal images of the intracellular distribution of core and E2 proteins. Indirect immunofluorescence was performed with R308 and ALP98 antibodies (specific for core and E2 respectively).
**B**. Glycosylation and relative molecular weights of E1 protein made by pSF/MUT constructs. Extracts were either treated (lanes 6-9) or not treated (lanes 1-5) with Endo H. After electrophoresis by SDS-PAGE and transfer of proteins to PVDF nitrocellulose membrane, Western blot analysis was performed with R528, an E1-specific antisera. Samples were as follows: lane 1, pSF/1-195; lanes 2 and 6, pSF/CE1E2; lanes 3 and 7, pSF/MUTI; lanes 4 and 8, pSF/MUTII; lanes 5 and 9, pSF/MUTIII. The positions of glycosylated and deglycosylated E1 (E1 and E1^{endoH} respectively) are indicated.

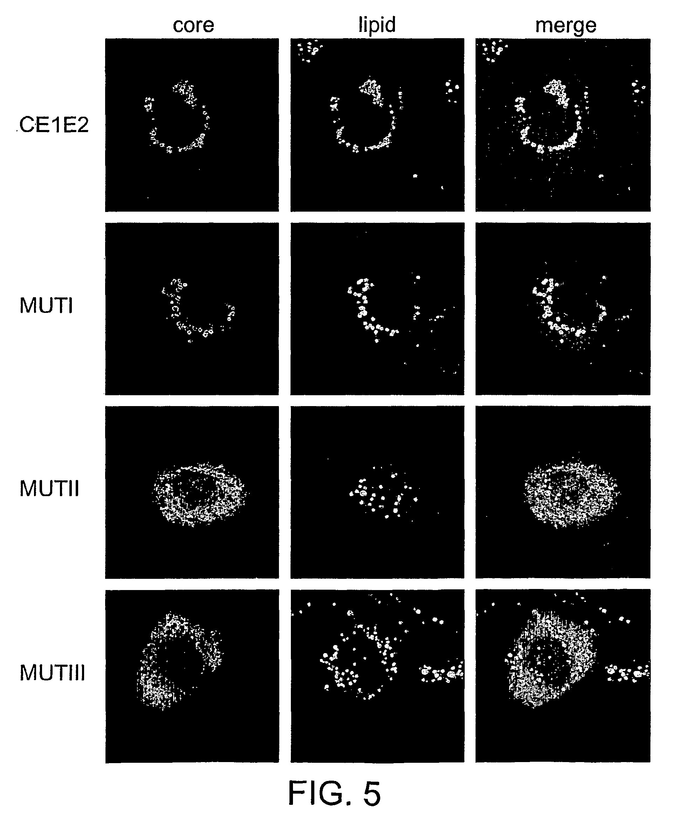

**Figure 5.** Confocal images of the intracellular localisation of core proteins and lipid droplets. BHK C13 cells were harvested 15 hours after electroporation with RNA from pSF/CE1E2 and pSF/MUT plasmids, and fixed with 4% paraformaldehyde, 0.1% Triton X-100.. Indirect immunofluorescence was performed with antibody JM122 and an anti-mouse secondary antibody conjugated with FITC. Lipid droplets were stained with oil red oil (ORO).

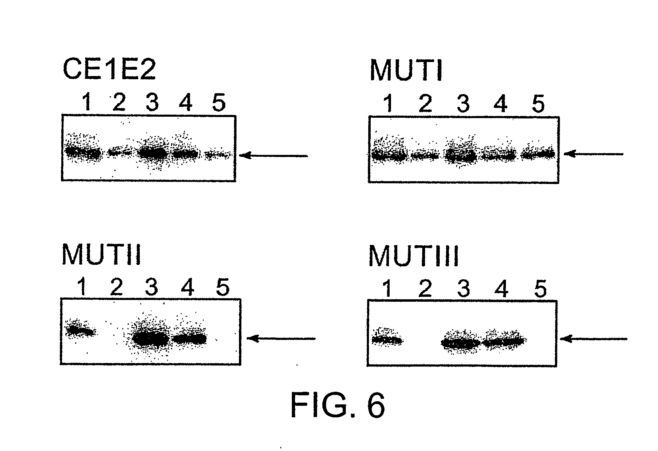

**Figure 6**. Effect of SPPase processing on the release of core from the ER.

BHK C13 cells were harvested 15 hours after electroporation with RNA from the pSF plasmids indicated. Cells were homogenised and membrane (P1) and supernatant fractions were prepared (SN1). Membranes were treated with Na₂CO₃ and extracted proteins (SN2) were separated from non-extracted material (P2). After electrophoresis by SDS-PAGE and transfer of proteins to PVDF nitrocellulose membrane, Western blot analysis was performed with R308, a core-specific antisera. Samples on gels for each pSF construct were as follows: lanes 1, whole cell extract; lanes 2, SN1; lanes 3, P1; lanes 4, P2; lanes 5, SN2. The positions of core protein are indicated.

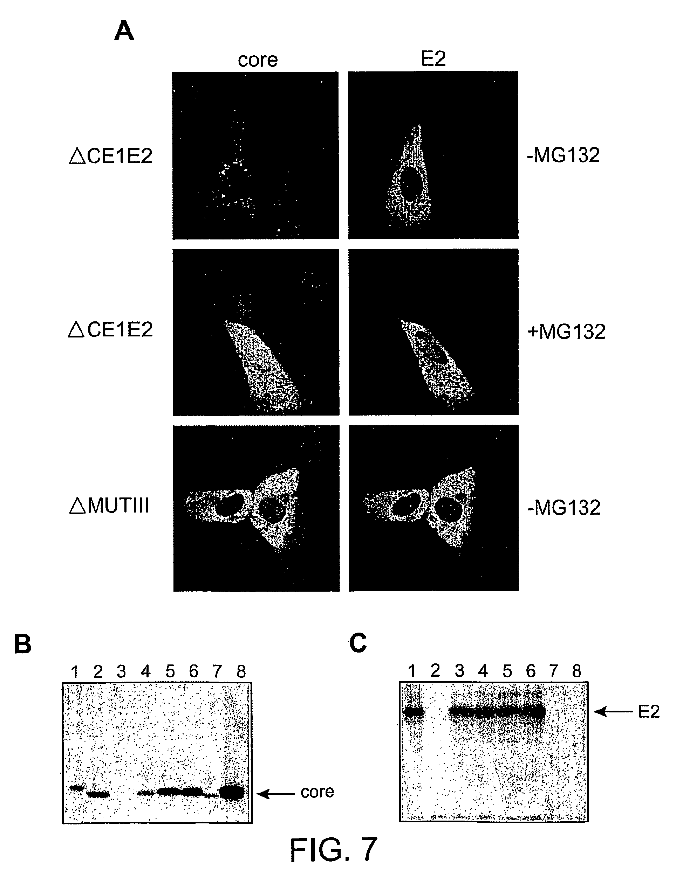

**Figure 7.** Effect of signal peptide mutations on degradation of a core variant made by pSF/ CE1E2.

BHK cells were electroporated with RNA and 5 hours after incubation at 37 °C, they were treated with MG132 for 12 hours at 37 °C. Cells were either fixed with methanol for indirect immunofluorescence (A) or extracts were prepared for Western blot analysis (B and C).
**A.** Confocal images of the intracellular distribution of core and E2 proteins. Indirect immunofluorescence was performed with R308 and ALP98 antibodies (specific for core and E2 respectively).
**B and C.** Abundance of core and E2 proteins in the presence and absence of MG132. After electrophoresis by SDS-PAGE and transfer to PVDF membranes, samples were probed with JM122 and ALP98 antibodies (B and C respectively). Samples in B and C were as follows: lanes 1, pSF/CE1E2; lanes 2 and 7, pSF/1-124,145-169; lanes 3 and 4, pSF/ CE1E2; lanes 5 and 6, pSF/ MUTIII; lanes 8, pSF/ 125-144. Samples in lanes 4 and 6 only were treated with MG132. Positions for core and E2 proteins are indicated.

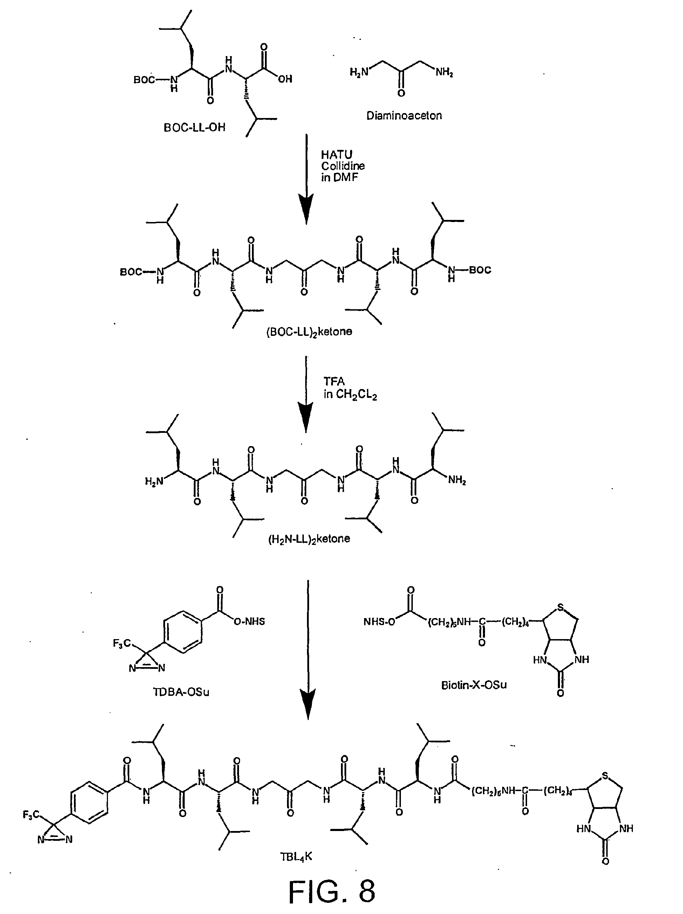

**Figure 8.** Outline of the stages in the synthesis of TBL₄K.

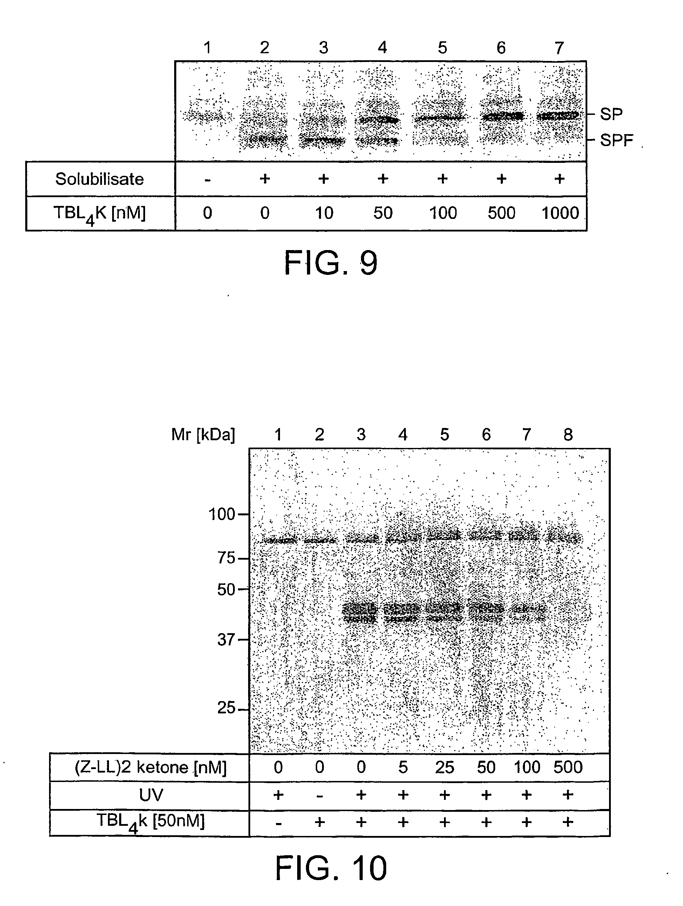

**Figure 9.** Inhibition of signal peptide peptidase processing by TBL₄K.
Radio-labelled p-Prl^{PP}/29 signal peptide was generated by *in vitro* translation in wheat germ extract containing [³⁵S]-methionine. Peptide was added to CHAPS-solubilised membranes in the absence or presence of TBL₄K (at the indicated final concentrations) and incubated for 1 hour at 30 °C. Proteins were precipitated with 10 % trichloroacetic acid and analysed by SDS-PAGE using Tris-bicine-urea acrylamide gels. Bands corresponding to signal peptide substrate (ps) and the fragment of signal peptide remaining after SPPase processing (SPF) are indicated.

**Figure 10.** Photo-labelling of proteins with TBL₄K.
CHAPS-solubilised ER membrane proteins were mixed with 50 nM TBL₄K (dissolved in DMSO) and samples incubated at 30 °C for 1 hour. (Z-LL)₂ ketone; at varying concentrations, also was added to the reactions indicated in the figure. Where indicated, samples were irradiated with UV light before precipitation with 10 % trichloroacetic acid. Proteins were resolved on Tris-glycine acrylamide gels, transferred to PVDF membrane and biotinylated proteins were visualised by western blotting with polyclonal anti-biotin antibody.

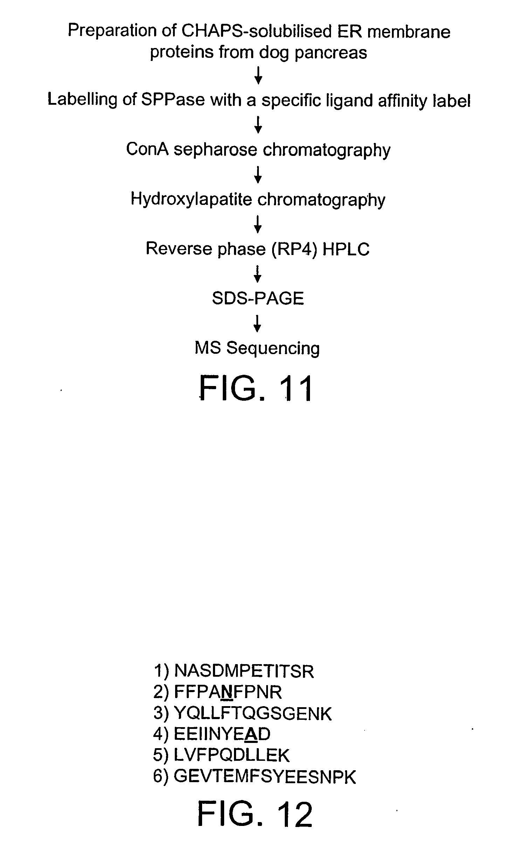

**Figure 11.** Scheme for the purification of protein targeted by TBL₄K.

**Figure 12.** Amino acid sequences of peptides determined by mass spectrometry. Residues not conserved in the human sequence are highlighted in bold and underlined.

**Figure 13.**
**A**. Predicted polypeptide sequence of the human protein (accession number gi 14772424) with sequence identity to the peptides in Figure 12. Peptides sequenced by mass spectometry (Figure. 12) are shown in bold and underlined. Motifs corresponding to those described in B are shown in bold and italics.
**B**. Motifs that have been identified in human SPPase. The locations of proposed N-glycosylation sites are based on the predicted topology of SPPase with respect to regions that are present either in the cytosolic or lumenal sides of the ER membrane.
**C**. cDNA sequence of the predicted polypeptide sequence shown in A.

**Figure 14.** Alignment of SPPase homologues from lower and higher eukaryotes.

### MATERIALS AND METHODS

### 1. Construction of Plasmids

### a) pGEM Plasmids.

Plasmids containing the coding region for the core protein of HCV strain Glasgow were obtained by combining fragments from two constructs called core.pTZ18 and 5'-NS2 (provided by M. McElwee and R. Elliott). Core.pTZ18 possesses nucleotide residues.337-915 of the HCV strain Glasgow genome and 5'- NS2 contains residues 1-2895. 5'- NS2 was modified for cloning purposes such that the sequences immediately upstream of residue 337 contained the recognition sequences for Bgl II and Kpn I restriction enzyme sites and immediately downstream of residue 2895, an oligonucleotide was inserted that encodes a translational stop codon followed by sequences for Bgl II and Hind III restriction enzyme sites. The resultant construct, 5'-NS2/Bgl II, contained a G to A nucleotide substitution at position 663, which was present also in the original 5'- NS2 plasmid, that introduced a stop codon into the coding sequences. To replace this defect, a Kpn I/Bst EII DNA fragment (containing HCV nucleotides 337 to 841) from core.pTZ18 was inserted into 5'- NS2/Bgl II cut with the same enzymes. The resultant plasmid was termed 5'- NS2/corr. To create pgHCV/CE1E2, a Bgl II/Hind III DNA fragment from 5'- NS2/corr, that was a partially digested fragment containing the core, E1, E2, p7 and part of the NS2 sequences, was inserted into the Bam HI and Hind III sites of pGEM1. The resultant plasmid was further modified by introducing a Bgl II enzyme site at the Eco RI site in the pGEM backbone, generating pgHCV/CE1E2. Thus, pgHCV/CE1E2 contains nucleotide residues 337-2895 of the HCV strain Glasgow genome and encodes the core, E1, E2, p7 and partial coding region of NS2.

Construction of a derivative plasmid, pgHCV/1-195 from pgHCV/CE1E2 was achieved by inserting an oligonucleotide (GCTGAGATCTA) that had both a translational stop codon and the sequences for a Bgl II enzyme site between a Fsp I enzyme site at nucleotide residue 925 in the HCV genome and a Hind III enzyme site in the pGEM backbone. Thus, pgHCV/1-195 encodes the N-terminal 195 amino acids (aa) of HCV strain Glasgow. From pgHCV/1-195 the following series of constructs were made which had various regions of the HCV coding region removed (i-iv below) or mutated (v below).

The numbers following pgHCV/ represent the aa residues of HCV strain Glasgow encoded by each construct:
i) pgHCV/1-169 was constructed by inserting an oligonucleotide GTAACCTTTGAGATCTA between the Bst EII (at nucleotide residue 841 in the HCV strain Glasgow genome) and Hind III enzyme sites (located in the pGEM backbone) in pgHCV/1-195.
ii) pgHCV/ 125-144 was constructed by inserting oligonucleotide CGATAGAGGCGCTGCCAGGGCC between the ClaI and BstXI sites (at nucleotide residues 710 and 792 respectively in the HCV strain Glasgow genome) in pgHCV/1-195.
iii) pgHCV/1-124,145-169 was made by inserting the oligonucleotide used to create pgHCV/ 125-144 between the ClaI and BstXI sites (at nucleotide residues 710 and 792 respectively in the HCV strain Glasgow genome) in pgHCV/1-169.
iv) pgHCV/ CE1E2 was made by replacing a BssHII/BstEII fragment (between nucleotide residues 480 and 841 in the HCV strain Glasgow genome) in pSFV/CE1E2 with the corresponding DNA fragment from pgHCV/ 125-144.

### b) pSV-SPORTl Plasmids and Signal Sequence Mutants.

For *in vitro* transcription and translation reactions, the Eco RI/Hind III fragment of pgHCV/CE1E2 was inserted into vector pSV-Sport1 (Life Technologies) that had been cut with the same enzymes to give plasmid pSV/CE1E2, respectively. In this plasmid, the HCV fragment was oriented such that it was under the control of the SP6 promoter. The signal sequence mutants pSV/MUTI, pSV/MUTII and pSV/MUTIII (Table 1) were generated by overlap extension polymerase chain reaction (PCR) using pSV/CE1E2 as the template.

### c) pSF plasmids.

For expression in tissue culture cells, Bgl II DNA fragments carrying the relevant HCV sequences were prepared from the pgHCV plasmid series in a) above and inserted into the Bam HI site of a Semliki Forest virus vector pSFV1 (Life Technologies). The resultant plasmids were termed the pSF/ series (e.g. pSF/1-195). For each of the pSF/MUT plasmids (see Table 2), Bst EII/Nru I fragments (nucleotide residues 841 to 1034) from the pSV/MUT constructs were transferred firstly into pgHCV/CE1E2 and pgHCV/ CE1E2 (for MUTIII only) to give pg/MUT plasmids and thereafter from these constructs into pSFV1 as described above for the pgHCV plasmids.

**Table 2. Details of Plasmid Constructs.**

| ***Name of Plasmid*** | ***Vector*** | ***Features*** |
|---|---|---|
| pSF/CE1E2 | pSFV1 | wild-type |
| pSF/MUTI | pSFV1 | Ala₁₈₀→Val₁₈₀ |
| pSF/MUTII | pSFV1 | Ser₁₈₃/Cys₁₈₄→Leu₁₈₃/Val₁₈₄ |
| pSF/MUTIII | pSFV1 | Ala₁₈₀→Val₁₈₀; |
| | | Ser₁₈₃/Cys₁₈₄→Leu₁₈₃/Val₁₈₄ |
| pSF/ CE1E2 | PSFV1 | 125-144 |
| pSF/ MUTIII | pSFV1 | 125-144; |
| | | Ala₁₈₀→Val₁₈₀; Ser₁₈₃/Cys₁₈₄→Leu₁₈₃/Val₁₈₄ |
| pSP/CE1E2 | pSV-SportI | wild-type |
| pSP/MUTI | pSV-SportI | Ala₁₈₀→ Val₁₈₀ |
| pSP/MUTII | pSV-SportI | Ser₁₈₃/Cys₁₈₄→Leu₁₈₃/Val₁₈₄ |
| pSP/MUTIII | pSV-SpottI | Ala₁₈₀→Val₁₈₀; |
| | | Ser₁₈₃/Cys₁₈₄→Leu₁₈₃/Val₁₈₄ |

### 2. Antibodies

The antibodies used to detect HCV core (monoclonal antibody [MAb] JM122 and rabbit antisera R308), E1 (rabbit antisera R528) and E2 proteins (MAb ALP98) have been described previously (Hope and McLauchlan, 2000, *J Gen Virol,* **81**, 1913-1925; Patel *et al.,* 2001, *Virology* **279,** 58-68).

### 3. In Vitro Transcription, Translation and Signal Peptide Processing

To prepare mRNA coding for core-E1/100 precursors (wt and signal peptide mutants), the respective coding region from the appropriate pSV plasmid (Table 1) was first amplified with PCR using *Pfu* DNA polymerase (Stratagene), SP6 primer and a reverse primer starting with 5'-NNNNNNNNNCTA- to introduce a TAG stop codon at aa position 101 of the E1 sequence. PCR-amplified DNA fragments were then transcribed *in vitro* with SP6 RNA polymerase at 42°C in the presence of 500 µM m⁷G(5')ppp(5')G CAP analogue (Nilsson and von Heijne, 1993 *J Biol Chem* **268,** 5798-5801). Likewise, mRNA coding for SP-E1/100 (wt and signal peptide mutants) was prepared from PCR-amplified DNA fragments, which were transcribed by SP6 RNA polymerase. For the latter PCR, the pSV plasmids (Table 2) served as templates, and the forward primer encoded the SP6 promotor, the Kozak initiation sequence, and codons 161-167 of HCV core.

The mRNAs were translated in 25 µl rabbit reticulocyte lysate (Promega) containing [³⁵S]-methionine (Amersham-Pharmacia) and, where indicated, 2 eq of nuclease-treated rough microsomes prepared from dog pancreas (Martoglio *et al.,* 1998, *Cell Biology: A Laboratory Handbook.* Academic Press, San Diego, CA, vol **2,** 265-274). To inhibit signal peptide peptidase (SPPase) processing, 10 µM (Z-LL)₂-ketone was added to reactions (Weihofen *et al.,* 2000 *J Biol Chem* **275,** 30951-30956), and reactions were supplemented with 30 µM N-benzoyl-Asn-Leu-Thr-methyamide to prevent N-glycosylation of translated products (Martoglio *et al.,* 1998 *, Cell Biology: A Laboratory Handbook.* Academic Press, San Diego, CA, vol **2,** 265-274). Samples were incubated for 30 min at 30 °C. Reactions containing microsomes were next diluted with 25 µl of RM buffer (50 mM HEPES-KOH, pH7.6, 50 mM KOAc, 2 mM Mg(OAc)₂, 1 mM DTT, 250 mM sucrose), and the salt concentration was raised to 500 mM KOAc. After incubation for 5 min on ice, membranes were separated by centrifugation through a 100 µl sucrose cushion (RM buffer with 500 mM KOAc and 500 mM sucrose) at 48,000 rpm for 3 min at 4 °C in a Beckman TLA100 rotor. Membrane pellets were prepared for SDS-PAGE as described (Weihofen *et al.,* 2000 *J Biol Chem* **275,** 30951-30956).

### 4. In Vitro. Transcription of pSF Constructs

Prior to electroporation, RNA was transcribed *in vitro* from the appropriate pSFV plasmid which had been linearised at a Spe I enzyme site. Typical reactions were carried out in a volume of 20 1 and contained 40 mM Tris-HCl (pH 7.5), 6 mM MgCl₂, 2 mM spermidine, 10 mM NaCl, 1 mM DTT, 1 mM ATP, 1 mM CTP, 1 mM UTP, 0.5 mM GTP, 1 mM m⁷G(5')ppp(5')G CAP analogue, 50 units RNasin, 50 units SP6 RNA polymerase and 2 g linearised DNA. Reactions were performed at 37 °C for 2 hours. Products of the reaction were analysed by agarose gel electrophoresis to examine the quality and quantity of RNA synthesised prior to use in electroporation.

### 5. Maintenance of Tissue Culture Cells and Treatment with MG132

For the purposes of the present invention, the cultured cells derived from baby hamster kidney (BHK) C13 cells were grown and maintained in Glasgow minimal Eagle's Medium supplemented with 10% new-born calf serum (CS), 4% tryptose phosphate broth, and 100 IU/ml penicillin/streptomycin (ETC10). To treat cells with MG132 (Boston Biochem), cultured cells were incubated for 5 hours after electroporation at 37 °C and the BHK media was replaced with fresh media containing the protease inhibitor at a final concentration of 2.5 g/ml. Incubation was continued at 37 °C for a further 12 hours before the cells were either harvested for Western blot analysis or fixed for indirect immunofluorescence studies.

### 6. Electroporation of Cells

BHK cells were washed and treated with trypsin for detachment from tissue culture containers. Detached cells were suspended in 20 ml of growth medium and centrifuged at 100 g for 5 min at room temperature. Cell pellets were suspended in 50 ml of PBSA (PBS solution that lacks Ca²⁺ and Mg²⁺ salts) and centrifuged as previously. Pellets were suspended in PBSA at a final concentration of about 2 x 10⁷ cells/ml. 0.8 ml of competent BHK cells were mixed with in vitro transcribed RNA in an electroporation cuvette (0.4 cm gap) and pulsed once at 1.2 kV, 25 F. Following electroporation, cells were diluted in growth medium and seeded onto either tissue culture dishes or coverslips in 24-well tissue culture plates and then incubated at 37 °C for 12 hours.

### 7. Preparation of Cell Extracts and Deglycosylation of Proteins

Cells were scraped into PBS and pelleted by centrifugation at 100 g for 5 mins at 4 °C. The cell pellet was solubilised in sample buffer consisting of 160 mM Tris-HCl (pH 6.7), 2 % SDS, 700 mM -mercaptoethanol, 10 % glycerol, 0.004 % bromophenol blue. Alternatively, sample buffer was added directly to cells that had been washed with PBS. Cells were solubilised at a concentration of approximately 4 x 10⁶ cell equivalents per ml sample buffer. Samples were heated to 100 °C for 5 mins to fully denature proteins and nucleic acids.

For deglycosylation, cells were dissolved in 0.5 % SDS, 1 % -mercaptoethanol at 100 °C for 10 min at a concentration of approximately 6 x 10⁶ cell equivalents per ml. Sodium citrate was added to 50 mM followed by 2000 units of Endo H and reactions were incubated at 37 °C for 1 hour. Sample buffer was added to dilute the extract to approximately 4 x 10⁶ cell equivalents per ml and samples were heated to 100 °C as described above.

### 8. Carbonate Extraction of Tissue Culture Cells

150mm tissue culture dishes of BHK cells, electroporated with RNA from the relevant pSF constructs, were incubated at 37 °C for 15 hours. Cells were washed twice and scraped into PBS. After pelleting at 300g for 5 mins at 4 °C, cells were swollen in 600 ul of Buffer A (10 mM HEPES-KOH, pH 7.9, 1.5 mM MgCl₂, 10 mM KCl, 0.5 mM DTT) for 10 min at 4 °C and pelleted as before. Cell pellets were resuspended in 600 ul of Buffer A and homogenised by passage ten times through a 0.45 gauge needle. Intact cells that survived homogenisation and nuclei were pelleted by centrifugation at 1000g for 10 mins at 4 °C. The resultant supernatant was centrifuged at 100,000g for 15 mins at 4 °C. This generated membrane (pellet, P1) and cytosolic fractions (supernatant, SN1). Pellet P1 was resuspended in 100 ul of freshly prepared 100 mM Na₂CO₃ (pH 11.3) and incubated on ice for 15 mins. Soluble and insoluble material was separated by centrifugation through 200 ul of sucrose cushion (100 mM Na₂CO₃, 250 mM sucrose) at 130,000g for 15 mins at 4 °C. The resultant pellet (P2) and supernatant fractions (SN2) represent non-extracted and Na₂CO₃-extracted material respectively. Prior to electrophoresis, lipids in SN1 and SN2 were solubilised by addition of acetone to 25% and proteins were precipitated by adding trichloroacetic acid to 15%. Precipitated proteins were recovered by centrifugation at 13,000g for 5 mins, washed with acetone, dried and resuspended in sample buffer prior to electrophoresis on polyacrylamide gels.

### 9. SDS-PAGE and Western Blot Analysis

Proteins and peptides were analysed by SDS-PAGE using either Tris-glycine acrylamide gels (13 % T, 2.7 % C) (Lämmli, 1970, *Nature* **227,** 570-574) or Tris-bicine-urea acrylamide gels (15 % T or 10 % T, 5 % C; 8 M urea) (Wiltfang *et al.,* 1997 *Electrophoresis,* **18,** 527-532). Labelled proteins were visualised by a STORM phosphorimager (Molecular Dynamics). For Western blot analysis, proteins were separated by SDS-PAGE and transferred to PVDF membrane: Two sets of conditions were used for probing membranes with antibodies and antisera. One set consisted of blocking membranes in 3 % gelatin, 20 mM Tris-HCl (pH 7.5), 500 mM NaCl for 2 hours at room temperature. Thereafter, incubations with the primary antibodies or antisera were performed in 1 % gelatin, 20 mM Tris-HCl (pH 7.5), 500 mM NaCl, 0.05 % Tween 20 at either room temperature or 37 °C for approximately 3-4 hours. Following extensive washing with 20 mM Tris-HCl (pH 7.5), 500 mM NaCl, 0.05 % Tween 20, the membrane was incubated for 2 hours at room temperature with the appropriate secondary antibody conjugated with horse radish peroxidase in the same solution as for the primary antibody and at a dilution of 1/1000. For the second set of conditions, membrane was blocked with 5 % BSA in TBST (20 mM Tris-HCl [pH 7.6], 140 mM NaCl, 0.1 % Tween 20) for 1 hour at room temperature followed by overnight incubation with primary antibody or antisera in blocking solution at 4 °C. After extensive washing with TBST, the membrane was incubated for 2 hours at room temperature with the appropriate secondary antibody conjugated with horse radish peroxidase in TBST at a dilution of 1/10-50000. Bound antibody was detected by enhanced chemiluminescence (Amersham-Pharmacia). The primary antibodies and antisera, and their dilutions were as follows: monoclonal antibodies JM122 and ALP98 (both diluted to 1/500), rabbit antisera R528 and R308 (both diluted to l/1000).

### 10. Indirect Immunofluorescence and Staining of Lipids

Cells on 13 mm coverslips were fixed in either methanol at -20 °C or 4% paraformaldehyde, 0.1 % Triton X-100 (prepared in PBS) at 4 °C for 30 mins. Following washing with PBS and blocking with PBS/CS (PBS containing 1 % new born calf serum), cells were incubated with primary antibody (diluted in PBS/CS at 1/200 for JM122 and ALP98 MAbs, 1/1000 for R308 and R528 antisera) for 2 hours at room temperature. Cells were washed extensively with PBS/CS and then incubated with conjugated secondary antibody (either anti-mouse or anti-rabbit IgG raised in goat) for 2 hours at room temperature. Cells were washed extensively in solutions of PBS/CS followed by PBS and finally H₂O before mounting on slides using Citifluor. Samples were analysed using a Zeiss LSM confocal microscope.

Following incubation with both antibodies and washing, lipid droplets were stained in paraformaldehyde-fixed cells by briefly rinsing coverslips in 60 % propan-2-ol followed by incubation with 0.5 ml of 60 % propan-2-ol containing oil red O for 1.5-2 mins at room temperature. Coverslips were briefly rinsed with 60 % propan-2-ol, washed with PBS and H₂O and mounted as described above. The oil red O staining solution was prepared from a saturated stock of approximately 1 % oil red O dissolved in propan-2-ol. Before staining, the stock was diluted with H₂O and then filtered.

### 11. Preparation of CHAPS-Solubilised ER Membrane Proteins

### a) Preparation of ER-Derived Rough Microsomes (RMs) from Dog Pancreas

Dogs (e. g. beagle or fox hound) were obtained from pharmaceutical companies and the pancreas removed within 10 min of death. The pancreas (20-30 g) was surgically excised, and connective tissue and fat removed:-The remaining pancreatic tissue was cut into small pieces, frozen and stored in liquid nitrogen. For the preparation of RMs, the frozen pieces of one pancreas were thawed in 120 ml of homogenisation buffer (250 mM sucrose, 50 mM HEPES-KOH, pH 7.6, 50 mM KOAc, 6 mM Mg(OAc)₂, 1 mM EDTA, 1 mM DTT, 30 µg/ml PMSF). Tissue was passed through a tissue press with a 1 mm mesh steel sieve followed by homogenisation in a 60 ml glass/teflon potter with 5 strokes at full speed (1500 rpm). Homogenate was transferred into 30 ml polypropylene tubes and centrifuged at 3,000 rpm for 10 min at 4 °C in a Sorvall SS34 rotor. Supernatant was collected, avoiding the floating lipid, and the pellet was homogenised again in 50 ml homogenisation buffer as described above. The supernatants from both extractions were pooled, transferred to 30 ml polypropylene tubes and centrifuged at 9,500 rpm for 10 min at 4 °C in a Sorvall SS34 rotor. The supernatants were transferred to fresh tubes and centrifugation was repeated. The resultant supernatant was applied carefully above 25 ml of sucrose cushion (1.3 M sucrose, 50 mM HEPES-KOH, pH 7.6, 50 mM KOAc, 6 mM Mg(OAc)₂, 1 mM EDTA, 1 mM DTT, 10 µg/ml PMSF) in four 70 ml polycarbonate tubes and centrifuged at 35,000 rpm for 1 hour at 4 °C in a Beckman Ti 45 rotor. The supernatant was discarded and the membrane pellet, that consisted of rough microsomes (RMs), was resuspended in 15 ml RM buffer (250 mM sucrose, 50 mM HEPES-KOH, pH 7.6, 50 mM KOAc, 2 mM Mg(OAc)₂, 1 mM DTT, 10 µg/ml PMSF) using a Dounce homogeniser. The absorption at 260 nm and 280 nm of a 1:1000 dilution of the RM suspension was measured in 0.5 % (^{w}/ᵥ) SDS. Typically, an absorption of 0.05-0.1 A₂₈₀/ml and a ratio A₂₆₀/A280 of ~1.6 were obtained.
Aliquots were frozen in liquid nitrogen and stored at -70 °C until use.

### b) Extraction of Ribosomes, Nascent Polypeptides and Proteins Peripherally Associated with the Cytosolic Surface of Microsomes

10-20,000 eq (500-1000 A₂₈₀) RMs, suspended in RM buffer, were centrifuged at 36,000 rpm for 1 hour at 4 °C in a Beckman Ti 70 rotor. The resulting RM pellet was resuspended in 15 ml of high salt buffer (50 mM HEPES-KOH, pH 7.6, 500 mM KOAc, 2 mM Mg(OAc)₂, 250 mM sucrose, 1 mM DTT, 10 µg/ml PMSF) using a Dounce homogeniser. Puromycin and GTP (final concentrations of 2 mM and 0.33 mM respectively) were added and the sample was rotated for 20 min at room temperature. 13.8 g sucrose (final concentration 1.8 M) was then added and incubation was continued at room temperature until the sucrose dissolved (approx. 15 min). The sample was distributed between four polycarbonate tubes (6 ml per tube) and overlaid with 3 ml of 1.5 M sucrose cushion (50 mM HEPES-KOH, pH 7.6, 500 mM KOAc, 2 mM Mg(OAc)₂, 1.5 M sucrose, 1 mM DTT, 10 µg/ml PMSF). Tubes were filled with RM buffer and the samples centrifuged at 36,000 rpm for 16-18 hours at 4 °C in a Beckman SW41 rotor. Puromycin/high salt washed RMs (PK-RMs) were collected from the top of the 1.5 M sucrose cushion and resuspended in RM buffer using a Dounce homogeniser (total volume approx. 25 ml).

### c) Depletion of Lumenal Proteins

25 ml of PK-RM suspension (prepared from 10-20,000 eq of RMs) were diluted with 250 ml of depletion buffer (50 mM HEPES, 50 mM CAPS adjusted to pH 9.6 with KOH) and incubated on ice for 30 min. Membranes (PKX-RMs) were recovered by centrifugation in a Beckman Ti 45 rotor at 40,000 rpm for 1 hour at 4 °C.

### d) Solubilisation of Membrane Proteins with CHAPS

Pelleted PKX-RMs were resuspended in 15 ml of solubilisation buffer (50 mM HEPES-KOH, pH 7.6, 50 mM KOAc, 2 mM Mg(OAc)₂, 125 mM sucrose, 1 mM DTT, 10 µg/ml PMSF, 2 % CHAPS) using a Dounce homogeniser. The sample was rotated for 1 hour at 4 °C. Proteins that failed to solubilise in the presence of detergent were removed by centrifugation (Beckman TLA100.4 rotor, 75,000 rpm, 1 hour, 4 °C). The supernatant contained CHAPS-solubilised ER membrane proteins.

### 12. Photo-Affinity Labelling of SPPase

### a) Chemical Synthesis of TBL₄K

A scheme for the synthesis of TBL₄K is presented in Fig. 7. TBL₄K was synthesised by initially coupling an excess of commercially available BOC-LL-OH (Bachem) with diamino acetone using the coupling reagent HATU and collidine. The resulting product was isolated by silica gel column chromatography and identified by nuclear magnetic resonance spectrometry. The BOC-capped derivative was de-protected by brief treatment with trifluoroacetic acid followed by precipitation in hexane. The two free amino termini of (H₂N-LL)₂ ketone were next modified with-1 equivalent of TDBAOSu (a gift from Prof. Brunner, ETH, Zurich) and 1 equivalent of Biotin-X-OSu (Molecular Probes). (TDBA-LL)(Biotin-X-LL) ketone (= TBL₄K) was isolated by preparative thin layer chromatography and identified by mass spectrometry.

### b) Inhibition of SPPase with TBL₄K

The inhibitory effect of TBL₄K on SPPase was assayed with CHAPS-solubilised ER membrane proteins and an *in vitro* translated peptide substrate. The substrate was a 29 aa residues long mutant signal peptide of pre-prolactin (p-Prl^{PP}/29; MDSKGSSQKGSRLLLLLVVSNLLLCQG**PP**). The VV→PP mutation at the end of the signal peptide prevents cleavage by signal peptidase, which can cleave the peptide at the cryptic cleavage site at G₂₇ when solubilised in CHAPS (B.M. unpublished). To make *in vitro* translated peptide, the coding region for p-Prl^{PP}/29 was first amplified from pGEM3Z/p-Prl (Weihofen et al., 2000 *J Biol Chem* **275**, 30951-30956) by PCR using *Pfu* DNA polymerase (Stratagene), a SP6 oligonucleotide primer and a reverse primer with the nucleotide sequence 5'-NNNNNNNNNCTACGGCGG-3' to insert a translational stop at codon 30 and the mutations for residues 28 and '29. RNA encoding this peptide was transcribed *in vitro* from the PCR-amplified product (see Section 3, Materials and Methods). Translation of RNA to produce radio-labelled p-Prl^{PP}/29 peptide was performed *in vitro* as described in Section 3 except that wheat germ extract (Promega) was utilised. 2 µl of the translation mixture (containing radio-labelled p-Prl^{PP}/29 peptide) was diluted with 35 µl SPPase buffer (25 mM HEPES-KOH, pH 7.6, 100 mM KOAc, 2 mM Mg(OAc)₂, 1 mM DTT) and 0.8 µl of SPPase inhibitor TBL₄K, dissolved as a 50x stock in DMSO, was added. Reactions were initiated by addition of 2 µl CHAPS-solubilised membrane proteins and samples were incubated for 1 hour at 30 °C. Proteins were precipitated by adding 4 µl of 100 % trichloroacetic acid, and analysed by SDS-PAGE using Tris-bicine-urea acrylamide gels (15 % T or 10 % T, 5 % C; 8 M urea) *(*Wiltfang *et al., 1997 Electrophoresis* **18,** 527-532).

### c) Labelling of Protein with TBL₄K

For analytical labelling, 2 µl of CHAPS-solubilised ER membrane proteins were diluted with 16 µl of SPPase buffer (25 mM HEPES-KOH, pH 7.6, 100 mM KOAc, 2 mM Mg(OAc)₂, 1 mM DTT) and supplemented with 50 nM TBL₄K (dissolved in DMSO). Samples were incubated at 30 °C for 1 hour and subsequently irradiated with UV light (30 seconds; 350 W high pressure mercury lamp with a pyrex filter, 10 cm distance to lamp). Proteins were precipitated with 10 % trichloroacetic acid and resolved on either Tris-glycine acrylamide gels (10 % T, 2.7 % C) (Lämmli, 1970 *Nature,* **227**, 570-574). Biotinylated proteins were visualised by Western blotting with a polyclonal anti-biotin antibody (Bethyl). Western blot analysis was performed as described in Section 3, Materials and Methods for R308 antisera.

For preparative labelling, membrane proteins were reacted with TBL₄K on a large scale. 15 ml of CHAPS-solubilised ER membrane proteins were diluted with 120 ml of SPPase buffer and supplemented with 20 µl of 500 µM TBL₄K in DMSO. The sample was incubated at 30 °C for 1 hour. For irradiation with UV light, the sample was distributed between four 50 ml polypropylene tubes (Falcon) and irradiated for 30 seconds. The aliquots were then pooled and proteins separated by column chromatography.

### 13. Column Chromatography

### a) ConA Sepharose Chromatography

CHAPS-solubilised ER membrane proteins (135 ml) containing TBL₄K-labelled species were supplemented with 15 ml of 5 M NaCl and 2.6 ml of 20 % reduced Triton X-100 (Sigma). The sample was applied to a 1 ml Concanavalin A-sepharose column (Amersham-Pharmacia), that had been equilibrated with EQ buffer I (50 mM HEPES-KOH, pH 7.6, 500 mM NaCl, 20 mM sucrose, 1 mM DTT, 0.35 % reduced Triton X-100). The'sample was circulated 5 times over the column at 0.2 ml/min and 4 °C. The column was next washed with 5 ml of EQ buffer I, and bound proteins containing TBL₄K-labelled species were eluted at room temperature with 15 ml elution buffer (1M methyl- -D-glucopyranoside, 50 mM HEPES-KOH, pH 7.6, 500 mM NaCl, 1 mM DTT, 0.35 % reduced Triton X-100).

### b) Hydroxylapatite Chromatography

Eluate (15 ml) from the ConA column was diluted 10 times with 50 mM HEPES-KOH, pH 7.6, 1 mM DTT, 0.35 % reduced Triton X-100 to reduce salt concentration. The sample was applied to a 2.5 ml hydroxylapatite column (BioRad), which had been equilibrated with EQ buffer II (50 mM HEPES-KOH, pH 7.6, 50 mM KOAc, 1 mM DTT, 0.35 % reduced Triton X-100) at 0.2 ml/min and 4°C. The column was next washed with 12.5 ml of EQ buffer II, and bound proteins containing TBL₄K-labelled species were eluted at room temperature with 3 ml elution buffer (50 mM HEPES-KOH, pH 7.6, 500 mM KOAc, 200 mM KPᵢ, 1 mM DTT, 0.35 % reduced Triton X-100).

### c) Reversed Phase HPLC

Eluate (3 ml) from the hydroxylapatite column was supplemented with 300 µl of 100 % trichloroacetic acid to precipitate proteins. After centrifugation (Eppendorf centrifuge, 14,000 rpm, 4 °C, 5 min), the protein pellet was washed with acetone and resuspended in 50 % formic acid in H₂O. The sample was applied to a RP4 reversed phase HPLC column (Machery Nagel, CC 125/4 Nucleosil 300-5 C4), which had been equilibrated with 50 % formic acid. The flow rate was 1 ml/min. Proteins were first eluted with a linear gradient of 50 % formic acid in H₂O to 50 % formic acid in acetonitrile. The column was next re-equilibrated with 50 % formic acid in H₂O and residual proteins including TBL₄K-labelled species were eluted with a linear gradient of 50 % formic acid in H₂O to 50 % formic acid in propan-2-ol.

### d) SDS-PAGE

Fractions containing the TBL₄K-labelled species were pooled and proteins were resolved by SDS-PAGE using a Tris-glycine acrylamide gel (10 % T, 2.7 % C) (Lämmli, 1970 *Nature,* **227,** 570-574). Proteins were visualized by staining with Coomassie brilliant blue and only 5 well resolved proteins remained in this fraction. Western blotting using anti-biotin antibody identified the TBL₄K-labelled protein. The corresponding Coomassie stained band was excised from the gel and subjected to sequencing by mass-spectrometry.

### 13. Sequencing by Mass Spectrometry

Peptide sequences were identified by the Protein Analysis Unit (University of Zurich).

### DISCUSSION

### 1. The Signal Sequence at the HCV core-E1 Junction is a Substrate for Clea-Veige by Signal Peptidase and Signal Peptide Peptidase.

Previous *in vitro* studies have provided evidence that the signal sequence separating the core and E1 proteins is cleaved in the presence of microsomal membranes, suggesting proteolysis by signal peptidase. This processing event was re-examined in an *in vitro* translation/translocation system. Two RNA substrates, that had been transcribed *in vitro,* were employed. One contained the E1 signal sequence and the N-terminal 100 aa of E1 (SP-E1/100), and the second consisted of the entire core protein, including the signal sequence, plus the N-terminal 100 aa of E1 (core-E1/100). These were translated in reticulocyte lysate in the presence of endoplasmic reticulum- (ER) derived rough microsomes and [³⁵S]-methionine. Microsomes were subsequently isolated and analysed for retention of radiolabelled translation products.

The protein targeting and membrane insertion functions of the internal signal sequence between core and E1 proteins was first examined with a precursor, SP-E1/100, which encoded HCV aa residues 161 to 291 preceded by a translation initiator codon. Translation of SP-E1/100 mRNA in the presence of microsomes yielded N-glycosylated E1/100 (E1/100g) indicating translocation of the protein into the microsomes (Figure 1A, lane 2). Inhibiting N-glycosylation by addition of acceptor tripeptide resulted in the generation of E1/100, which, in turn, revealed removal of the signal peptide by signal peptidase at residue 191 (Figure 1A, lane 3). The released signal peptide should have an apparent molecular weight of about 4 kDa and co-migrate with a reference peptide (Figure 1A, lane 5). However, a product of this size was barely detectable in the membrane fraction suggesting that it had been further processed and released from the membrane (Figure 1A, lanes 2 and 3).

It has been suggested that processing of the signal peptide could be performed by either signal peptidase or an unknown SPPase activity in microsomal membranes (Hüssy *et al.,* 1996 *Virology* **224,** 93-104). In previous studies, we have shown that (Z-LL)₂ ketone is a specific inhibitor of mammalian SPPase but does not affect cleavage by signal peptidase (Weihofen *et al.,* 2000 *J Biol Chem* **275,** 30951-30956). On addition of (Z-LL)₂ ketone to reactions, the signal peptide generated from SP-E1/100 was readily detected and co-migrated with the reference peptide, indicating that cleavage by SPPase had been blocked (Figure 1A, lanes 4 and 5). These results demonstrate that N-terminal of a pre-protein, the internal signal sequence at the junction of core-E1 functions as a typical ER targeting signal and is a substrate not only for signal peptidase but also for SPPase.

The function of the signal sequence at the junction of core-E1 was next assayed in the context of the entire core protein. Translation of core-E1/100 in the presence of microsomes yielded several products including unprocessed precursor protein and a 12 kDa product that corresponded to unglycosylated E1 (E1/100), which had been generated by signal peptidase cleavage of the precursor protein (Fig. 1B, lane 2). Two other major bands migrated with apparent molecular weights of 21 kDa and 19 kDa. The higher molecular weight species comprised two proteins, the glycosylated form of E1 (E1/100g) and core protein that was the N-terminal product of signal peptidase cleavage of the precursor protein at residue 191. To confirm that the 21 kDa species contained both of these proteins, acceptor tripeptide was added to reactions to block N-glycosylation of E1. Accordingly, the intensity of the 21 kDa band was reduced while there was an increased amount of unglycosylated E1 (Figure 1B, lane 3). The 19 kDa species has been observed previously and corresponds to core protein that is further processed at the region containing the signal peptide. This species also co-migrated with a reference peptide of 179 aa and with mature core protein generated in tissue culture cells by a plasmid pSF/CE1E2 that expressed the N-terminal 837 aa of the HCV polyprotein (Figure 3A, wt, lanes 2-4). Addition of (Z-LL)₂ ketone to reactions blocked production of the 19 kDa species but not the 21 kDa protein that corresponds to core generated by signal peptidase (Fig. 1B, lanes 4 and 5). Together with the results presented above, these data provide compelling evidence that the signal peptide at the junction of core-E1 sequences in HCV is first cleaved by signal peptidase and subsequently processed by SPPase.

### 2. Processing of Core Requires Helix-Breaking Residues in the Transmembrane Region of the Signal Peptide Sequence.

SPPase promotes intramembrane proteolysis at helix-breaking residues in the centre of the hydrophobic region of a signal sequence (Weihofen *et al.,* 2000, *J. Biol. Chem.* **275,** 30951-30956). Based on these criteria, the signal sequence between core and E1 contains two potential SPPase cleavage sites, Leu₁₇₉/Ala₁₈₀ (Site 1, a minor helix bend in the centre of the hydrophobic region), or Leu₁₈₂/Ser₁₈₃-Cys₁₈₄ (Site 2, the major helix-break in the transmembrane region). Replacing helix-breaking residues in the hydrophobic region of a signal sequence with amino acids that have long, hydrophobic side chains reduces the susceptibility of the signal peptide to SPPase processing, but does not affect the function of the signal sequence in protein targeting, translocation, and cleavage by signal peptidase (M. Lemberg and B. Martoglio, unpublished data). In an attempt to determine the SPPase cleavage site(s) in the HCV core signal sequence, processing was assayed with three mutants (Table 1). Two mutants had substitutions at either of the predicted sites (Ala₁₈₀→Val₁₈₀ [MUTI] and Ser₁₈₃/Cys₁₈₄→Leu₁₈₃/Val₁₈₄ [MUTII]) and a double mutant that combined mutations at both sites (Ala₁₈₀/Ser₁₈₃/Cys₁₈₄→ Val₁₈₀/Leu₁₈₃/Val₁₈₄ [MUTIII]).

*In vitro* translation/translocation assays with SP-E1/100 precursors revealed less than 5% processing of the signal peptide by SPPase with pSP/MUTIII (Fig. 2A, MUTIII, compare lanes 2 and 3; Fig. 2B). Analysis of the constructs with mutations at either of the recognition sites showed that cleavage of the signal peptide was reduced with both mutants, but to a greater extent with pSP/MLTTII as compared to pSP/MUTI (Fig. 2A, MLTTI and MUTII, lanes 2 and 3; Fig. 2B). With the corresponding mutant core-E1/100 precursors (Table 1), the data had a similar pattern although the effects were more marked. Hence, mutation of Site 1 reduced efficiency of processing from 40% to 12% (Fig. 3A, compare wt and MUTI, lanes 4; Fig. 3B). The two other precursors, C-MUTTI and C-MUTIII, failed to generate any detectable product that had been cleaved by SPPase (Fig. 3A, MUTII and MUTIII, lanes 4; Fig. 3B).

Transferring the signal peptide mutants into SFV construct, pSF/CE1E2, we tested the effect of these mutations on the extent of processing of core in tissue culture cells. The constructs were termed pSF/MUTI, pSF/MUTII and pSF/MUTIII (Table 1). Extracts from cells electroporated with RNA from these constructs were examined alongside corresponding *in vitro* reactions by Western blot analysis (Fig. 3A). The data showed that mutation at Site 1 did reduce the amount of mature core protein produced by SPPase (Fig. 3A, MUTI, lane 3) but to a lesser extent than was detected *in vitro.* For pSF/MUTII and pSF/MUTIII, the core proteins made by these constructs co-migrated with *in vitro* translated products of 191 aa. This represents cleavage by signal peptidase but there was no evidence of processed forms produced by SPPase (Fig. 3A, MUTII and MUTIII, lanes 3). We conclude that mutation at the helix-break positions 183 and 184 (Site 2) exerts a far greater effect on SPPase processing of core as compared with alteration at position 180 (Site 1). Given that the mature core protein migrates with a reference peptide of 179 aa and not 182 aa, it is likely that the helix-break at Site 2 does not constitute the cleavage site for SPPase but is critical for processing by the protease.

### 3. Mutation at the Signal Peptide Peptidase Sites in the Core-E1 Signal Peptide Blocks Trafficking of Core to Lipid Droplets.

A defining feature of core protein in mammalian cells after processing from the HCV polyprotein is its association with cytoplasmic lipid droplets (Hope and McLauchlan, 2000, *J Gen Virol* **81,** 1913-1925). To test whether processing by SPPase in the core-E1 signal peptide influenced the properties of the proteins in tissue culture cells, the mutations analysed using the *in vitro* system were examined using the pSF series of plasmids. Western blot analysis of cell extracts indicated that pSF/CE1E2 and each of the pSF/MUT constructs produced glycosylated E1 (Figure 4B, lanes 2-5) of identical apparent molecular weight. From Endo H digestion of the samples, the native forms of E1 (Figure 4B, lanes 6-9) also had the same mobilities on gels. Moreover, there was no evidence that uncleaved core-E1 species could be detected either by core (data not shown) or E1 antibodies (Figure 4B). Finally, immunofluorescence analysis did not detect any difference in the localisation of E1 (data not shown) and E2 made from any of the constructs and is consistent with association with the ER (Figure 4A). We conclude from these data that mutations introduced into the signal peptide do not affect the translocation and cleavage of HCV precursor molecules by signal peptidase. This is in agreement with the *in vitro* data presented in Figures 1-3.

The core protein produced by pSF/CE1E2 can be detected at the surface of cytoplasmic lipid droplets by combining indirect immunofluorescence and specific staining of these storage structures (Fig. 5; Hope and McLauchlan, 2000, *J.Gen Virol* **81**, 1913-1925). An identical localisation was observed for the core species made by pSF/MUTI (Figure 5). By contrast, constructs pSF/MUTII and pSF/MUTIII generated core proteins that were distributed in a reticular pattern throughout the cytoplasm and showed no co-localisation with lipid droplets (Figure 5). The distributions of these mutant forms of core were indistinguishable from those for E1 and E2 (Figure 4A), indicative of localisation at the ER. Western blot analysis of core produced by pSF/CE1E2 identified a product that co-migrated with a 179 aa reference core protein and core protein produced *in vitro* by SPPase (Figure 3B, wt, lanes 1-4). The major core product made by pSF/MUTI also had an identical size to that generated by the wt construct (Figure 3B, I, lanes 1-4). However, a small amount of protein, with a size of 191 aa and consistent with cleavage only by signal peptidase, also was detected in cell extracts. This is in agreement with the *in vitro* data, which indicate that processing at the signal peptide is reduced by mutation at aa residue 180. By contrast, pSF/MUTII and pSF/MUTIII only gave core proteins of 191 aa, indicating processing by signal peptidase alone (Figure 3B, II and III, lanes 1-4). These data emphasise that mutation at positions 183 and 184 (Site 2) in the signal peptide have a pronounced inhibitory effect on processing by SPPase. We postulate that inhibiting cleavage in the transmembrane region of the signal peptide abolishes the release of core protein from the ER membrane and subsequent association with lipid droplets.

### 4. Release of Core Protein from the ER Requires Cleavage by SPPase

The lipid droplets present in the cytoplasm are not bound to the ER and thus proteins attached to droplets, such as SPPase-processed core, should be distinct from membrane-associated species. In addition, the lipid droplet binding sequences in core have the capacity to direct the protein to the ER to permit processing of the signal peptide by signal peptidase and SPPase (Hope and McLauchlan, 2000, *J. Gen. Virol.* **81,** 1913-1925). However, the lipid droplet binding sequences in core are less hydrophobic than those in the signal peptide and hence may associate with the ER in a different manner from the signal peptide. To examine the relative properties of wild-type core and the signal peptide mutants, the ability to detect core protein in cytosolic fractions both before and after extraction of membranes with Na₂CO₃ was determined. Results revealed that core proteins made by wild-type construct pSF/CE1E2 and pSF/MUTI; which are substrates for SPPase, could be detected in cytosolic fractions after separation from membranes (Figure 6, CE1E2 and MUTI, lanes 2). Given the intracellular distribution of the protein, this represents core attached to lipid droplets. By contrast, core proteins produced by both pSF/MUTII and pSF/MUTIII, which are not processed by SPPase, were absent in such fractions (Figure 6, MUTII and MUTIII, lanes 2). Following treatment of membranes with Na₂CO₃, a proportion of core made by pSF/CE1E2 and pSF/MUTI could be extracted (Figure 6, CE1E2 and MUTI, compare lanes 4 and 5). In the case of pSF/MUTII and pSF/MUTIII, Na₂CO₃ did not remove core from membranes to any appreciable extent and almost no protein was detected in supernatant fractions (Figure 6, MUTII and MUTIII, compare lanes 4 and 5). Since the core proteins made by pSF/MUTII and pSF/MUTIII are cleaved by signal peptidase alone, the results conclusively show that release of core from the ER to allow association with lipid droplets requires processing by SPPase. Moreover, core associated with membranes via the lipid droplet binding sequences is less avidly attached to membranes compared to core that contains both the lipid droplet binding sequences and the core-E1 signal peptide.

### 5. Release from the ER Membrane and Degradation of an Unstable Form of Core is Inhibited by Abolishing Signal Peptide Peptidase Cleavage.

To test whether inhibition of SPPase prevents release of core from the ER membrane, we made use of a variant of core that lacks aa between residues 125 and 144. Without this hydrophobic region, core does not associate with lipid droplets and is degraded by the proteasome in the cytosol upon release from the core-E1-E2 polyprotein (Hope and McLauchlan, 2000, *J Gen. Virol,* **81**, 1913-1925), We hypothesised that blocking cleavage by SPPase may reduce degradation since immature core protein would be retained at the ER membrane. Hence, a construct, pSF/ MUTIII (Table 1) was made that lacked sequences between residues 125 and 144 and had all three mutations in the signal sequence. Comparing levels of protein made by this construct and pSF/ CE1E2 (as well as pSF/CE1E2) did not reveal any differences in the amount of E1 (data not shown) and E2 present in cell extracts (Figure 7C, lanes 1 and 3-6). However, there was a considerable difference in the relative amounts of core protein. In the case of pSF/ CE1E2, core protein was barely detected yet it was readily recognised in pSF/ MUTIII cell extracts (Figure 7B, lanes 3 and 5). To confirm that pSF/ CE1E2 did produce core protein, cells were incubated in the presence of the proteasome inhibitor MG132. In agreement with our previous analysis (Hope and McLauchlan, 2000, *J Gen. Virol,* **81**, 1913-1925), enhanced levels of core produced by pSF/ CE1E2 were detected upon treatment of cells with MG132 (Figure 7B, lanes 3 and 4). This difference in detectable amounts of core is evident also from indirect immunofluorescence analysis (Figure 7A). By contrast, MG132 did not affect the amount of core recognised in cells electroporated with pSF/ MUTIII RNA (Figure 7B, lanes 5 and 6). These results are consistent with cleavage of core, made from pSF/ MUTIII, with signal peptidase but not SPPase, leading to anchoring of the immature protein at the ER membrane. Failure to release core from the membrane by SPPase-catalysed proteolysis does not expose the protein to the degradative processes that act on core made by pSF/ CE1E2.

### 6. Inhibition of SPPase Activity by TBL₄K.

As disclosed above, the data unveiled inhibition of signal peptide processing by SPPase activity using the symmetrical protease inhibitor, (Z-LL)₂ ketone. This prompted the inventors of the present invention that the inhibitor targets the active site residue of SPPase. To identify the protein target, the inventors of the present invention solubilised membrane proteins from canine pancreas microsomes and subsequently labelled the proteins with a derivative of (Z-LL)₂ ketone-termed TBL₄K (Figure 8). To test first whether-TBL₄K could inhibit SPPase activity, signal peptide processing reactions were performed with a substrate, p-Prl^{PP}/29, that corresponds to a mutated signal peptide of pre-prolactin, and CHAPS-solubilised ER membrane proteins containing SPPase activity. Addition of TBL₄K to reactions progressively inhibited SPPase activity as its concentration increased (IC₅₀ ~50 nM; Figure 9, lanes 3-7). At the highest concentration used (1 M), SPF production was abolished by TBL₄K. This concentration and the IC₅₀ value are within the range for inhibition of SPPase by (Z-LL)₂ ketone (Weihofen *et al.,* 2000, *J Biol Chem,* **275**, 30951-30956) and indicates that the modifications in TBL₄K do not affect its ability to target SPPase.

### 7. Photo-Labelling of the Protein Target for TBL₄K.

The chemical properties of TBL₄K and (Z-LL)₂ ketone suggest that they both reversibly attack the active site residue of the elusive SPPase via their central ketone moiety. However, when activated with UV light (364 nm), the diazirin moiety of TBL₄K is converted to a highly reactive carbene, which will irreversibly react within nanoseconds with its immediate neighbouring molecule irrespective of its chemical nature. Thus, in a protein solution, TBL₄K preferentially labels the protein to which it has high affinity. To identify the protein bound to TBL₄K, CHAPS-solubilised membrane proteins were mixed with the inhibitor and exposed to UV light. Western blot analysis using anti-biotin antibody to detect the biotin moiety in TBL₄K revealed labelling of a major product of about 42 kDa; a minor product with slightly greater mobility (~40 kDa) also was detected (Figure 10, lane 3). To confirm the specificity of TBL₄K for these species, (Z-LL)₂ ketone was added to reactions before UV activation. Increasing the concentration of (Z-LL)₂ ketone progressively reduced the abundance of the TBL₄K-labelled proteins and, labelling by TBL₄K was almost completely abolished at the highest concentration of (Z-LL)₂ ketone (Figure 10, lanes 4-8). This indicates that the major 42 kDa protein as well as the minor 40 kDa species are targeted by both TBL₄K and (Z-LL)₂ ketone. Treatment with endoglycosidase H shifted both proteins into a single band of ~38 kDa suggesting that they are identical but differentially glycosylated (data not shown).

### 8. Identification of the Protein Target for TBL₄K.

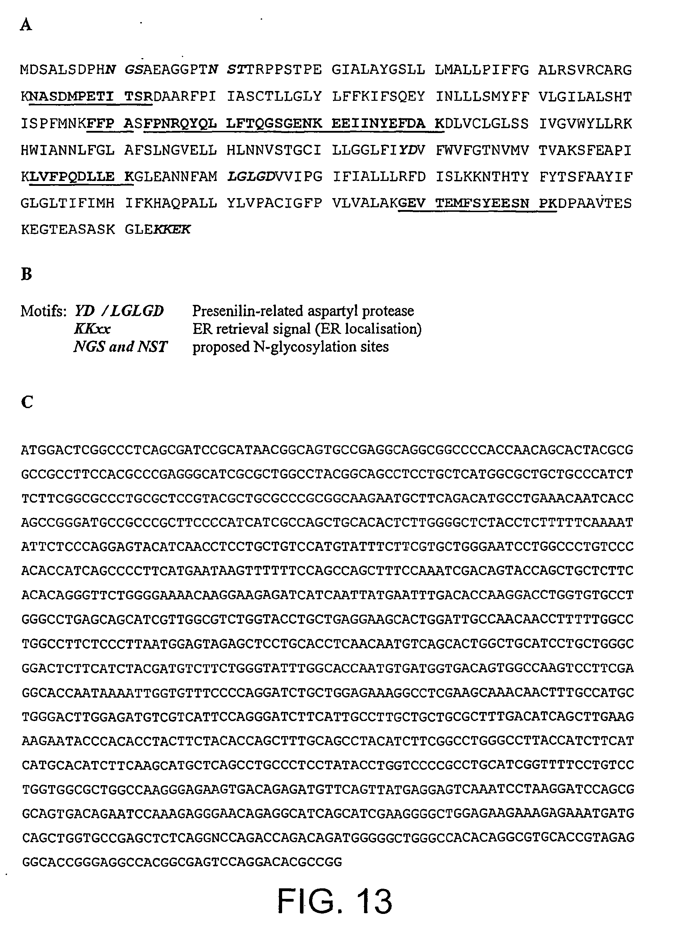

To identify the 42 kDa species, photo-labelling with TBL₄K was performed on a preparative scale and the labelled protein was further purified by chromatography (scheme outlined in Figure 11). At each stage of purification, the presence of TBL₄K-labelled protein in the respective fraction was confirmed by Western blot analysis using anti-biotin antibody. Following the final stage of separation by reverse phase HPLC, SDS-PAGE analysis of pooled fractions containing TBL₄K-labelled protein and subsequent Coomassie staining revealed only 5 well separated proteins. Among the stained bands, the TBL₄K-labelled protein was identified by Western blot analysis. This band was excised from a preparative gel and analysed by mass spectrometry. Sequences were obtained for 6 peptides (Figure 12) that were compared with predicted translated products in databases. With the exception of 2 aa residues highlighted in peptides 2 and 4 (Figure 12), all of the peptide sequences matched a human protein of unknown function (accession number gi 14772424, homo sapiens). The predicted human protein (Figure 13, SEQ ID No: 1 and No: 2) contains 7 putative transmembrane regions, two N-glycosylation sites close to the N-terminus (with two other N-glycosylation sites predicted to be located in cytosolic loops) and a C-terminal ER-retrieval signal, KKEK. In addition, the protein contains the characteristic aspartyl protease motifs (YD and LGLGD) in opposite transmembrane regions as is found in presenilin, the putative γ-secretase, which holds a key function in the development of Alzheimer's disease.

It is concluded that the identified protein is identical to SPPase.

In the absence of any previous formal definition of the activity that cleaves signal peptides, we propose that SPPase is a presenilin-related, ER localised aspartyl protease that promotes intramembrane proteolysis of signal peptides. Further comparisons with databases have indicated that there are proteins of unknown function with sequence identity to the human SPPase encoded by genomes from *Mus musculus, Caenorhabditis elegans, Drosophila melanogaster, Arabidopsis thaliana, Saccharomyces pombe* and *Saccharomyces cerevisiae*

This suggests that SPPase is conserved in all eukaryotic organisms.

### 9. In Vitro Binding Assay to Test Effects of Candidate Agents. In vitro synthesis of HCV-core peptide peptidase targeting sequences and recombinant SPPase protein made exogenously.

Constructs suitable for *in vitro* synthesis of mRNA are produced by placing nucleotide sequences encoding core protein (e.g. pgHCV/CE1E2) or any other protein with HCV-core peptide peptidase targeting sequences (e.g. pgHCV CE1E2) are placed under the control of a suitable bacterial or bacteriophage RNA polymerase promoter e.g. SP6 or T7. RNA transcripts are prepared from each of these constructs using standard methods and the yields of RNA determined. Recombinant SPPase protein can be obtained in a detergent-soluble form (See Sections 10 and 11b in the materials and Methods and Section 5 in the Results).

To produce *in vitro* translated protein, the *in vitro* synthesised RNA is added to an extract capable of synthesising polypeptides e.g. a reticulocyte lysate prepared from rabbits or an extract from wheat germ; proteins may be radio-labelled by including an isotopically labelled amino acid such as ³⁵S-methionine.

### Assessing binding of proteins to lipid

Membranous material prepared from animal cells (e.g. microsomal membranes) or synthetically prepared mixtures of triacylglycerol or cholesterol and phospholipid are mixed with the translated proteins and incubated. Lipid fractions are then recovered by centrifugation. The incorporation of proteins into lipid fractions may be determined either directly by SDS-PAGE of radio-labelled proteins, or indirectly by using antibodies which are functional in Western blot and/or ELISA procedures.

Combinations of RNA from core protein and SPPase may be mixed in the same reaction in various proportions to test the relative affinity of each protein for lipid. An example of a combination would be 9 parts pgHCV/CE1E2 RNA to 1 part SPPase RNA.

Candidate agents at a range of concentrations, such as from 1 M to 100 mM, are added to reactions and the effect of these agents on protein binding to lipid analysed using the methods indicated above. A suitable candidate agent is typically an agent that enhances or does not significantly impair lipid association of core but which reduces or abolishes binding of core to SPPase.

### 10. In Vivo Binding Assay to Test Effects of Candidate Agents.

BHK C13 cells are electroporated with SFV encoding HCV core protein (for example SFV.1-195) as described above. At various time points after electroporation, candidate agents are added to cells at concentrations which are not cytotoxic (as determined with mock electroporated cells, for example). Following incubation at 37 °C, cells are harvested and cell extracts prepared. Extracts are analysed by Western blot analysis (antibody JM122) to examine the abundance of core protein and determine the efficiency of cleavage at the internal processing site. The relative abundance of core protein in treated as compared to untreated cells could also be quantitated by ELISA.

In addition, cells are fixed and examined by a combination of indirect immunofluorescence (for the core and SPPase proteins) and oil red oil staining (for lipid droplets) as described above. This can be used to determine whether the intracellular distribution and abundance of core and SPPase has been altered by the presence of the candidate agents. Western blot analysis may also be used to confirm whether the candidate agent had any affect on SPPase levels.

A suitable candidate agent is typically an agent that prevents or reduces core association with droplets, causes reduced levels of core protein and/or impaired cleavage at the internal processing site. By contrast, the candidate agent should preferably not affect the intracellular distribution of SPPase and its abundance is typically either similar to or higher than in cells that do not express the core protein.

### 11. Assessing anti-viral affects of candidate agents in transgenic animals expressing core or in chimpanzees

In transgenic animals expressing liver-specific core protein or animals (chimpanzees) infected with HCV, test agents are added at concentrations which were not toxic to the host.

### i) Transgenic animals expressing core protein in a liver-specific manner

Transgenic mice which give liver-specific expression of core protein are known in the art. Expression of core protein is associated with the development of steatosis and hepatocellular carcinoma in two lines of such animals (Moriya, K. *et al.,* 1998, *Nature Medicine,* **4**, 1065-1067; Moriya, K. *et al.,* 1997, *J Gen Virol,* **78**, 1527-1531); both pathologies are associated with HCV infection. Similar transgenic mice may be produced with similar phenotypes and used to examine the effect of agents which prevent core association with lipid droplets on these pathological changes.

The effect of candidate agents on core protein localisation and levels may be determined in transgenic animals using cells obtained by liver biopsies and tested using the techniques described in Example 8. Alternatively, or in addition, the effect of a candidate agent of the development of steatosis and hepatocellular carcinoma in these animals may be determined. The efficacy of candidate agents may be measured by a reduction in the pathological changes which occur e.g. reduced hepatosteatosis and significant delays or prevention of the onset of carcinoma.

### ii) Chimpanzees infected with HCV

The effect of an agent on HCV replication in infected chimpanzees is assessed by RT-PCR analysis of sera taken at regular intervals from the animal. Biopsy material from the liver may also be tested for the presence of negative-strand HCV RNA by RT-PCR using standard techniques (see Conry-Cantilena, 1997, *Tibtech* **15**, 71-76, for review and references contained therein). Efficacy of the candidate agent is assessed by the reduction in the levels of HCV RNA as measured in either or both assays. A reduction in viral titre by a factor of at least 2 to 3 logs is indicative of an anti-viral effect.

## Claims

1. A method for identifying a candidate agent capable of affecting a viral infection, which method comprises:
(a) providing a first component comprising a signal peptide peptidase cleavage targeting sequence;
(b) providing a second component comprising a signal peptide peptidase;
(c) contacting the two components with an agent to be tested under conditions that would permit the two components to interact in the absence of the agent; and
(d) determining whether the agent modulates the interaction between the first and second components
wherein the signal peptide peptidase targeting sequence is derivable from hepatitis C virus (HCV) core protein or a homologue thereof having at least 60% identity thereto.

2. A method according to claim 1, wherein the candidate agent is administered to a cell and wherein, the signal peptide peptidase cleavage targeting sequence is expressed in said cell.

3. A method according to claim 1 or claim 2, wherein the signal peptide peptidase is a recombinant signal peptide peptidase or a natural constituent of said cell.

4. A method according to any of the preceding claims further comprising:
(a) administering a virus to a cell in the absence of said candidate agent which has been determined to modulate the interaction between the first and second components;
(b) administering the virus to the cell in the presence of the said agent; and
(c) determining if the said agent reduces or abolishes the susceptibility of the cell to viral infection or the effects of viral infection.

5. A method according to claim 4, wherein the signal peptide peptidase targeting sequence comprises SEQ ID No: 3 of the HCV core protein or a homologue thereof having at least 60% identity thereto.

6. A method for identifying an agent for treating or preventing a hepatitis infection or other viral infection of the human or animal liver, which method comprises determining whether said agent can modulate expression or activity of a signal peptide peptidase enzyme in a mammalian cell.

7. A method according to claim 6, wherein said cell is a liver cell.

8. A protein fragment consisting of a signal peptide peptidase targeting sequence
wherein said fragment consists of SEQ ID No: 3 of the HCV core protein or consists of SEQ ID No: 4, 5 or 6.

9. A protein fragment according to claim 8 for use in preventing or treating a viral infection.

10. A polynucleotide encoding the protein fragment according to claim 8 for use in treating a viral infection.

11. Use of the protein fragment according to claim 8 or the polynucleotide according to claim 9 in the manufacture of a medicament for use in treating a viral infection.

12. A method according to any of claims 1-5, wherein the viral infection is a hepatitis infection or other viral infection of the human or animal liver.

## Patentansprüche

1. Verfahren zum Identifizieren eines Kandidatenmittels, welches eine virale Infektion beeinflussen kann, wobei das Verfahren folgendes umfaßt:
(a) Bereitstellen einer ersten Komponente, welche eine Zielsequenz für die Spaltung von Signalpeptidpeptidase umfaßt,
(b) Bereitstellen einer zweiten Komponente, welche eine Signalpeptidpeptidase umfaßt,
(c) Inkontaktbringen der beiden Komponenten mit einem zu testenden Mittel unter Bedingungen, die eine Wechselwirkung der beiden Komponenten in Abwesenheit des Mittels gestatten, und
(d) Bestimmen, ob das Mittel die Wechselwirkung zwischen der ersten und der zweiten Komponente moduliert,
wobei die Zielsequenz der Signalpeptidpeptidase von Hepatitis C-Virus- (HCV-) Kernprotein oder einem Homologen davon mit wenigstens 60% Identität dazu ableitbar ist.

2. Verfahren nach Anspruch 1, wobei das Kandidatenmittel an eine Zelle verabreicht wird und
wobei die Zielsequenz für die Spaltung der Signalpeptidpeptidase in der Zelle exprimiert wird.

3. Verfahren nach Anspruch 1 oder Anspruch 2, wobei die Signalpeptidpeptidase eine rekombinante Signalpeptidpeptidase oder ein natürlicher Bestandteil der Zelle ist.

4. Verfahren nach einem der vorangegangenen Ansprüche, welches weiterhin folgendes umfaßt:
(a) Verabreichen eines Virus an eine Zelle in Abwesenheit des Kandidatenmittels, von dem festgestellt wurde, daß es die Wechselwirkung zwischen der ersten und der zweiten Komponente moduliert,
(b) Verabreichen des Virus an die Zelle in Gegenwart des Mittels und
(c) Bestimmen, ob das Mittel die Anfälligkeit der Zelle für eine virale Infektion oder die Auswirkungen einer viralen Infektion reduziert oder beseitigt.

5. Verfahren nach Anspruch 4, wobei die Zielsequenz der Signalpeptidpeptidase SEQ ID NO:3 des HCV-Kernproteins oder ein Homologes davon mit wenigstens 60% Identität dazu umfaßt.

6. Verfahren zum Identifizieren eines Mittels zur Behandlung oder Prävention einer Hepatitis-Infektion oder einer anderen viralen Infektion der menschlichen oder tierischen Leber, wobei das Verfahren umfaßt, daß man bestimmt, ob das Mittel die Expression oder die Aktivität eines Signalpeptidpeptidaseenzyms in einer Säugerzelle modulieren kann.

7. Verfahren nach Anspruch 6, wobei die Zelle eine Leberzelle ist.

8. Proteinfragment, bestehend aus einer Zielsequenz von Signalpeptidpeptidase, wobei das Fragment aus SEQ ID NO:3 des HCV-Kernproteins besteht oder aus SEQ ID NO:4, 5 oder 6 besteht.

9. Proteinfragment nach Anspruch 8 zur Verwendung bei der Prävention oder Behandlung einer viralen Infektion.

10. Polynukleotid, welches das Proteinfragment nach Anspruch 8 codiert, zur Verwendung bei der Behandlung einer viralen Infektion.

11. Verwendung des Proteinfragments nach Anspruch 8 oder des Polynukleotids nach Anspruch 9 bei der Herstellung eines Medikaments zur Verwendung bei der Behandlung einer viralen Infektion.

12. Verfahren nach einem der Ansprüche 1 bis 5, wobei die virale Infektion eine Hepatitis-Infektion oder eine andere virale Infektion der menschlichen oder tierischen Leber ist.

## Revendications

1. Procédé pour identifier un agent candidat capable d'agir sur une infection virale, ce procédé comprenant les étapes consistant à :
(a) fournir un premier composant comprenant une séquence cible de clivage par signal-peptide-peptidase ;
(b) fournir un second composant comprenant une signal-peptide-peptidase ;
(c) mettre en contact les deux composants avec un agent à tester dans des conditions qui permettraient aux deux composants d'interagir en l'absence de l'agent ; et
(d) déterminer si l'agent module l'interaction entre les premier et second composants
dans lequel la séquence cible de signal-peptide-peptidase peut être dérivée de la protéine nucléocapsidique du virus de l'hépatite C (VHC) ou d'un de ses homologues ayant au moins 60 % d'identité avec celle-ci.

2. Procédé selon la revendication 1, dans lequel l'agent candidat est administré à une cellule et dans lequel la séquence cible de clivage par signal-peptide-peptidase est exprimée dans ladite cellule.

3. Procédé selon la revendication 1 ou la revendication 2, dans lequel la signal-peptide-peptidase est une signal-peptide-peptidase recombinante ou un constituant naturel de ladite cellule.

4. Procédé selon l'une quelconque des revendications précédentes, consistant de plus à :
(a) administrer un virus à une cellule en l'absence dudit agent candidat qui a été déterminé comme modulant l'interaction entre les premier et second composants ;
(b) administrer le virus à la cellule en présence dudit agent ; et
(c) déterminer si ledit agent réduit ou supprime la sensibilité de la cellule à l'infection virale ou les effets de l'infection virale.

5. Procédé selon la revendication 4, dans lequel la séquence cible de signal-peptide-peptidase comprend SEQ ID N° 3 de la protéine nucléocapsidique de VHC ou d'un de ses homologues ayant au moins 60 % d'identité avec celle-ci.

6. Procédé pour identifier un agent destiné à traiter ou prévenir une infection du genre hépatite ou autre infection virale du foie humain ou animal, lequel procédé comprend l'étape consistant à déterminer si ledit agent peut moduler l'expression ou l'activité d'une enzyme signal-peptide-peptidase dans une cellule de mammifère.

7. Procédé selon la revendication 6, dans lequel ladite cellule est une cellule hépatique.

8. Fragment de protéine consistant en une séquence cible de signal-peptide-peptidase dans laquelle ledit fragment est constitué par SEQ ID N° 3 de la protéine nucléocapsidique de VHC ou est constitué par SEQ ID N° 4, 5 ou 6.

9. Fragment de protéine selon la revendication 8, pour son utilisation dans la prévention ou le traitement d'une infection virale.

10. Polynucléotide codant pour le fragment de protéine selon la revendication 8, pour son utilisation dans le traitement d'une infection virale.

11. Utilisation du fragment de protéine selon la revendication 8 ou du polynucléotide selon la revendication 9, dans la fabrication d'un médicament à utiliser dans le traitement d'une infection virale.

12. Procédé selon l'une quelconque des revendications 1 à 5, dans lequel l'infection virale est une infection du genre hépatite ou autre infection virale du foie humain ou animal.
